# EUROPÄISCHE PATENTSCHRIFT

(11) **EP 1 163 231 B1**
(45) Veröffentlichungstag und Bekanntmachung des Hinweises auf die Patenterteilung: **05.10.2005**
(21) Anmeldenummer: 00910838.2
(22) Anmeldetag: 15.03.2000
(51) Int. Cl.: C07D 257/02, A61K 49/00, A61K 31/555, A61P 39/04

(54) **PERFLUORALKYLAMIDE, IHRE HERSTELLUNG UND IHRE VERWENDUNG IN DER DIAGNOSTIK**
PERFLUOROALKYLAMIDE, THE PRODUCTION THEREOF AND THE USE THEREOF IN DIAGNOSTICS
PERFLUOROALKYLAMIDE, LEUR PRODUCTION ET LEUR UTILISATION DANS DES DIAGNOSTICS

(30) Priorität: 22.03.1999 DE 19914101
(43) Veröffentlichungstag der Anmeldung: 19.12.2001
(73) Patentinhaber: Schering Aktiengesellschaft, 13342 Berlin (DE)
(72) Erfinder: PLATZEK, Johannes, Dr., 12621 Berlin (DE); NIEDBALLA, Ulrich, Dr., 14195 Berlin (DE); SÜLZLE, Detlev, Dr., 10589 Berlin (DE); SCHLECKER, Wolfgang, Dr., 84032 Altdorf (DE); RADÜCHEL, Bernd, Dr., 13465 Berlin (DE); WEINMANN, Hanns-Joachim, Dr., 14129 Berlin (DE); FRENZEL, Thomas, Dr., 12247 Berlin (DE); MISSELWITZ, Bernd, Dr., 16548 Glienicke (DE); EBERT, Wolfgang, Dr., 15831 Mahlow (DE)
(86) Internationale Anmeldenummer: PCT/EP2000/002285
(87) Internationale Veröffentlichungsnummer: WO 2000/056723

(56) Entgegenhaltungen:
- DE-A- 4 317 588
- DE-A- 19 603 033
- DE-A- 19 608 278

## Beschreibung

Die Erfindung betrifft den in den Patentansprüchen gekennzeichneten Gegenstand, das heißt makrocyclische Perfluoralkylamide, ihre Herstellung und ihre Verwendung in der Diagnostik.

In der kernmagnetischen Resonanz kommt nach dem Element Wasserstoff dem Element Fluor die größte Bedeutung zu.
1) Fluor hat eine hohe Empfindlichkeit von 83 % der des Wasserstoffs.
2) Fluor hat nur ein NMR-aktives Isotop.
3) Fluor hat eine dem Wasserstoff ähnliche Resonanzfrequenz - Fluor und Wasserstoff können mit der gleichen Anlage gemessen werden.
4) Fluor ist biologisch inert.
5) Fluor kommt in biologischem Material (Ausnahme: Zähne) nicht vor und kann deshalb als Sonde oder Kontrastmittel vor einem störsignalfreiem Untergrund eingesetzt werden.

Diese Eigenschaften führten dazu, daß Fluor einen breiten Raum in der diagnostischen Patentliteratur mit der magnetischen Kernresonanz als Grundlage einnimmt: Fiuor-19-imaging, Funktionsdiagnose, Spektroskopie.

So werden in dem US Patent 4,639,364 (Mallinckrodt) Trifluormethansulfonamide als Kontrastmittel für das Fluor-19-imaging vorgeschlagen:

CF₃SO₂NH₂

CF₃SO₂NH-CH₂-(CHOH)₄-CH₂OH

Ebenfalls mit dem Fluor-19-imaging beschäftigt sich das deutsche Patent DE 4203254 (Max-Planck-Gesetischaft), in dem ein Anilinderivat vorgeschlagen wird:

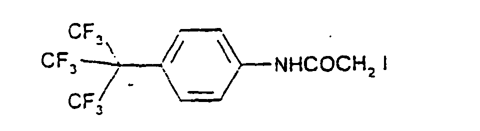

Das Fluor-19-imaging ist Gegenstand der Anmeldung WO 93/07907 (Mallinckrodt), in der ebenfalls Phenylderivate als Kontrastmittel beansprucht werden:

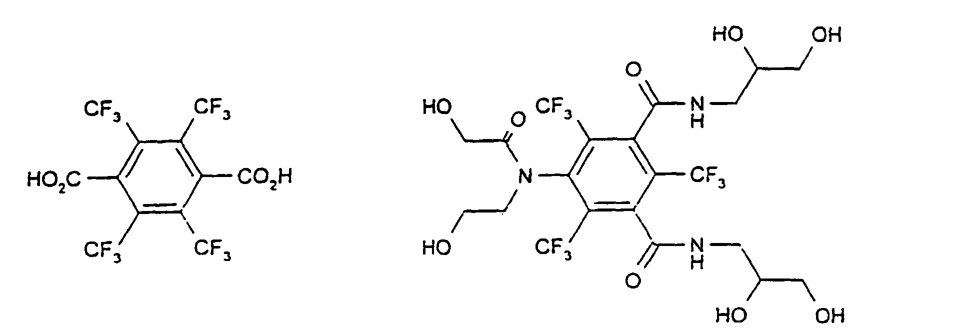

Für das Fluor-19-imaging werden auch erheblich einfacher gebaute Verbindungen beansprucht. So wird im Patent US 4,586,511 (Children's Hospital Medical Center) das Perfluoroctylbromid

CF₃(CF₂)₇-Br

genannt,
im Eurooäischen Patent EP 307863 (Air Products) der Pertluor-15-krone-5-ether 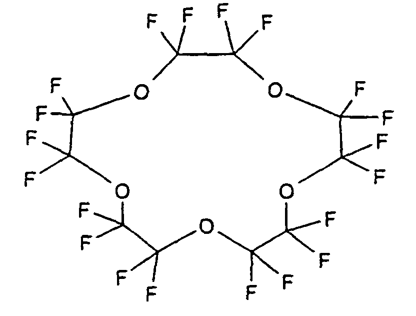 und im amerikanischen Patent US 4,558,279 (University of Cincinnati, Children's Hospital Research Foundation) Perfluorkohlenstoffverbindungen wie Perfluorcyclononan oder -octan, perfluorierte Ether wie das Tetrahydrofuran 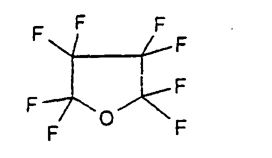 oder Diether wie der Perfluor-propylenglycol-diether

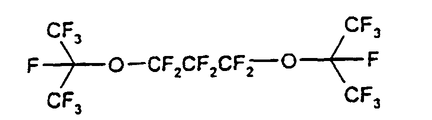

Ebenfalls für das Fluor-19-imaging dienen die in der Anmeldung WO 94/22368 (Molecular Biosystems) genannten Verbindungen z. B. 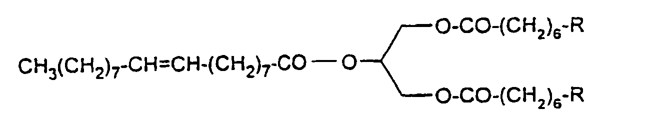 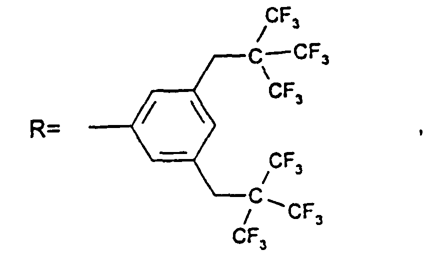 die als
fluorhaltigen Rest die Perfluor-1H, 1H-neopentylgruppe besitzen.

Einen weiteren Strukturtyp mit erweiterter diagnostischer Nutzung zeigt das amerikanische Patent US 5,362,478 (VIVORX) auf, in dem die Kombination Fluorkohlenstoff / polymere Hülle für imaging-Zwecke beansprucht wird. Genannt werden Perfluornonan und Humanserumalbumin. Diese Kombination erweist sich darüberhinaus als geeignet, das Fluoratom als Sonde für lokale Temperaturmessung und zur Bestimmung des Sauerstoffpartialdruckes einzusetzen.

Perfluorkohlenstoffe werden auch im US Patent 4,586,511 zur Sauerstoffbestimmung beansprucht.

In der deutschen Patentschrift DE 4008179 (Schering) werden fluorhaltige Benzolsulfonamide als pH-Sonden beansprucht.

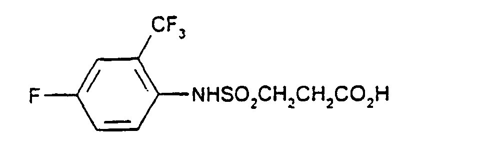

Für die NMR-Diagnostik werden auch Verbindungen, die Jod- und Fluoratome enthalten, als kontrastverstärkende Mittel beansprucht, so in WO 94/05335 und WO 94/22368 (beide Molecular Biosystems):

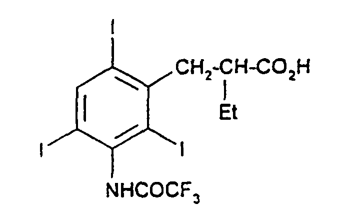

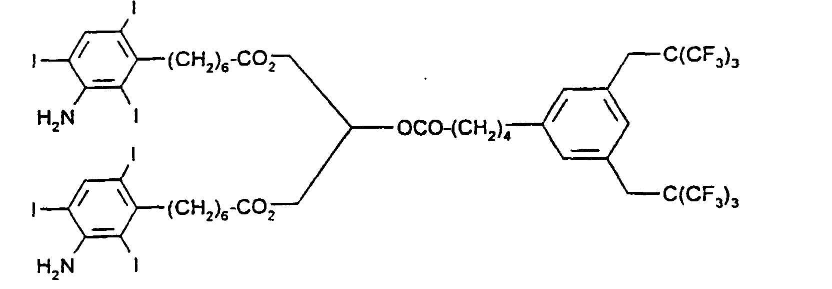

Auch die Kombination Fluor-paramagnetisches Metallion wird für das Fluor-19-imaging beansprucht, und zwar für offenkettige Komplexe in WO 94/22368 (Molecular Biosystems) mit z.B.: 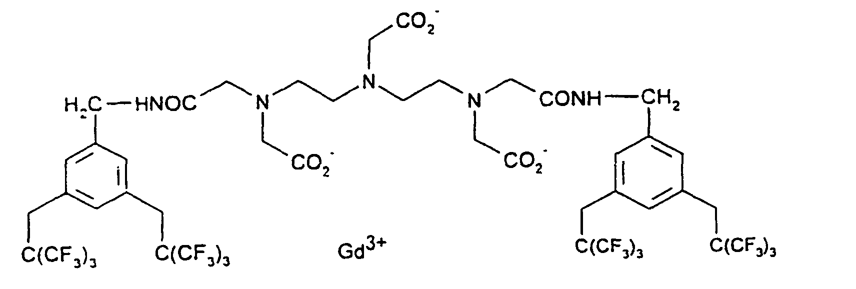 und in EP 292 306 (TERUMO Kabushiki Kaisha) mit z.B.: 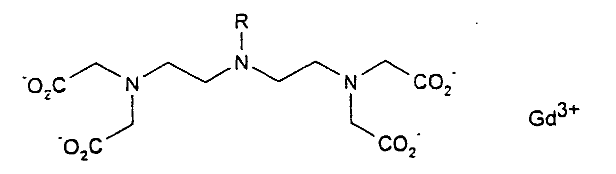 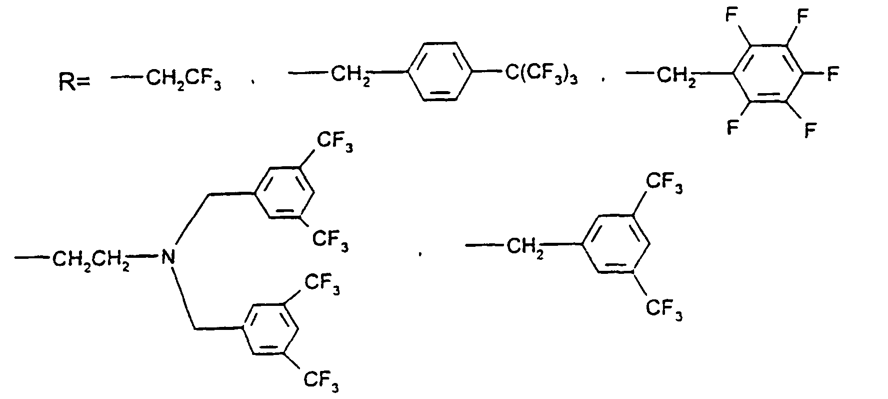 aber auch für cyclische Verbindungen, wie sie in EP 628 316 (TERUMO Kabushiki Kaisha) 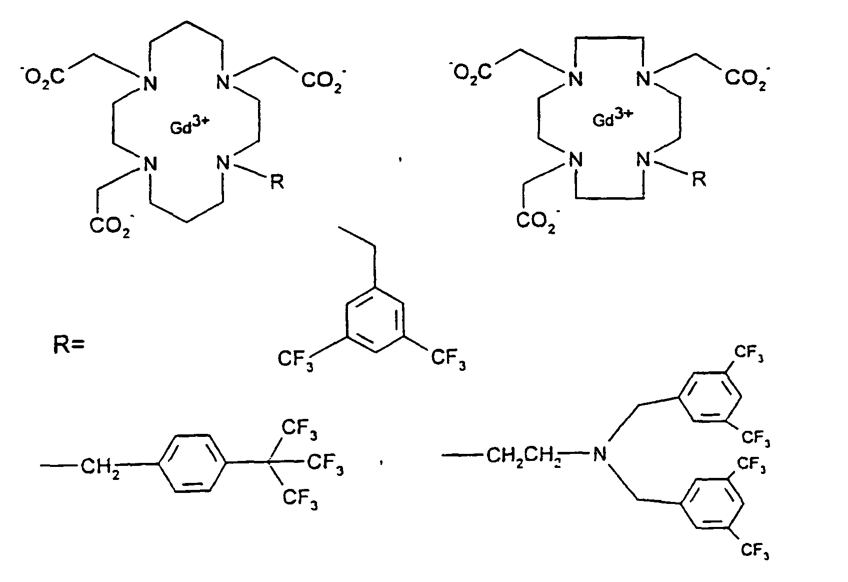 genannt werden.

Die Kombination Fluoratom-Seltenes Erdmetall wird auch für die NMR-spektroskopischen Temperaturmessungen in DE 4317588 (Schering) beansprucht:

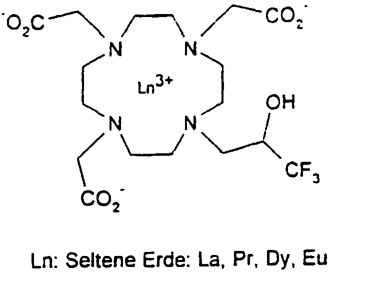

Während bei Verbindungen, die die Elemente Fluor und Jod enthalten, keine Wechselwirkungen zwischen den beiden Kernen stattfinden, findet in Verbindungen, die Fluor und paramagnetische Zentren (Radikale, Metallionen) enthalten, eine intensive Wechselwirkung statt, die sich in einer Verkürzung der Relaxationszeit des Fluorkernes äußern. Die Größe dieses Effekts hängt von der Anzahl der ungepaarten Elektronen des Metallions (Gd³⁺ > Mn²⁺ > Fe³⁺ > Cu²⁺) und von der Entfernung zwischen dem paramagnetischen Ion und dem ¹⁹F-Atom ab.

Je mehr ungepaarte Elektronen des Metallions vorhanden sind und je näher diese an das Fluor gebracht werden, desto größer ist die Verkürzung der Relaxationszeit des Fluorkemes.

Die Verkürzung der Relaxationszeit als Funktion des Abstandes vom paramagnetischen Ion macht sich bei allen Kernen mit ungerader Spinzahl bemerkbar, so auch beim Proton, und Gadoliniumverbindungen finden deshalb breite Anwendung als Kontrastmittel in der Kernspintomographie (Magnevist®, Prohance®, Omniscan®, Dotarem®).

Beim ¹H-MR-imaging (¹H-MRI) wird jedoch die Relaxationszeit T¹ oder T² der Protonen, das heißt vor allem der Protonen des Wassers, und nicht die Relaxationszeit der Fluorkerne gemessen und für die Bildgebung verwendet. Das quantitative Maß für die Verkürzung der Relaxationszeit ist die Relaxivity [L/mmol·s]. Zur Verkürzung der Relaxationszeiten werden mit Erfolg Komplexe paramagnetischer Ionen eingesetzt. In der folgenden Tabelle ist die Relaxivity einiger Handelspräparate angegeben:

| | **T**^{**1**}**-relaxivity in Wasser** [L/mmol·s ,39°C, 0.47 T] | **T**^{**1**}**-relaxivity in Plasma** [L/mmol·s,39°C, 0.47 T] |
|---|---|---|
| **MAGNEVIST ®** | **3,8** | **4,8** |
| **DOTAREM ®** | **3,5** | **4,3** |
| **OMNISCAN ®** | **3,8** | **4,4** |
| **PRO HANCE ®** | **3,7** | **4,9** |

In diesen Verbindungen finden nur Wechselwirkungen zwischen Protonen und dem Gadoliniumion statt. Für diese Kontrastmittel in Wassers wird also eine Relaxivity von ca. 4 [L/mmol·s] beobachtet.

Es werden also erfolgreich für das MR-imaging sowohl Fluor-Verbindungen für Fluor-19-imaging, bei dem die verkürzte Relaxationszeit des Fluor-Kernes ausgenutzt wird, als auch nicht Fluor-haltige Verbindungen, bei denen die Relaxationszeit der Protonen des Wassers gemessen wird, verwendet.

Bei der Einführung eines perfluorkohlenstoffhaltigen Restes in ein paramagnetisches Kontrastmittel, das heißt bei der Kombination von Eigenschaften, die bisher nur für Fluor-imaging-Verbindungen als geeignet bekannt waren, mit Verbindungen, die für Protonen-imaging verwendet wurden, steigt überraschenderweise auch die Relaxivity betreffend die Protonen des Wassers rapide an. Sie erreicht nun Werte von 10-50 [L/mmol·s] im Vergleich zu Werten zwischen 3,5 und 3,8 [L/mmol·s] wie sie für einige Handelsprodukte in obiger Tabelle bereits aufgeführt wurden.

Aus DE 196 03 033.1 sind perfluoralkylhaltige Metallkomplexe bekannt, die sich jedoch im Aufbau der Seitenkette von den Verbindungen der vorliegenden Erfindung unterscheiden.
Die Verbindungen der vorliegenden Erfindung zeichnen sich jedoch durch bessere Eigenschaften aus, wie zum Beispiel höhere Lymphknotenanreicherung in drei aufeinanderfolgenden Lymphknotenstationen, bessere Ausscheidung, größere Verträglichkeit (was besonders für die i.v. L ymphographie interessant ist), und sehr gute lokale Verträglichkeit bei der interstitiellen Gabe. Das eröffnet die Möglichkeit, die Verbindungen in höheren Dosen zu geben.

Die MRI-Kontrastmittel werden hauptsächlich für die Darstellung von malignen Tumoren verwendet.

Maligne Tumoren metastasieren gehäuft in regionale Lymphknoten, wobei auch mehrere Lymphknotenstationen beteiligt sein können. So werden Lymphknotenmetastasen in etwa 50 - 69% aller Patienten mit malignen Tumoren gefunden (Elke, Lymphographie, in: Frommhold, Stender, Thurn (eds.), Radiologische Diagnostik in Klinik und Praxis, Band IV, Thieme Verlag Stuttgart, 7th ed., 434-496, 1984).). Die Diagnose eines metastatischen Befalls von Lymphknoten ist im Hinblick auf die Therapie und Prognose maligner Erkrankungen von großer Bedeutung. Mit den modernen bildgebenden Methoden (CT, US und MRI) werden lymphogene Absiedlungen von malignen Tumoren nur unzureichend erkannt, da zumeist nur die Größe des Lymphknotens als Diagnosekriterium herangezogen werden kann. Damit sind kleine Metastasen in nicht-vergrößerten Lymphknoten (< 2 cm) nicht von Lymphknotenhyperplasien ohne malignen Befall zu unterscheiden (Steinkamp et al., Sonographie und Kernspintomographie: Differentialdiagnostik von reaktiver Lymphknoten-vergrößerung und Lymphknotenmetastasen am Hals, Radiol.diagn. 33:158, 1992).

Wünschenswert wäre es, daß bei Einsatz von spezifischen Kontrastmitteln Lymphknoten mit metastatischem Befall und hyperplastische Lymphknoten unterschieden werden können.

Bekannt ist die direkte Röntgen-Lymphographie (Injektion einer öligen Kontrastmittelsuspension in ein präpariertes Lymphgefäß) als eine nur noch selten benutzte invasive Methode, die nur wenige Lymphabflußstationen darstellen kann.

Experimentell werden in Tierexperimenten auch Fluoreszenz-markierte Dextrane verwendet, um nach deren interstitieller Applikation den Lymphabfluß beobachten zu können. Allen gebräuchlichen Markern für die Darstellung von Lymphwegen und Lymphknoten nach interstitieller/intrakutaner Applikation ist also gemein, daß es sich um Substanzen mit partikulärem Charakter ("particulates", z.B. Emulsionen und Nanokristallsuspensionen) oder große Polymere handelt (s.a. WO 90/14846). Die bisher beschriebenen Zubereitungen erwiesen sich jedoch aufgrund ihrer mangelnden lokalen und systemischen Verträglichkeit sowie ihrer geringen Lymphgängigkeit, die eine unzureichende diagnostischen Effizienz bedingt, als noch nicht optimal für die indirekte Lymphographie geeignet.

Da die Darstellung von Lymphknoten von zentraler Bedeutung für die frühe Erkennung des metastatischen Befalls bei Krebspatienten ist, besteht ein großer Bedarf an lymphspezifischen Kontrastmittelzubereitungen zur Diagnose entsprechender Veränderungen des Lymphsystems.
Möglichst hohe Kontrastmittelbeiadung und hohe Stabilität sind ebenso wünschenswert wie diagnostisch relevante, möglichst gleichmäßige Lymphanreicherung über mehrere Lymphstationen hinweg. Die Belastung des Gesamtorganismus sollte durch rasche und vollständige Ausscheidung des Kontrastmittels gering gehalten werden. Ein rascher Wirkungseintritt möglichst bereits innerhalb weniger Stunden nach Kontrastmittelapplikation ist für die radiologische Praxis von Bedeutung. Eine gute Verträglichkeit ist notwendig.

Die Aufgabe der Erfindung wird durch die makrocyclischen Perfluoralkyl-Verbindungen der allgemeinen Formel I 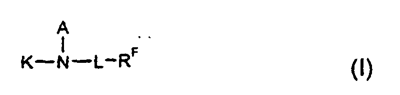 worin
- K: einen Komplexbildner oder einen Metallkomplex der allgemeinen Formel II 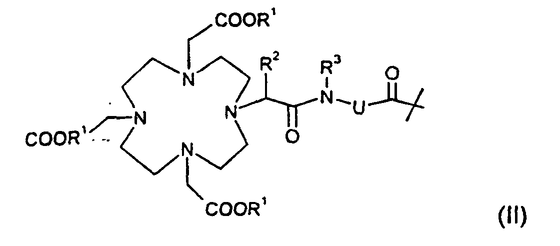 bedeutet,
wobei
R¹ für ein Wasserstoffatom oder ein Metallionenäquivalent der Ordnungszahlen 21-29, 31, 32, 37-39, 42-44, 49 oder 57-83,
R² und R³ für ein Wasserstoffatom, eine C₁-C₇-Alkylgruppe, eine Benzylgruppe, eine Phenylgruppe, -CH₂OH oder -CH₂-OCH₃, und
U für den Rest L, wobei L und U unabhängig voneinander gleich oder verschieden sein können, steht,
- A: ein Wasserstoffatom, eine gereadkettige oder verzweigte C₁-C₃₀-Alkyl-gruppe, die gegebenenfalls unterbrochen ist durch 1-15 Sauerstoffatome, und/oder gegebenenfalls substituiert ist mit 1-10 Hydroxygruppen, 1-2 COOH-Gruppen, einer Phenylgruppe, einer Benzylgruppe und/oder 1-5 - OR⁴-Gruppen, mit R⁴ in der Bedeutung eines Wasserstoffatoms oder eines C₁-C₇-Alkylrestes, oder -L-R^{F} bedeutet,
- L: eine geradkettige oder verzweigte C₁-C₃₀-Alkylengruppe, die gegebenenfalls unterbrochen ist durch 1-10 Sauerstoffatome, 1-5 -NH-CO-Gruppen, 1-5 -CO-NH- Gruppen, durch eine gegebenenfalls durch eine COOH-Gruppe substituierte Phenylengruppe, 1-3 Schwefelatome, 1-2 -N(B¹)-SO₂- Gruppen, und/oder 1-2 -SO₂-N(B¹)- Gruppen mit B¹ in der Bedeutung von A, und/oder gegebenenfalls substituiert ist mit dem Rest R^{F},
bedeutet und
- R^{F}: einen geradkettigen oder verzweigten perfluorierten Alkylrest der Formel CₙF₂ₙX,
wobei 4 ≤ n ≤ 20 ist und
X für ein endständiges Fluoratom, Chloratom, lodatom oder ein Wasserstoffatom steht, bedeutet,
und gegebenenfalls vorhandene Säuregruppen gegebenenfalls als Salze organischer und/oder anorganischer Basen oder Aminosäuren oder Aminosäureamide vorliegen können,
gelöst.

Die neuen erfindungsgemäßen perfluoralkylhaltigen Verbindungen der allgemeinen Formel I gemäß Patentanspruch 1 umfassen sowohl Komplexbildner als auch Metallkomplexe. Verbindungen der allgemeinen Formel I in denen das im Makrocyclus K gebundene Metallionenäquivalent fehlt, werden als Komplexbildner bezeichnet und Verbindungen mit einem im Makrocyclus K gebundenen Metallionenäquivalent werden als Metallkomplexe bezeichnet.

Als Metallionenäquivalent kommen je nach angestrebter Anwendung der erfindungsgemäßen Verbindungen die folgenden Metalle in Frage:
1. Bei Anwendung in der NMR- und Röntgen-Diagnostik: Komplexe mit den Ionen der Elemente mit den Ordnungszahlen 21 - 29, 39, 42, 44 und 57 - 83;
2. Bei Anwendung in der Radiodiagnostik und Radiotherapie: Komplexe mit den Radioisotopen der Elemente mit den Ordnungszahlen 27, 29. 31, 32, 37 - 39, 43, 49, 62, 64, 70, 75 und 77.

Bevorzugt werden die Ionen der Elemente mit den Ordnungszahlen 21 - 29, 39, 42, 44 und 57 - 83.

Besonders bevorzugt wird Gadolinium.

Die Alkylgruppen R², R³, R⁴ können geradkettig oder verzweigt sein. Beispielhaft seien Methyl, Ethyl, Propyl, Isopropyl, n-Butyl, 1-Methylpropyl, 2-Methylpropyl, n-Pentyl, 1-Methylbutyl, 2-Methylbutyl, 3-Methylbutyl, 1,2-Dimethylpropyl genannt.

Bevorzugt werden für R², R³ und R⁴ Wasersoff und C₁-C₄-Alkylgruppen, besonders bevorzugt Wasserstoff und die Methylgruppe.

Die Benzylgruppe und die Phenylgruppe R², A und B¹ können im Phenylring substituiert sein. Als Substituent kommt die COOH-Gruppe in Frage.

Enthält die Verbindung der Formel I gleichzeitig die Reste L und U, so können L und U voneinander verschieden sein.

Die C₁-C₃₀-Alkylengruppen U können geradkettig oder verzweigt sein. Beispielhaft seien Methylen, Ethylen, Propylen, Isopropylen, n-Butylen, 1-Methylpropylen, 2-Methylpropylen, n-Pentylen, 1-Methylbutylen, 2-Methylbutylen, 3-Methylbutylen, 1,2-Dimethylpropylen, genannt.
Für U in der Bedeutung Alkylen werden C₁-C₁₀ Alkylengruppen bevorzugt, besonders bevorzugt sind C₁-C₄ Alkylengruppen.

Die C₁-C₃₀-Alkylgruppen A können geradkettig oder verzweigt sein. Beispielhaft seien Methyl, Ethyl, Propyl, Isopropyl, n-Butyl, 1-Methylpropyl, 2-Methylpropyl, n-Pentyl, 1-Methylbutyl, 2-Methylbutyl, 3-Methylbutyl, 1,2-Dimethylpropyl, n-Hexyl, genannt.

Die C₁-C₃₀-Alkylgruppen A können durch 1-15 Sauerstoffatome unterbrochen, und/oder mit 1-10 Hydroxy-, 1-5 Alkoxy- oder 1-2 COOH-Gruppen substituiert sein wie z.B.
C₂H₄-O-CH₃, C₃H₆-O-CH₃,
C₂H₄-O-(C₂H₄-O)ₜ-C₂H₄-OH, C₂H₄-O-(C₂H₄-O)ₜ-C₂H₄-OCH₃ mit t = 0 bis 13
C₂H₄OH, C₃H₆OH, C₄H₈OH, C₅H₁₀OH, C₆H₁₂OH, C₇H₁₄OH, sowie ihre verzweigten Isomeren,
CH(OH)CH₂OH,
CH(OH)CH(OH)CH₂OH, CH₂[CH(OH)]ᵤCH₂OH, mit u = 1-10
CH[CH₂(OH)]CH(OH)CH₂OH,
C₂H₄CH(OH)CH₂OH,
(CH₂)_{S}COOH mit s = 1 bis 15,
C₂H₄-O-(C₂H₄-O)ₜ-CH₂COOH mit t = 0 bis 13,
C₂H₄-O-(C₂H₄-O)ₜ-C₂H₄-CₙF₂ₙX mit t = 0 bis 13, n = 4 bis 20 und X = ein Fluor-, Chlor-, Brom- oder lodatom.

Bevorzugte Bedeutungen für A sind Wasserstoff, C₁-C₁₀-Alkyl,
C₂H₄-O-CH₃, C₃H₆-O-CH₃,
C₂H₄-O-(C₂H₄-O)ₓ-C₂H₄-OH, C₂H₄-O-(C₂H₄-O)ₓ-C₂H₄-OCH₃ mit x = 0 bis 5, C₂H₄OH, C₃H₆OH,
CH₂[CH(OH)]_{y}CH₂OH, mit y = 1-6
CH[CH₂(OH)]CH(OH)CH₂OH,
(CH₂)_{w}COOH mit w = 1 bis 10,
C₂H₄-O-(C₂H₄-O)ₓ-CH₂COOH mit x = 0 bis 5,
C₂H₄-O-(C₂H₄-O)ₓ-C₂H₄-CₚF₂ₚX mit x = 0 bis 5, p =4 bis 15 und X = ein Fluor-atom.

Enthält die Verbindung der allgmeinen Formel I zwei Reste L-R^{F}, so können diese Reste verschieden voneinander sein.

Für die Reste L seien beispielhaft genannt, wobei α für die Bindung an das Stickstoffatom und β für die Bindung an den Rest R^{F} steht:
α-(CH₂)ₖ-β mit k = 1 - 15
α-CH₂-CH₂-(O-CH₂-CH₂-)ᵣ-β mit r = 1 - 6
α-CH₂-(O-CH₂-CH₂-)ᵣ-β mit r = 1 - 6
α-CH₂-NH-CO-β
α-CH₂-CH₂-NH-SO₂-β
α-CH₂-NH-CO-CH₂-N(CH₂COOH)-SO₂-β
α-CH₂-NH-CO-CH₂-N(C₂H₅)-SO₂-β
α-CH₂-NH-CO-CH₂-N(C₁₀H₂₁)-SO₂-β
α-CH₂-NH-CO-CH₂-N(C₆H₁₃)-SO₂-β
α-CH₂-NH-CO-(CH₂)₁₀-N(C₂H₅)-SO₂-β
α-CH₂-NH-CO-CH₂-N(-CH₂-C₆H₅)-SO₂-β
α-CH₂-NH-CO-CH₂-N(-CH₂-CH₂-OH)SO₂-β
α-CH₂-NHCO-(CH₂)₁₀-S-CH₂CH₂-β
α-CH₂NHCOCH₂-O-CH₂CH₂-β
α-CH₂-CH₂NHCOCH₂-O-CH₂CH₂-β
α-CH₂-(CH₂-CH₂-O)ᵣ-(CH₂)₃NHCO-CH₂-O-CH₂CH₂-β mit r = 1-6
α-CH₂NHCO(CH₂)₁₀-O-CH₂CH₂-β
α-CH₂CH₂NHCO(CH₂)₁₀-O-CH₂CH₂-β
α-CH₂-C₆H₄-O-CH₂CH₂-β wobei die Phenylengruppe 1,4 oder 1,3 verknüpft ist
α-CH₂-O-CH₂-C(CH₂-OCH₂CH₂-C₆F₁₃)₂-CH₂-OCH₂-CH₂-β
α-CH₂-NHCOCH₂CH₂CON-CH₂CH₂NHCOCH₂N(C₂H₅)SO₂C₈F₁₇β
α-CH₂-CH₂NHCOCH₂N(C₂H₅)-SO₂-β
α-CH₂-O-CH₂-CH(OC₁₀H₂₁)-CH₂-O-CH₂CH₂-β
α-(CH₂NHCO)₄-CH₂O-CH₂CH₂-β
α-(CH₂NHCO)₃-CH₂O-CH₂CH₂-β
α-CH₂-OCH₂C(CH₂OH)₂-CH₂-O-CH₂CH₂-β 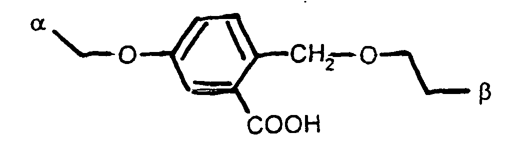 α-CH₂NHCOCH₂N(C₆H₅)-SO₂-β
α-NHCO-CH₂-CH₂-β
α-NHCO-CH₂-O-CH₂CH₂-β
α-NH-CO-β
α-NH-CO-CH₂-N(CH₂COOH)-SO₂-β
α-NH-CO-CH₂-N(C₂H₅)-SO₂-β
α-NH-CO-CH₂-N(C₁₀H₂₁)-SO₂-β
α-NH-CO-CH₂-N(C₆H₁₃)-SO₂-β
α-NH-CO-(CH₂)₁₀-N(C₂H₅)-SO₂-β
α-NH-CO-CH₂-N(-CH₂-C₆H₅)-SO₂-β
α-NH-CO-CH₂-N(-CH₂-CH₂-OH)SO₂-β
α-NH-CO-CH₂-β
α-CH₂-O-C₆H₄-O-CH₂-CH₂-β
α-CH₂-C₆H₄-O-CH₂-CH₂-β
α-N(C₂H₅)-SO₂-β
α-N(C₆H₅)-SO₂-β
α-N(C₁₀H₂₁)-SO₂-β
α-N(C₆H₁₃)-SO₂-β
α-N(C₂H₄OH)-SO₂-β
α-N(CH₂COOH)-SO₂-β
α-N(CH₂C₆H₅)-SO₂-β
α-N-[CH(CH₂OH)₂]-SO₂-β
α-N-[CH(CH₂OH)CH(OH)(CH₂OH)]-SO₂-β

Bevorzugt werden:
α-CH₂-O-CH₂CH₂-β
α-CH₂-CH₂-(O-CH₂-CH₂-)_{y}-β mit y = 1 - 6
α-CH₂-(O-CH₂-CH₂-)_{y}-β mit y = 1 - 6
α-CH₂-CH₂-NH-SO₂-β Bsp. 10
α-CH₂NHCOCH₂-O-CH₂CH₂-β
α-CH₂-CH₂NHCOCH₂-O-CH₂CH₂-β
α-CH₂-(CH₂-CH₂-O)_{y}-(CH₂)₃NHCO-CH₂-O-CH₂CH₂-β mit y = 1-6
α-CH₂NHCO(CH₂)₁₀-O-CH₂CH₂-β
α-CH₂CH₂NHCO(CH₂)₁₀-O-CH₂CH₂-β
α-CH₂-O-CH₂-CH(OC₁₀H₂₁)-CH₂-O-CH₂CH₂-β
α-CH₂-O-C₆H₄-O-CH₂-CH₂-β
α-CH₂-C₆H₄-O-CH₂-CH₂-β

Erfindungsgemäß ganz besonders bevorzugt sind die Reste L der in den Beispielen der vorliegenden Erfindungsbeschreibung genannten Verbindungen.

Für U gelten die oben aufgeführten Reste für L und die bevorzugt und besonders bevorzugt gekennzeichneten Reste, sowie die für die Bedeutung Alkylen oben aufgeführten und gegebenenfalls bevorzugten und besonders bevorzugten Reste, mit der Maßgabe, daß kein α-ständiges Stickstoffatom und keine endständige (β-ständige) SO₂- oder CO-Gruppe vorhanden sein darf.

Bevorzugte Reste B¹ sind Wasserstoff, geradkettige oder verzweigte C₁-C₁₀-Alkylreste, die gegebenenfalls durch 1-5 Sauerstoffatome unterbrochen, und/oder gegebenenfalls substituiert sind mit 1-5 Hydroxygruppen. 1-2 COOH-Gruppen, einer gegebenenfalls durch eine COOH-Gruppe substituierte Phenylgruppe, einer Benzylgruppe und/oder 1-5-OR⁴-Gruppen, mit R⁴ in der Bedeutung eines Wasserstoffatoms oder eines C₁-C₃-Alkylrestes.

Bevorzugte Reste R^{F} sind geradkettige oder verzweigte perfluorierte Alkylreste der Formel CₚF₂ₚX, wobei 4 kleiner gleich p und p kleiner gleich 15 ist und X für ein endständiges Fluoratom steht.

Die Herstellung der erfindungsgemäßen Verbindungen der allgemeinen Formel I 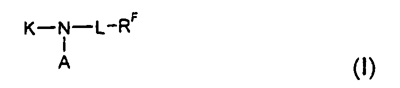 mit
- K: in der Bedeutung eines Komplexbildners oder eines Metallkomplexes der allgemeinen Formel II 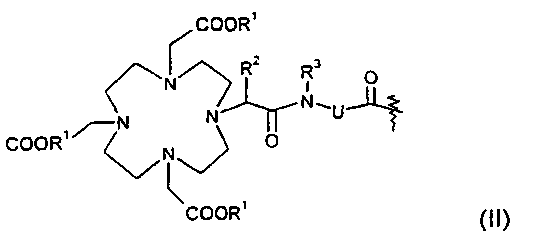
kann nach folgenden Verfahren erfolgen:

### Verfahren A.

Die Carbonsäure der Formel III enthält bereits das Metallionenäquivalent R¹.

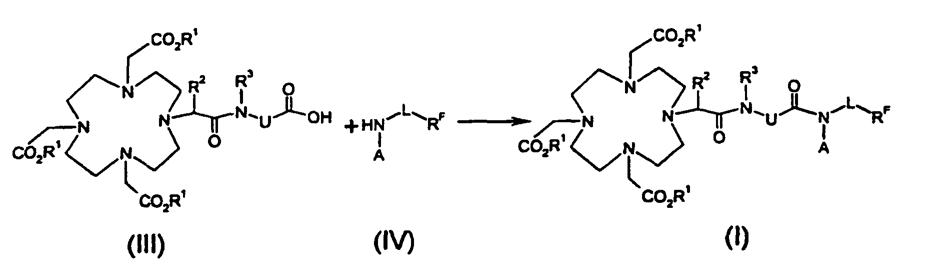

Die gegebenenfalls in situ aktivierte Carbonsäure III mit R¹ in der Bedeutung eines Metallionenäquivalents wird mit einem Amin IV in einer Kupplungsreaktion zu einem Amid I umgesetzt.
Dieses Verfahren zur Herstellung von Metallkomplexcarbonsäureamiden ist aus DE 196 52 386 bekannt.

Das in die Kupplungsreaktion eingesetzte Gemisch aus Metallkomplexcarbonsäure III, die gegebenenfalls vorhandene Carboxy- und/oder Hydroxygruppen in geschützter Form enthält, und mindestens einem lösungsvermittelndem Stoff in einer Menge bis zu 5, vorzugsweise 0,5-2 Moläquivalenten bezogen auf die Metallkomplexcarbonsäure kann sowohl in einer vorgeschalteten Reaktionsstufe hergestellt und (z.B. durch Eindampfen, Gefriertrocknung oder Sprühtrocknung einer wäßrigen oder mit Wasser mischbaren Lösung der Bestandteile oder durch Fällung mit einem organischen Lösungsmittel aus einer derartigen Lösung) isoliert werden und anschließend in DMSO mit wasserabspaltendem Reagenz und gegegebenenfalls einem Kupplungs-Hilfsstoff umgesetzt werden als auch in situ gegebenenfalls durch Zusatz von lösungsvermittelndem/n Stoff(en) zur DMSO-Suspension von Metallkomplexcarbonsäure, wasserabspaltendem Reagenz und gegebenenfalls einem Kupplungs-Hitfsstoff gebildet werden.

Die nach einem dieser Verfahren hergestellte Reaktionslösung wird zur Vorbehandlung (Säureaktivierung) 1 bis 24, vorzugsweise 3 bis 12 Stunden bei Temperaturen von 0 bis 50° C, vorzugsweise bei Raumtemperatur, gehalten.

Anschließend wird ein Amin der allgemeinen Formel IV 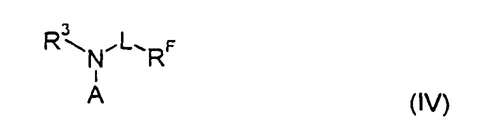 worin die Reste R³, L, R^{F} und A die oben angegebenen Bedeutungen haben, ohne Lösungsmittel oder gelöst, zum Beispiel in Dimethylsulfoxid, Alkoholen wie z.B. Methanol, Ethanol, Isopropanol oder deren Gemischen, Formamid, Dimethylformamid, Wasser oder Mischungen der aufgeführten Lösungsmittel, vorzugsweise in Dimethylsulfoxid, in Wasser oder in mit Wasser gemischten Lösungsmitteln, zugesetzt. Zur Amidkupplung wird die so erhaltene Reaktionslösung bei Temperaturen von 0 bis 70° C, vorzugsweise 30 bis 60° C. 1 bis 48, vorzugsweise 8 bis 24 Stunden gehalten.

In einigen Fällen hat es sich als vorteilhaft erwiesen, das Amin in Form seiner Salze, z.B. als Hydrobromid oder Hydrochlorid in die Reaktion einzusetzen. Zur Freisetzung des Amins wird eine Base wie z.B. Triethylamin, Diisopropylethylamin, N-Methylmorpholin, Pyridin, Tripropylamin, Tributylamin, Lithiumhydroxid, Lithiumcarbonat, Natriumhydroxid oder Natriumcarbonat zugesetzt.

Die gegebenenfalls noch vorhandenen Schutzgruppen werden anschließend abgespalten.

Die Isolierung des Reaktionsprodukts erfolgt nach den dem Fachmann bekannten Methoden, vorzugsweise durch Ausfällung mit organischen Lösungsmitteln, vorzugsweise Aceton, 2-Butanon, Diethylether, Essigester, Methyl-t.-butylether, Isopropanol oder deren Mischungen. Die weitere Reinigung kann beispielsweise durch Chromatographie, Kristallisation oder Ultrafiltration erfolgen.

Als lösungsvermittelnde Stoffe sind Alkali-, Erdalkali-, Trialkylammonium-, Tetraalkylammoniumsalze, Harnstoffe, N-Hydroxyimide, Hydroxyaryltriazole, substituierte Phenole und Salze von heterocyclischen Aminen geeignet. Beispielhaft genannt seien: Lithiumchlorid, Lithiumbromid, Lithiumjodid, Natriumbromid, Natriumjodid. Lithiummethansulfonat, Natriummethansulfonat, Lithiump-toluolsulfonat. Natrium-p-toluolsulfonat, Kaliumbromid, Kaliumjodid, Natriumchlorid, Magnesiumbromid, Magnesiumchlorid, Magnesiumjodid, Tetraethylammonium-p-toluolsulfonat, Tetramethylammonium-p-toluolsulfonat, Pyridinium-p-toluolsulfonat. Triethylammonium-p-toluolsulfonat. 2-Morpholinoethylsulfonsäure. 4-Nitrophenol, 3,5-Dinitrophenol, 2,4-Dichlorphenol, N-Hydroxysuccinimid, N-Hydroxyphthalimid, Harnstoff. Tetramethylharnstoff. N-Methylpyrrolidon, Formamid sowie cyclische Harnstoffe. wobei die fünf erstgenannten bevorzugt sind.

Als wasserabspaitende Reagenzien dienen alle dem Fachmann bekannten Mittel. Beispielhaft genannt seien Carbodiimide und Onium-Reagenzien wie z.B. Dicyclohexylcarbodiimid (DCCI), 1-Ethyl-3-(3-dimethylaminopropyl)-carbodiimid-hydroxychlorid (EDC), Benzotriazol-1-yloxytris(dimethylamino)-phosphoniumhexafluorophosphat (BOP) und O-(Benzotriazol-1-yl)-1,1,3,3-tetramethyluroniumhexafluorophosphat (HBTU), vorzugsweise DCCI.
In der Literatur sind zum Beispiel folgende geeignete Verfahren beschrieben:
◆ Aktivierung von Carbonsäuren. Übersicht in Houben-Weyl, Methoden der Organischen Chemie, Band XV/2, Georg Thieme Verlag Stuttgart. 1974 (und J.Chem. Research (S) 1996, 302).
◆ Aktivierung mit Carbodiimiden. R. Schwyzer u. H. Kappeler, Helv. 46: 1550 (1963).
◆ E. Wünsch et al., B. 100: 173 (1967).
◆ Aktivierung mit Carbodümiden/Hydroxysuccinimid: J. Am. Chem. Soc. 86: 1839 (1964) sowie J. Org. Chem. 53: 3583 (1988). Synthesis 453 (1972).
◆ Anhydridmethode, 2-Ethoxy-1-ethoxycarbonyl-1,2-dihydrochinolin: B. Belleau et al., J. Am. Chem. Soc., 90: 1651 (1986), H. Kunz et al., Int. J. Pept. Prot. Res., 26: 493 (1985) und J. R. Voughn, Am. Soc. 73: 3547 (1951).
◆ Imidazoiid-Methode: B.F. Gisin, R.B. Menifeld, D.C. Tosteon, Am. Soc. 91: 2691 (1969).
◆ Säurechlorid-Methoden, Thionylchlorid: Helv., 42: 1653 (1959).
◆ Oxalylchlorid: J. Org. Chem., 29: 843 (1964).

Als gegebenenfalls zu verwendende Kupplungs-Hilfsstoffe sind alle dem Fachmann bekannten geeignet (Houben-Weyl, Methoden der organischen Chemie, Bd. XV/2, Georg Thieme-Verlag, Stuttgart, 1974). Beispielhaft genannt seien 4-Nitrophenol, N-Hydroxysuccinimid, 1-Hydroxybenzotriazol, 1-Hydroxy-7-azabenzotriazol, 3,5-Dinitrophenol und Pentafluorphenol. Bevorzugt sind 4-Nitrophenol und N-Hydroxysuccinimid, besonders bevorzugt ist dabei das erstgenannte Reagenz.

Die Abspaltung der Schutzgruppen erfolgt nach den dem Fachmann bekannten Verfahren, beispielsweise durch Hydrolyse, Hydrogenolyse, alkalische Verseifung der Ester mit Alkali in wässrig-alkoholischer.Lösung bei Temperaturen von 0° bis 50° C, saure Verseifung mit Mineralsäuren oder im Fall von z.B. tert.-Butylestern mit Hilfe von Trifluoressigsäure.[Protective Groups in Organic Synthesis, 2nd Edition, T.W. Greene and P.G.M. Wuts, John Witey and Sons, Inc. New York, 1991], im Falle von Benzylethern mit Wasserstoff/Palladium/Kohle.

Die Herstellung des Ausgangsmaterials, der Verbindungen der Formel III, 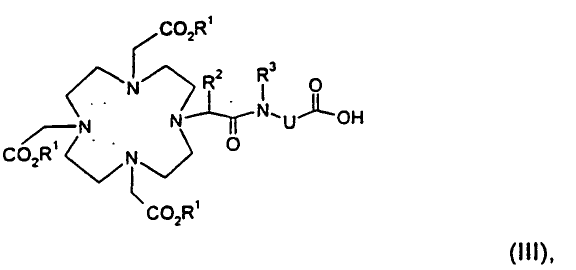 ist aus DE 196 52 386 bekannt.

Die Amine der ailgmeinen Formel IV 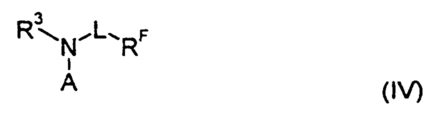 sind Kaufware (Fluorochem, ABCR) oder können nach folgendem Verfahren erhalten werden aus Verbindungen der allgemeinen Formel V durch Umsetzung mit einem Amin der allgemeinen Formel VI und anschließender Reduktion der Verbindungen der allgemeinen Formel VII: 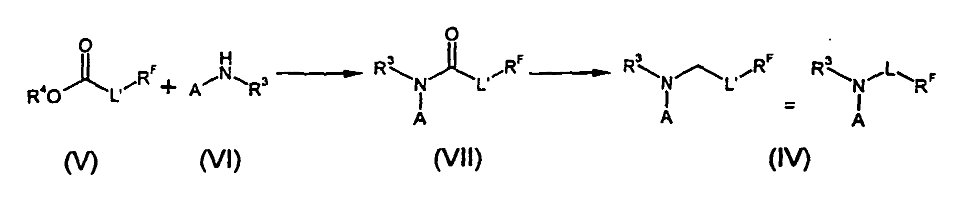 in denen
R^{F}, A, L und R³ die oben genannte Bedeutung haben, und L' die Bedeutung der Gruppe L hat, bei der die α-CH₂-Gruppe noch fehlt, und
R⁴ für Wasserstoff oder eine Methylgruppe steht.

Nach den bereits oben für die Aktivierung der Carbonsäure III offenbarten in der Literatur beschriebenen Verfahren wird die Säure V vor der Umsetzung mit dem Amin VI aktiviert. Für R⁴ in der Bedeutung einer Methylgruppe wird eine Aminolyse durchführt.

Die Verbindungen der allgemeinen Formel V sind Kaufware (Fluorochem, ABCR) oder werden, wie in DE 196 03 033 offenbart, hergestellt.

Die Verbindungen der Formel VI sind Kaufware (Fluorochem, ABCR) oder können wie in Houben-Weyl, Methoden der organischen Chemie, XI/2 Stickstoffverbindungen, Georg Thieme Verlag Stuttgart, 1957, S. 680; J.E. Rickman and T. Atkins, Am. Chem. Soc., 96:2268, 1974, 96:2268; F. Chavez and A.D. Sherry, J. Org. Chem. 1989, 54:2990 beschrieben, hergestellt werden:
Die Verbindungen der allgemeinen Formel IV werden in an sich bekannter Weise [Helv. Chim. Acta, 77: 23 (1994)] durch Reduktion der Verbindungen der allgmeinen Formel VII zum Beispiel mit Diboran oder Lithiumaluminiumhydrid und Abspaltung der Schutzgruppen gewonnen.

### Verfahren B.

Als Ausgangsmaterial dient die Carbonsäure der Formel IIIa mit R¹ in der Bedeutung von Wasserstoff - sie enthält noch kein Metallionenäquivalent R¹. Die Carboxylgruppen werden nach den dem Fachmann bekannten Verfahren geschützt und eine Verbindung der Formel IIIb erhalten, wobei R⁵ für eine beliebige Schutzgruppe steht.

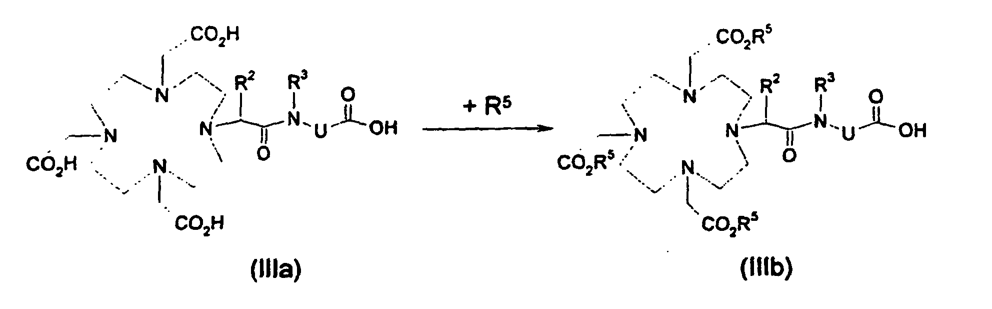

Als Carboxylschutzgruppe kommen z.B. geradkettige oder verzweigte C₁-C₆-Alkyl-, Aryl- und Aralkylgruppen, beispielsweise die Methyl-, Ethyl-, Propyl-, Butyl-, Phenyl-, Benzyl-, Diphenylmethyl-, Triphenylmethyl-, bis(p-Nitrophenyl)-methylgruppe sowie Trialkylsilylgruppen in Frage. Bevorzugt ist die t-Butylgruppe.

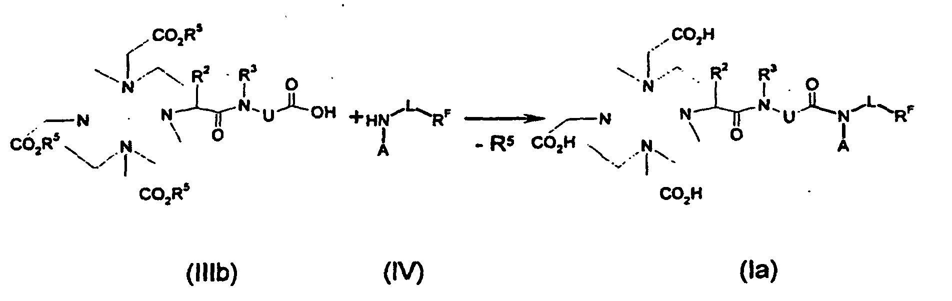

Die Umsetzung der geschützten Carbonsäure IIIb mit dem Amin der Formel IV und die Abspaltung der Schutzgruppen erfolgt wie unter Verfahren A beschrieben und in einem Folgeschritt wird die erhaltene Carbonsäure la noch mit mindestens einem Metalloxid oder Metallsalz eines Elements der gewünschten Ordnungszahl umgesetzt wie es z.B. in DE 195 25 924 offenbart ist.

Enthält der aus Verfahren A oder B erhaltene Metallkomplex noch freie COOH-Gruppen, so können diese Gruppen auch als Salze physiologisch verträglicher anorganischer oder organischer Basen vorliegen.
Die Neutralisation eventuell noch vorhandener freier Carboxygruppen erfolgt dann mit Hilfe anorganischer Basen (zum Beispiel Hydroxiden, Carbonaten oder Bicarbonaten) von zum Beispiel Natrium, Kalium, Lithium, Magnesium oder Calcium und/oder organischer Basen wie unter anderem primärer, sekundärer und tertiärer Amine, wie zum Beispiel Ethanolamin. Morpholin. Glucamin. N-Methyl- und N,N-Dimethylglucamin, sowie basischer Aminosäuren, wie zum Beispiel Lysin. Arginin und Ornithin oder von Amiden ursprünglich neutraler oder saurer Aminosäuren.

Zur Herstellung der neutralen Kompiexverbindungen kann man beispielsweise den sauren Komplexsalzen in wäßriger Lösung oder Suspension soviel der gewünschten Basen zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie zum Beispiel niederen Alkoholen (Methanol, Ethanol, Isopropanol und andere), niederen Ketonen (Aceton und andere), polaren Ethern (Tetrahydrofuran. Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Mit den erfindungsgemäßen Verbindungen werden höhere Blutkonzentrationen erreicht als mit extrazellulären Kontrastmitteln. Sie verteilen sich nach i.v. Applikation nur im Intravasalraum und haben damit einen entschiedenden Vorteil gegenüber den extrazellulären Kontrastmitteln.

Eine bessere Elimination aus dem Blut über die Nieren gewährleistet eine geringe Belastung des Gesamtorganismus.

Die Verbindungen der vorliegenden Erfindung zeichnen sich durch eine bessere Verträglichkeit, höhere Lymphknotenanreicherung in drei aufeinanderfolgenden Lymphknotenstationen (was besonders für die i.v. Lymphographie wichtig ist). Sie sind damit besonders gut geeignet für die Anwendung in der MRT-Lymphographie.

Die erfindungsgemäßen Verbindungen sind für die NMR- und Röntgen-Diagnostik und für die Radiodiagnostik und Radiotherapie geeignet.

Gegenstand der Erfindung ist daher auch die Verwendung der erfindungsgemäßen Verbindungen zur Herstellung eines Kontrastmitels für die Anwendung in der NMR- und Röntgen-Diagnostik, für die Radiodiagnostik und die Radiotherapie.

Gegenstand der Erfindung sind ferner pharmazeutische Mittel, die mindestens eine physiologisch verträgliche Verbindung der allgemeinen Formel I enthalten, gegebenenfalls mit den in der Galenik üblichen Zusätzen.

Die Herstellung der erfindungsgemäßen pharmazeutischen Mittel erfolgt in an sich bekannter Weise, indem man die erfindungsgemäßen Komplexverbindungen - gegebenenfalls unter Zugabe der in der Galenik üblichen Zusätze - in wäßrigem Medium suspendiert oder löst und anschließend die Suspension oder Lösung gegebenenfalls sterilisiert. Geeignete Zusätze sind beispielsweise physiologisch unbedenkliche Puffer (wie zum Beispiel Tromethamin), Zusätze von Komplexbildnern oder schwachen Komplexen (wie zum Beispiel Diethylentriaminpentaessigsäure oder die zu den erfindungsgemäßen Metallkomplexen korrespondierenden Ca-Komplexe) oder - falls erforderlich - Elektrolyte wie zum Beispiel Natriumchlorid oder - falls erforderlich - Antioxidantien wie zum Beispiel Ascorbinsäure.

Sind für die enterale bzw. parenterale Verabreichung oder andere Zwecke Suspensionen oder Lösungen der erfindungsgemäßen Mittel in Wasser oder physiologischer Salzlösung erwünscht, werden sie mit einem oder mehreren in der Galenik üblichen Hilfsstoff(en) [zum Beispiel Methyl-cellulose, Lactose, Mannit] und/oder Tensid(en) [zum Beispiel Lecithine, Tween®, Myrj® ] und/oder Aromastoff(en) zur Geschmackskorrektur [zum Beispiel ätherischen Ölen] gemischt.

Prinzipiell ist es auch möglich, die erfindungsgemäßen pharmazeutischen Mittel ohne Isolierung der Komplexe herzustellen. In jedem Fall muß besondere Sorgfalt darauf verwendet werden, die Chelatbildung so vorzunehmen, daß die erfindungsgemäßen Komplexe praktisch frei sind von nicht komplexierten toxisch wirkenden Metallionen.

Dies kann beispielsweise mit Hilfe von Farbindikatoren wie Xylenolorange durch Kontrolltitrationen während des Herstellungsprozesses gewährleistet werden. Die Erfindung betrifft daher auch Verfahren zur Herstellung der Komplexverbindungen und ihrer Salze. Als letzte Sicherheit bleibt eine Reinigung des isolierten Komplexes.

Bei der in-vivo-Applikation der erfindungsgemäßen Mittel können diese zusammen mit einem geeigneten Träger wie zum Beispiel Serum oder physiologischer Kochsalzlösung und zusammen mit einem anderen Protein wie zum Beispiel Humanserumalbumin (HSA) verabreicht werden.

Die erfindungsgemäßen Mittel werden üblicherweise parenteral, vorzugsweise i.v., appliziert. Sie können auch intravasal oder interstitiell/intrakutan appliziert werden, je nachdem, ob Körpergefäße oder -gewebe untersucht werden sollen.

Die erfindungsgemäßen pharmazeutischen Mittel enthalten vorzugsweise 0.1µ Mol - 1 Mol/l des Komplexes und werden in der Regel in Mengen von 0,0001 - 5 mMol/kg dosiert.

Die nachfolgenden Beispiele dienen der näheren Erläuterung der Erfindungsgegenstandes, ohne ihn auf diese beschränken zu wollen.

### Beispiel 1

### a) 2H, 2H, 4H, 4H, 5H, 5H-3-Oxa-perfluortridecansäure-N-(2-methoxy)-ethylamid

Zu 30 g (57,45 mmol) 2H, 2H, 4H, 4H. 5H, 5H-3-oxa-perfluortridecansäure in 300 ml Dichlormethan gibt man 8,90 g (70 mmol) Oxalylchlorid und rührt 12 Stunden bei Raumtemperatur. Es wird im Vakuum zur Trockne eingedampft. Der Rückstand wird in 100 ml Dichlormethan gelöst und bei 0°C zu einer Lösung aus 4,51 g (60 mmol) 2-Methoxyethylamin und 6,07 g (60 mmol) Triethylamin, gelöst in 200 ml Dichlormethan getropft. Man rührt 3 Stunden bei 0°C, anschließend 6 Stunden bei Raumtemperatur. Man gibt 300 ml 5 %ige aqu. Salzsäure zu und rührt 15 Minuten gut durch. Die organische Phase wird abgetrennt, über Magnesiumsulfat getrocknet und im Vakuum zur Trockne eingedampft. Der Rückstand wird an Kieselgel (Laufmittel: Dichlormethan/Aceton= 20:1) chromatographiert.
Ausbeute: 30,28 g (91 % d. Th.) eines farblosen Feststoffes

| Elementaranalyse: | | | | |
|---|---|---|---|---|
| ber. | C 31,10 | H 2,44 | N 2,42 | F 55,76 |
| gef. | C 30,87 | H 2,58 | N 2,35 | F 55,51 |

### b) N-(2-Methoxyethyl)-N-(1H, 1H, 2H, 2H, 4H, 4H, 5H, 5H-3-oxa)-perfluortridecylamin

30 g (51,79 mmol) der Titelverbindung aus Beispiel 1a werden in 300 ml Tetrahydrofuran gelöst und 31 ml 10 M Borandimethylsulfid (in Tetrahydrofuran) zugegeben. Man kocht 16 Stunden unter Rückfluß. Es wird auf 0°C abgekühlt und 200 ml Methanol zugetropft, anschließend wird im Vakuum zur Trockne eingedampft. Der Rückstand wird in einer Mischung aus 300 ml Ethanol/50 ml 10 %iger aqu. Salzsäure aufgenommen und 8 Stunden bei 40°C gerührt. Man dampft im Vakuum zur Trockne ein, nimmt den Rückstand in 300 ml 5 %iger aqu. Natronlauge auf und extrahiert 3 mal mit je 300 ml Dichlormethan. Die organischen Phasen werden über Magnesiumsulfat getrocknet, im Vakuum zur Trockne eingedampft und der Rückstand an Kieselgel chromatographiert (Laufmittel: Dichlormethan/2 Propanol= 20:1).
Ausbeute: 26,93 g (92 % d. Th.) eines farblosen Feststoffes

| Elementaranalyse (berechnet auf wasserfreie Substanz): | | | | |
|---|---|---|---|---|
| ber. | C 31,87 | H 2,85 | N 2,48 | F 57,14 |
| gef. | C 31,69 | H 3,10 | N 2,27 | F 56,88 |

### c) 1,4,7-Tris(carboxylatomethyl)-10-[(3-aza-4-oxo-hexan-5-yl)-säure-N-(2-methoxyethyl)-N-(1H, 1H, 2H, 2H, 4H, 4H, 5H, 5H-3-oxa)-perfluortridecyl)-amid]-1,4,7,10-tetraazacyclododecan, Gadoliniumkomplex

10 g (15,88 mmol) vom Gadoliniumkomplex der 10-[1-(carboxymethylcarboamoyl)-ethyl]-1,4,7,10-tetraazacyclododecan-1,4,7-triessigsäure und 1,35 g (31,76 mmol) Lithiumchlorid werden bei 60 °C in 100 ml Dimethylsulfoxid gelöst. Man kühlt auf 15 °C ab und gibt 8,98 g (15,88 mmol) der Titelverbindung aus Beispiel 1 b zu. Man rührt 10 Minuten und gibt dan 7,42 g (30 mmol) 2-Ethoxy-1-ethoxycarbonyl-1,2-dihydrochinolin zu. Es wird 12 Stunden bei Raumtemperatur gerührt. Man gießt die Lösung in eine Mischung aus 200 ml Aceton /1300 ml Diethylether und rührt 2 Stunden bei Raumtemperatur. Man filtriert den ausgefallenen Niederschlag ab, löst ihn in einer Mischung aus wenig Ethanol/Wasser und chromatographiert an Kieselgel RP-18 (Laufmittel: Gradient aus Tetrahydrofuran/Acetonitril/Wasser).
Ausbeute: 15,14 g (81 % d. Th.) eines farblosen, amorphen Pulvers
Wassergehalt: 5,7 %

| Elementaranalyse (berechnet auf wasserfreie Substanz): | | | | | |
|---|---|---|---|---|---|
| ber. | C 34,70 | H 3,77 | N 7,14 | F 27,44 | Gd 13,36 |
| gef. | C 34,51 | H 3,94 | N 7,02 | F 27,25 | Gd 13,18 |

### Beispiel 2

### a) 2H, 2H, 4H, 4H, 5H, 5H-3-oxa)-perfluortridecansäure-N-(2,3-dihydroxypropyl)-amid

Zu 30 g (57,45 mmol) 2H, 2H, 4H, 4H, 5H, 5H-3-oxa-perfluortridecansäure in 300 ml Dichlormethan gibt man 8,90 g (70 mmol) Oxalylchlorid und rührt 12 Stunden bei Raumtemperatur. Es wird im Vakuum zur Trockne eingedampft. Der Rückstand wird in 100 ml Dichlormethan gelöst und bei 0°C zu einer Lösung aus 5,47 g (60 mmol) 2,3- Dihydroxypropylamin und 6,07 g (60 mmol) Triethylamin, gelöst in 200 ml Dichlormethan getropft. Man rührt 3 Stunden bei 0°C, anschließend 6 Stunden bei Raumtemperatur. Man gibt 300 ml 5 %ige aqu. Salzsäure zu und rührt 15 Minuten gut durch. Die organische Phase wird abgetrennt, über Magnesiumsulfat getrocknet und im Vakuum zur Trockne eingedampft. Der Rückstand wird an Kieselgel (Laufmittel: Dichlormethan/Ethanol= 15:1) chromatographiert.
Ausbeute: 29,70 g (87 % d. Th.) eines farblosen Feststoffes

| Elementaranalyse: | | | | |
|---|---|---|---|---|
| ber. | C 30,32 | H 2,20 | N 2,36 | F 54,35 |
| gef. | C 30,12 | H 2,41 | N 2,18 | F 54,15 |

### b) N-(2,3-Dihydroxypropyl)-N-(1H, 1H, 2H, 2H, 4H, 4H, 5H, 5H-3-oxa-perfluortridecyl)-amin

30 g (48,8 mmol) der Titelverbindung aus Beispiel 2a werden in 300 ml Tetrahydrofuran gelöst und 50 ml 10 M Borandimethylsulfid (in Tetrahydrofuran) zugegeben. Man kocht 16 Stunden unter Rückfluß. Es wird auf 0°C abgekühlt und 300 ml Methanol zugetropft, anschließend wird im Vakuum zur Trockne eingedampft. Der Rückstand wird in einer Mischung aus 300 ml Ethanol/50 ml 10 %iger aqu. Salzsäure aufgenommen und 8 Stunden bei 60°C gerührt. Man dampft im Vakuum zur Trockne ein, nimmt den Rückstand in 300 ml 5 %iger aqu. Natronlauge auf und extrahiert 3 Mal mit je 300 ml Dichlormethan. Die organischen Phasen werden über Magnesiumsulfat getrocknet, im Vakuum zur Trockne eingedampft und der Rückstand an Kieselgel chromatographiert (Laufmittel: Dichlormethan/Methanol= 15:1).
Ausbeute: 24.07 g (85 % d. Th.) eines farblosen Feststoffes

| Elementaranalyse: | | | | |
|---|---|---|---|---|
| ber. | C 31,05 | H 2,61 | N 2,41 | F 55,66 |
| gef. | C 31,91 | H 2,78 | N 2,33 | F 55,47 |

### c) 1,4,7-Tris(carboxylatomethyl)-10-[(3-aza-4-oxo-hexan-5-yl)-säure-N-(2,3-dihydroxypropyl)-N-(1H, 1H, 2H, 2H, 4H, 4H, 5H, 5H-3-oxa)-perfluortridecyl)-amid]-1,4,7,10-tetraazacyclododecan, Gadoliniumkomplex

10 g (15,88 mmol) vom Gadoliniumkomplex der 10-[1-(carboxymethylcarboamoyl)-ethyl]-1,4,7,10-tetraazacyciododecan-1,4,7-triessigsäure und 1.35 g (31,76 mmol) Lithiumchlorid werden bei 60 °C in 100 ml Dimethylsulfoxid gelöst. Man kühlt auf 15 °C ab und gibt 9,21 g (15,88 mmol) der Titelverbindung aus Beispiel 2b zu. Man rührt 10 Minuten und gibt dan 7,42 g (30 mmol) 2-Ethoxy-1-ethoxycarbonyl-1,2-dihydrochinolin zu. Es wird 12 Stunden bei Raumtemperatur gerührt. Man gießt die Lösung in eine Mischung aus 200 ml Aceton /1300 ml Diethylether und rührt 2 Stunden bei Raumtemperatur. Man filtriert den ausgefallenen Niederschlag ab. löst ihn in einer Mischung aus wenig Ethanol/Wasser und chromatographiert an Kieselgel RP-18 (Laufmittel: Gradient aus Tetrahydrofuran/Acetonitril/Wasser).
Ausbeute: 16,09 g (85 % d. Th.) eines farblosen, amorphen Pulvers
Wassergehalt: 6,3 %

| Elementaranalyse (berechnet auf wasserfreie Substanz): | | | | | |
|---|---|---|---|---|---|
| ber. | C 34,26 | H 3,64 | N 7,05 | F 27,10 | Gd 13,19 |
| gef. | C 34,12 | H 3,83 | N 6,91 | F 26.88 | Gd 12.93 |

### Beispiel 3

### a) 2H, 2H, 4H, 4H, 5H, 5H-3-oxa-perfluortridecansäure-N-(5-hydroxy-3-oxa-pentyl)-amid

Zu 30 g (57,45 mmol) 2H, 2H, 4H, 4H, 5H, 5H-3-oxa-pertluortridecansäure in 300 ml Dichlormethan gibt man 8,90 g (70 mmol) Oxalylchlorid und rührt 12 Stunden bei Raumtemperatur. Es wird im Vakuum zur Trockne eingedampft. Der Rückstand wird in 100 ml Dichlormethan gelöst und bei 0°C zu einer Lösung aus 6,25 g (60 mmol)
5-Hydroxy-3-oxa-pentylamin und 6.07 g (60 mmol) Triethylamin, gelöst in 200 ml Dichlormethan getropft. Man rührt 3 Stunden bei 0°C, anschließend 6 Stunden bei Raumtemperatur. Man gibt 300 ml 5 %ige aqu. Salzsäure zu und rührt 15 Minuten gut durch. Die organische Phase wird abgetrennt, über Magnesiumsulfat getrocknet und im Vakuum zur Trockne eingedampft. Der Rückstand wird an Kieselgel (Laufmittel: Dichlormethan/Aceton= 15:1) chromatographiert.
Ausbeute: 32,20 g (92 % d. Th.) eines farblosen Feststoffes

| Elementaranalyse: | | | | |
|---|---|---|---|---|
| ber. | C 31,54 | H 2,65 | N 2,30 | F 53,01 |
| gef. | C 31,61 | H 2,84 | N 2,14 | F 52,85 |

### b) N-(5-Hydroxy-3-oxa-pentyl)-N-(1H, 1H, 2H, 2H, 4H, 4H, 5H, 5H-3-oxa-perfluortridecyl)-amin

30 g (49.24 mmol) der Titelverbindung aus Beispiel 3a werden in 300 ml Tetrahydrofuran gelöst und 31 ml 10 M Borandimethylsulfid (in Tetrahydrofuran) zugegeben. Man kocht 16 Stunden unter Rückfluß. Es wird auf 0°C abgekühlt und 200 ml Methanol zugetropft, anschließend wird im Vakuum zur Trockne eingedampft. Der Rückstand wird in einer Mischung aus 300 ml Ethanol/50 ml 10 %iger aqu. Salzsäure aufgenommen und 10 Stunden bei 50°C gerührt. Man dampft im Vakuum zur Trockne ein, nimmt den Rückstand in 300 ml 5 %iger aqu. Natronlauge auf und extrahiert 3 Mal mit je 300 ml Dichlormethan. Die organischen Phasen werden über Magnesiumsulfat getrocknet, im Vakuum zur Trockne eingedampft und der Rückstand an Kieselgel chromatographiert (Laufmittel: Dichlormethan/2-Propanol= 20:1).
Ausbeute: 26,09 g (89 % d. Th.) eines farblosen Feststoffes

| Elementaranalyse: | | | | |
|---|---|---|---|---|
| ber. | C 32,28 | H 3,05 | N 2.35 | F 54,25 |
| gef. | C 32,12 | H 3,21 | N 2,18 | F 54,09 |

### c) 1,4,7-Tris(carboxylatomethyl)-10-[(3-aza-4-oxo-hexan-5-yl)-säure-N-(5-hydroxy-3-oxa-pentyl)-N-(1H, 1H, 2H, 2H, 4H, 4H, 5H, 5H-3-oxa)-perfluortridecyl)-amid]-1,4,7,10-tetraazacyclododecan, Gadoliniumkomplex

10 g (15,88 mmol) vom Gadoliniumkomplex der 10-[1-(carboxymethylcarboamoyl)-ethyl]-1,4,7,10-tetraazacyclododecan-1,4,7-triessigsäure und 1,35 g (31,76 mmol) Lithiumchlorid werden bei 60 °C in 100 ml Dimethylsulfoxid gelöst. Man kühlt auf 15 °C ab und gibt 9,45 g (15,88 mmol) der Titelverbindung aus Beispiel 3b zu. Man rührt 10 Minuten und gibt dan 7,42 g (30 mmol) 2-Ethoxy-1-ethoxycarbonyl-1,2-dihydrochinolin zu. Es wird 12 Stunden bei Raumtemperatur gerührt. Man gießt die Lösung in eine Mischung aus 200 ml Aceton /1300 ml Diethylether und rührt 2 Stunden bei Raumtemperatur. Man filtriert den ausgefallenen Niederschlag ab. löst ihn in einer Mischung aus wenig Ethanol/Wasser und chromatographiert an Kieselgel RP-18 (Laufmittel: Gradient aus Tetrahydrofuran/Acetonitril/Wasser).
Ausbeute: 16.10 g (84 % d. Th.) eines farblosen, amorphen Pulvers
Wassergehalt: 5,7 %

| Elementaranalyse (berechnet auf wasserfreie Substanz): | | | | | |
|---|---|---|---|---|---|
| ber. | C 34,83 | H 3,84 | N 6,96 | F 26,76 | Gd 13,03 |
| gef. | C 34,65 | H 3,96 | N 6,84 | F 26,62 | Gd 12.91 |

### Beispiel 4

### a) 2H, 2H, 4H, 4H, 5H, 5H-3-oxa-perfluortridecansäure-N-(2-hydroxyethyl)-amid

Zu 30 g (57,45 mmol) 2H, 2H, 4H, 4H, 5H, 5H-3-oxa-perfluortridecansäure in 300 ml Dichlormethan gibt man 8,90 g (70 mmol) Oxalylchlorid und rührt 12 Stunden bei Raumtemperatur. Es wird im Vakuum zur Trockne eingedampft. Der Rückstand wird in 100 ml Dichlormethan gelöst und bei 0°C zu einer Lösung aus 3,66 g (60 mmol) 2-Aminoethanol und 6,07 g (60 mmol) Triethylamin, gelöst in 200 ml Dichlormethan getropft. Man rührt 3 Stunden bei 0°C, anschließend 6 Stunden bei Raumtemperatur. Man gibt 300 ml 5 %ige aqu. Salzsäure zu und rührt 15 Minuten gut durch. Die organische Phase wird abgetrennt, über Magnesiumsulfat getrocknet und im Vakuum zur Trockne eingedampft. Der Rückstand wird an Kieselgel (Laufmittel: Dichlormethan/Aceton= 20:1) chromatographiert.
Ausbeute: 28,90 g (89 % d. Th.)

| Elementaranalyse: | | | | |
|---|---|---|---|---|
| ber. | C 29,75 | H 2,14 | N 2,48 | F 57,14 |
| gef. | C 29,61 | H 2,29 | N 2,37 | F 57,01 |

### b) N-(2-Hydroxyethyl)-N-(1H, 1H, 2H, 2H, 4H, 4H, 5H, 5H-3-oxa)-perfluortridecyl)-amin

28 g (49,54 mmol) der Titelverbindung aus Beispiel 4a werden in 300 ml Tetrahydrofuran gelöst und 31 ml 10 M Borandimethylsulfid (in Tetrahydrofuran) zugegeben. Man kocht 16 Stunden unter Rückfluß. Es wird auf 0°C abgekühlt und 200 ml Methanol zugetropft, anschließend wird im Vakuum zur Trockne eingedampft. Der Rückstand wird in einer Mischung aus 300 ml Ethanol/50 ml 10 %iger aqu. Salzsäure aufgenommen und 10 Stunden bei 50°C gerührt. Man dampft im Vakuum zur Trockne ein, nimmt den Rückstand in 300 ml 5 %iger aqu. Natronlauge auf und extrahiert 3 mal mit je 300 ml Dichlormethan. Die organischen Phasen werden über Magnesiumsulfat getrocknet, im Vakuum zur Trockne eingedampft und der Rückstand an Kieselgel chromatographiert (Laufmittel: Dichlormethan/2 Propanol= 15:1).
Ausbeute: 25.12 g (92 % d. Th.) eines farblosen Feststoffes

| Elementaranalyse (berechnet auf wasserfreie Substanz): | | | | |
|---|---|---|---|---|
| ber. | C 30,50 | H 2,56 | N 2,54 | F 58,59 |
| gef. | C 30.32 | H 2.71 | N 2,48 | F 58,43 |

### c) 1,4,7-Tris(carboxylatomethyl)-10-[(3-aza-4-oxo-hexan-5-yl)-säure-N-(2-hydroxyethyl)-N-(1H, 1H, 2H, 2H, 4H, 4H, 5H, 5H-3-oxa)-perfluortridecyl)-amin-amid]-1,4,7,10-tetraazacyclododecan, Gadoliniumkomplex

10 g (15.88 mmol) vom Gadoliniumkomplex der 10-[1-(carboxymethylcarboamoyl)-ethyl]-1,4,7,10-tetraazacyclododecan-1,4,7-triessigsäure und 1,35 g (31,76 mmol) Lithiumchlorid werden bei 60 °C in 100 ml Dimethylsulfoxid gelöst. Man kühlt auf 15 °C ab und gibt 8,75 g (15,88 mmol) der Titelverbindung aus Beispiel 4b zu. Man rührt 10 Minuten und gibt dan 7,42 g (30 mmol) 2-Ethoxy-1-ethoxycarbonyl-1,2-dihydrochinolin zu. Es wird 12 Stunden bei Raumtemperatur gerührt. Man gießt die Lösung in eine Mischung aus 200 ml Aceton /1300 ml Diethylether und rührt 2 Stunden bei Raumtemperatur. Man filtriert den ausgefallenen Niederschlag ab. löst ihn in einer Mischung aus wenig Ethanol/Wasser und chromatographiert an Kieselgel RP-18 (Laufmittel: Gradient aus Tetrahydrofuran/Acetonitril/Wasser).
Ausbeute: 16.81 g (91 % d. Th.) eines farblosen, amorphen Pulvers
Wassergehalt: 7,2 %

| Elementaranalyse (berechnet auf wasserfreie Substanz): | | | | | |
|---|---|---|---|---|---|
| ber. | C 34,08 | H 3,64 | N 7,23 | F 27,77 | Gd 13.52 |
| gef. | C 33.91 | H 3.82 | N 7,14 | F 27,58 | Gd 13,41 |

### Beispiel 5

### a) 2H, 2H, 4H, 4H, 5H, 5H-3-oxa-perfluortridecansäureamid

Zu 30 g (57,45 mmol) 2H, 2H, 4H, 4H, 5H, 5H-3-oxa-pertiuortridecansäure in 300 ml Dichlormethan gibt man 8,90 g (70 mmol) Oxalylchlorid und rührt 12 Stunden bei Raumtemperatur. Es wird im Vakuum zur Trockne eingedampft. Der Rückstand wird in 200 ml Dichlormethan gelöst. Dann wird bei 0°C Ammoniakgas für ca. 2 Stunden in die Lösung geleitet. Man rührt 4 Stunden bei 0°C nach, anschließend 2 Stunden bei Raumtemperatur. Man gibt 300 ml 5 %ige aqu. Salzsäure zu und rührt 15 Minuten gut durch. Die organische Phase wird abgetrennt, über Magnesiumsulfat getrocknet und im Vakuum zur Trockne eingedampft. Der Rückstand wird an Kieselgel (Laufmittel: Dichlormethan/Aceton= 20:1) chromatographiert.
Ausbeute: 27,85 g (93 % d. Th.)

| Elementaranalyse: | | | | |
|---|---|---|---|---|
| ber. | C 27,66 | H 1,55 | N 2,69 | F 61,97 |
| gef. | C 27,49 | H 1,72 | N 2,54 | F 61,81 |

### b) 1H, 1H, 2H, 2H, 4H, 4H, 5H, 5H-3-oxa-perfluortridecylamin, Hydrochlorid

27 g (51,8 mmol) der Titelverbindung aus Beispiel 5a werden in 300 ml Tetrahydrofuran gelöst und 31 ml 10 M Borandimethylsulfid (in Tetrahydrofuran) zugegeben. Man kocht 16 Stunden unter Rückfluß. Es wird auf 0°C abgekühlt und 200 ml Methanol zugetropft, anschließend wird im Vakuum zur Trockne eingedampft. Der Rückstand wird in einer Mischung aus 400 ml Ethanol/100 ml 10 %iger aqu. Salzsäure aufgenommen und 8 Stunden bei 60°C gerührt. Man dampft im Vakuum zur Trockne ein und kristallisiert den Rückstand aus wenig Ethanol/Diethylether um.
Ausbeute: 26,75 g (95 % d. Th.) eines farblosen, kristallinen Feststoffes

| Elementaranalyse: | | | | | |
|---|---|---|---|---|---|
| ber. | C 26,51 | H 2,04 | N 2,58 | F 59,41 | Cl 6,52 |
| gef. | C 26,37 | H 2,21 | N 2,46 | F 59,25 | CI. 6,38 |

### c) 3,6,9,12,15-Pentaoxahexadecansäure-N-(1H, 1H, 2H, 2H, 4H, 4H, 5H, 5H-3-oxa)-perfluortridecyl)-amid

Zu 26,5 g (48,74 mmol) der Titelverbindung aus Beispiel 5b und 14,8 g (146,2 mmol) Triethylamin, gelöst in 300 ml Dichlormethan tropft, gibt man bei 0°C 14,24 g (50 mmol) 3,6,9,12,15-Pentaoxahexadecansäurechlorid und rührt 3 Stunden bei 0°C. Man gibt 300 ml 5 % ige aqu. Salzsäure zu und rührt 30 Minuten gut durch. Die organische Phase wird abgetrennt, über Magnesiumsulfat getrocknet und im Vakuum zur Trockne eingedampft. Der Rückstand wird an Kieselgel chromatographiert (Laufmittel: Dichlormethan/Aceton: 20:1).
Ausbeute: 32.03 g (87 % d. Th.) eines farblosen Öls

| Elementaranalyse: | | | | |
|---|---|---|---|---|
| ber. | C 36,57 | H 4,00 | N 1,85 | F 42,75 |
| gef. | C 36,46 | H 4,12 | N 1,76 | F 42,53 |

### d) N-(3,6,9,12,15-Pentaoxahexadecyt)-N-(1H, 1H, 2H, 2H, 4H, 4H-3-oxa)-perfluortridecyl)-amin

31 g (41,03 mmol) der Titelverbindung aus Beispiel 5c werden in 300 ml Tetrahydrofuran gelöst und 25 ml 10 M Borandimethylsulfid (in Tetrahydrofuran) zugegeben. Man kocht 16 Stunden unter Rückfluß. Es wird auf 0°C abgekühlt und 200 ml Methanol zugetropft, anschließend wird im Vakuum zur Trockne eingedampft. Der Rückstand wird in einer Mischung aus 300 ml Ethanol/50 ml 10 %iger aqu. Salzsäure aufgenommen und 8 Stunden bei 40°C gerührt. Man dampft im Vakuum zur Trockne ein, nimmt den Rückstand in 300 ml 5 %iger aqu. Natronlauge auf und extrahiert 3 mal mit je 300 ml Dichlormethan. Die organischen Phasen werden über Magnesiumsulfat getrocknet, im Vakuum zur Trockne eingedampft und der Rückstand an Kieselgel chromatographiert (Laufmittel: Dichlormethan/2 Propanol= 15:1).
Ausbeute: 27,68 g (91 % d. Th.)

| Elementaranalyse: | | | | |
|---|---|---|---|---|
| ber. | C 37.26 | H 4.35 | N 1.89 | F 43.56 |
| gef. | C 37.11 | H 4,51 | N 1,73 | F 43,41 |

### e) 1,4,7-Tris(carboxylatomethyl)-10-{(3-aza-4-oxo-hexan-5-yl)-säure-[N-3,6,9,12.15-pentaoxa)-hexadexyl)-N-(1H, 1H. 2H, 2H, 4H, 4H, 5H, 5H-3-oxa)-perfluortridecyl]-amid}-1,4,7,10-tetraazacyclododecan, Gadoliniumkomplex

10 g (15,88 mmol) vom Gadoliniumkomplex der 10-[1-(carboxymethylcarboamoyl)-ethyl]-1,4,7,10-tetraazacyclododecan-1,4,7-triessigsäure und 1,35 g (31,76 mmol) Lithiumchlorid werden bei 60 °C in 100 ml Dimethylsulfoxid gelöst. Man kühlt auf 15 °C ab und gibt 11,77 g (15,88 mmol) der Titelverbindung aus Beispiel 5d zu. Man rührt 10 Minuten und gibt dan 7,42 g (30 mmol) 2-Ethoxy-1-ethoxycarbonyl-1,2-dihydrochinolin zu. Es wird 12 Stunden bei Raumtemperatur gerührt. Man gießt die Lösung in eine Mischung aus 200 ml Aceton /1300 ml Diethylether und rührt 2 Stunden bei Raumtemperatur. Man filtriert den ausgefallenen Niederschlag ab, löst ihn in einer Mischung aus wenig Ethanol/Wasser und chromatographiert an Kieselgel RP-18 (Laufmittel: Gradient aus Tetrahydrofuran/Acetonitril/Wasser).
Ausbeute: 18,05 g (84 % d. Th.) eines farblosen. amorphen Pulvers
Wassergehalt: 6.2 %

| Elementaranalyse (berechnet auf wasserfreie Substanz): | | | | | |
|---|---|---|---|---|---|
| ber. | C 37,28 | H 4,47 | N 6,21 | F 23,87 | Gd 11,62 |
| gef. | C 37,11 | H 4,61 | N 6,03 | F 23,64 | Gd 11,42 |

### Beispiel 6

### a) 1,4,7-Tris(carboxylatomethyl)-10-[(3-aza-4-oxo-hexan-5-yl)-säure-N-(13-amino-4,7,13-trioxa-decyl)-amid]-1,4,7,10-tetraazacyclododecan, Gadoliniumkomplex

10 g (15,88 mmol) vom Gadoliniumkomplex der 10-[1-(carboxymethylcarboamoyl)-ethyl]-1,4,7,10-tetraazacyclododecan-1,4,7-triessigsäure und 1,35 g (31,76 mmol) Lithiumchlorid und 3,66 g (31,76 mmol) N-Hydroxysuccinimid werden bei 60 °C in 100 ml Dimethylsulfoxid gelöst. Man kühlt auf 15 °C ab und gibt 3.51 g (17 mmol) N,N'-Dicyclohexylcarbodiimid zu und rührt 5 Stunden bei 15 °C. Zur Abtrennung des Harnstoffes wird die Lösung filtriert. Zum Filtrat gibt man 14,66 g (60 mmol) 1,13-Diamino-4,7,13-trioxadecan und 2,02 g (20 mmol) Triethylamin zu und rührt 12 Stunden bei Raumtemperatur. Man gießt die Lösung in 1500 ml Diethylether/50 ml n-Butanol und rührt 30 Minuten. Der ausgefallene Feststoff wird abfiltriert und an Kieselgel RP-18 chromatographiert (Laufmittel: Gradient aus Tetrahydrofuran/ Acetonitril/Wasser).
Ausbeute: 12,66 g (69 % d. Th.) eines farblosen, amorphen Pulvers
Wassergehalt: 3,5 %

| Elementaranalyse (berechnet auf wasserfreie Substanz): | | | | | |
|---|---|---|---|---|---|
| ber. | C 30,16 | H 4,54 | N 8,49 | F 27,96 | Gd 13,61 |
| gef. | C 30,02 | H 4,68 | N 8,35 | F 27,81 | Gd 13,45 |

### b) 1,4,7-Tris(carboxylatomethyl)-10-{(3-aza-4-oxo-hexan-5-yl)-säure-[N-4,7,10,17-tetraoxa-14-aza-17-oxo-C₂₀-C₂₈-hepta-decafluor)-heptacosyl]-amid}-1,4,7,10-tetraazacyclododecan, Gadoliniumkomplex

11,3 g (21,64 mmol) 2H, 2H, 4H, 4H, 5H, 5H-3-oxa-perfluortridecansäure, 0,85 g (20 mmol) Lithiumchlorid und 4,95 g (43 mmol) N-Hydroxysuccinimid werden bei 25 °C in 150 ml Dimethylsulfoxid gelöst. Man kühlt auf 15 °C ab und gibt 6.19 g (30 mmol) N,N'-Dicyclohexylcarbodiimid zu und rührt 5 Stunden bei 15 °C. Zur Abtrennung des Harnstoffes wird die Lösung filtriert. Zum Filtrat gibt man 12,5 g (10,82 mmol) der Titelverbindung aus Beispiel 6a und 3,29 g (32,47 mmol) Triethylamin zu und rührt 12 Stunden bei Raumtemperatur. Man gießt die Lösung in 1300 ml Diethylether/100 ml Aceton und rührt 30 Minuten. Der ausgefallene Feststoff wird abfiltriert und an Kieselgel RP-18 chromatographiert (Laufmittel: Gradient aus Tetrahydrofuran/ Acetonitril/Wasser).
Ausbeute: 13,01 g (90 % d. Th.)
Wassergehalt: 6,7 %

| Elementaranalyse (berechnet auf wasserfreie Substanz): | | | | | |
|---|---|---|---|---|---|
| ber. | C 36,86 | H 4,30 | N 7.34 | F 24.17 | Gd 11.77 |
| gef. | C 36,68 | H 4,41 | N 7,25 | F 24,03 | Gd 11.55 |

### Beispiel 7

### 1,4,7-Tris(carboxylatomethyl)-10-[(3-aza-4-oxo-hexan-5-yl)-säure-N-(1H, 1H, 2H, 2H, 4H, 4H, 5H, 5H-3-oxa-perfluortridecyl)-amid]-1,4,7,10-tetraazacyclododecan, Gadoliniumkomplex

10 g (15,88 mmol) vom Gadoliniumkomplex der 10-[1-(carboxymethylcarboamoyl)-ethyl]-1,4,7,10-tetraazacyclododecan-1,4,7-triessigsäure und 1,35 g (31,76 mmol) Lithiumchlorid und 3,66 g (31,76 mmol) N-Hydroxysuccinimid werden bei 60 °C in 100 ml Dimethylsulfoxid gelöst. Man kühlt auf 15 °C ab und gibt 3,51 g (17 mmol) N,N'-Dicyclohexylcarbodiimid zu und rührt 5 Stunden bei 15 °C. Zur Abtrennung des Harnstoffes wird die Lösung filtriert. Zum Filtrat gibt man 8,63 g (15,88 mmol) der Titelverbindung aus Beispiel 5b und 5,06 g (50 mmol) Triethylamin zu und rührt 12 Stunden bei Raumtemperatur. Man gießt die Lösung in 1500 ml Diethylether/100 ml Aceton und rührt 30 Minuten. Der ausgefallene Feststoff wird abfiltriert und an Kieselgel RP-18 chromatographiert (Laufmittel: Gradient aus Tetrahydrofuran/ Acetonitril/Wasser).
Ausbeute: 13,86 g (78 % d. Th.) eines farblosen, amorphen Pulvers
Wassergehalt: 9,3 %

| Elementaranalyse (berechnet auf wasserfreie Substanz): | | | | | |
|---|---|---|---|---|---|
| ber. | C 33,28 | H 3,42 | N 7,51 | F 28,87 | Gd 14,05 |
| gef. | C 33,12 | H 3,61 | N 7.37 | F 28.69 | Gd 13.89 |

### Beispiel 8

### a) 2H, 2H, 4H, 4H, 5H, 5H-3-oxa-perfluortridecansäure-N-(2,3,4,5,6-pentahydroxy)-hexylamid

Zu 30 g (57,45 mmol) 2H, 2H, 4H, 4H. 5H, 5H-3-oxa-perfluortridecansäure in 300 ml Dichlormethan gibt man 8,90 g (70 mmol) Oxalylchlorid und rührt 12 Stunden bei Raumtemperatur. Es wird im Vakuum zur Trockne eingedampft. Der Rückstand wird in 100 ml Dichlormethan gelöst und bei 0°C zu einer Lösung aus 10,87 g (60 mmol) Glucamin und 6,07 g (60 mmol) Triethylamin, gelöst in 150 ml Dichlormethan/150 ml Dioxan getropft. Man rührt 3 Stunden bei 0°C, anschließend 8 Stunden bei Raumtemperatur. Man gibt 400 ml 5 %ige aqu. Salzsäure zu und rührt 15 Minuten gut durch. Die organische Phase wird abgetrennt, über Magnesiumsulfat getrocknet und im Vakuum zur Trockne eingedampft. Der Rückstand wird an Kieselgel (Laufmittel: Dichlormethan/Methanol= 5:1) chromatographiert.
Ausbeute: 30,71 g (78 % d. Th.)

| Elementaranalyse: | | | | |
|---|---|---|---|---|
| ber. | C 31,55 | H 2,94 | N 2,04 | F 47,13 |
| gef. | C 31,44 | H 3,09 | N 1,97 | F 47,01 |

### b) N-(2,3,4,5,6-pentahydroxyhexyl)-N-1H, 1H, 2H, 2H, 4H, 4H, 5H, 5H-3-oxa-perfluortridecyl)-amin

30 g (43,77 mmol) der Titelverbindung aus Beispiel 8a werden in 300 ml Tetrahydrofuran gelöst und 50 ml 10 M Borandimethylsulfid (in Tetrahydrofuran) zugegeben. Man kocht 48 Stunden unter Rückfluß. Es wird auf 0°C abgekühlt und 500 ml Methanol zugetropft, anschließend wird im Vakuum zur Trockne eingedampft. Der Rückstand wird in einer Mischung aus 500 ml Ethanol/100 ml 10 %iger aqu. Salzsäure aufgenommen und 15 Stunden bei 60°C gerührt. Man dampft im Vakuum zur Trockne ein, nimmt den Rückstand in 400 ml 5 %iger aqu. Natronlauge auf und extrahiert 5 mal mit je 400 ml Chloroform. Die organischen Phasen werden über Magnesiumsulfat getrocknet, im Vakuum zur Trockne eingedampft und der Rückstand an Kieselge chromatographiert (Laufmittel: Dichlormethan/Methanol= 3:1).
Ausbeute: 19.69 g (67 % d. Th.) eines farblosen Feststoffes

| Elementaranalyse: | | | | |
|---|---|---|---|---|
| ber. | C 32,20 | H 3,30 | N 2,09 | F 48.11 |
| gef. | C 32.05 | H 3,43 | N 1,97 | F 47,93 |

### c) 1,4,7-Tris(carboxylatomethyl)-10-{(3-aza-4-oxo-hexan-5-yl)-säure-(N-2,3,5,6-penthydroxy)-hexyl-N-(1H, 1H, 2H, 2H, 4H, 4H, 5H, 5H-3-oxa-perfluortridecyl]-amid}-1,4,7,10-tetraazacyclododecan, Gadoliniumkomplex

10 g (15.88 mmol) vom Gadoliniumkomplex der 10-[1-(carboxymethylcarboamoyl)-ethyl]-1,4,7,10-tetraazacyclododecan-1,4,7-triessigsäure und 1.35 g (31,76 mmol) Lithiumchlorid werden bei 60 °C in 100 ml Dimethylsulfoxid gelöst. Man kühlt auf 15 °C ab und gibt 15,88 g (15,88 mmol) der Titelverbindung aus Beispiel 8b zu. Man rührt 10 Minuten und gibt dan 7,42 g (30 mmol) 2-Ethoxy-1-ethoxycarbonyl-1,2-dihydrochinolin zu. Es wird 12 Stunden bei Raumtemperatur gerührt. Man gießt die Lösung in eine Mischung aus 200 ml Aceton /1300 ml Diethylether und rührt 2 Stunden bei Raumtemperatur. Man filtriert den ausgefallenen Niederschlag ab, löst ihn in einer Mischung aus wenig Ethanol/Wasser und chromatographiert an Kieselgel RP-18 (Laufmittel: Gradient aus Tetrahydrofuran/Acetonitril/Wasser).
Ausbeute: 16,10 g (79 % d. Th.) eines farblosen, amorphen Pulvers
Wassergehalt: 6,3 %

| Elementaranalyse (berechnet auf wasserfreie Substanz): | | | | | |
|---|---|---|---|---|---|
| ber. | C 36,64 | H 3,93 | N 6,55 | F 25,17 | Gd 12,26 |
| gef. | C 34,49 | H 4,13 | N 6,48 | F 25,03 | Gd 12,11 |

### Beispiel 9

### a) 2H, 2H, 4H, 4H, 5H, 5H-3-oxa-perfluortridecansäure-N-(2,2-dimethyl-5-hydroxy-1,3-dioxepan-6-yl)-amid

Zu 30 g (57,45 mmol) 2H, 2H, 4H, 4H, 5H, 5H-3-oxa-perfluortridecansäure in 300 ml Dichlormethan gibt man 8,90 g (70 mmol) Oxalylchlorid und rührt 12 Stunden bei Raumtemperatur. Es wird im Vakuum zur Trockne eingedampft. Der Rückstand wird in 100 ml Dichlormethan gelöst und bei 0°C zu einer Lösung aus 9,67 (60 mmol) 5-Amino-2,2-dimethyl-1,3-dioxepan-6-ol und 6,07 g (60 mmol) Triethylamin, gelöst in 200 ml Dichlormethan getropft . Man rührt 3 Stunden bei 0°C, anschließend 5 Stunden bei Raumtemperatur. Man gibt 300 ml Wasser zu und rührt 15 Minuten gut durch. Die organische Phase wird abgetrennt, über Magnesiumsulfat getrocknet und im Vakuum zur Trockne eingedampft. Der Rückstand wird an Kieselgel (Laufmittel: Dichlormethan/Aceton= 15:1) chromatographiert.
Ausbeute: 27,62 g (85 % d. Th.)

| Elementaranalyse: | | | | |
|---|---|---|---|---|
| ber. | C 34,30 | H 3,03 | N 2,11 | F 48,54 |
| gef. | C 34,15 | H 3,19 | N 2,04 | F 48,37 |

### b) N-(1-Hydroxymethyl-2,3-dihydroxypropyl)-N-(1H, 1H, 2H, 2H. 4H, 4H, 5H, 5H-3-oxa-perfluortridecyl)-amin

27 g (40,58 mmol) der Titelverbindung aus Beispiel 9a werden in 300 ml Tetrahydrofuran gelöst und 26 ml 10 M Borandimethylsulfid (in Tetrahydrofuran) zugegeben. Man kocht 20 Stunden unter Rückfluß. Es wird auf 0°C abgekühlt und 300 ml Methanol zugetropft, anschließend wird im Vakuum zur Trockne eingedampft. Der Rückstand wird in einer Mischung aus 300 ml Ethanol/100 ml 10 %iger aqu. Salzsäure aufgenommen und 6 Stunden bei 60°C gerührt. Man dampft im Vakuum zur Trockne ein, nimmt den Rückstand in 400 ml 5 %iger aqu. Natronlauge auf und extrahiert 5 mal mit je 250 ml Chloroform. Die organischen Phasen werden über Magnesiumsulfat getrocknet, im Vakuum zur Trockne eingedampft und der Rückstand an Kieselgel chromatographiert (Laufmittel: Dichlormethan/Methanol= 6:1). Ausbeute: 20.09 g (81 % d. Th.) eines farblosen Feststoffes

| Elementaranalyse: | | | | |
|---|---|---|---|---|
| ber. | C 31,44 | H 2,97 | N 2,29 | F 52,83 |
| gef. | C 31,26 | H 3,11 | N 2,18 | F 52,67 |

### c) 1,4,7-Tris(carboxylatomethyl)-10-{(3-aza-4-oxo-hexan-5-yl)-säure-[N-1-hydroxymethyl-2,3-dihydroxypropyl)-N-(1H, 1H, 2H, 2H, 4H, 4H, 5H, 5H-3-oxa-perfluortridecyl]-amid}-1,4,7,10-tetraazacyclododecan, Gadoliniumkomplex

10 g (15.88 mmol) vom Gadoliniumkomplex der 10-[1-(carboxymethylcarboamoyl)-ethyl]-1,4,7,10-tetraazacyclododecan-1,4,7-triessigsäure und 1,35 g (31,76 mmol) Lithiumchlorid werden bei 60 °C in 100 ml Dimethylsulfoxid gelöst. Man kühlt auf 15 °C ab und gibt 9,71 g (15,88 mmol) der Titelverbindung aus Beispiel 9b zu. Man rührt 10 Minuten und gibt dan 7,42 g (30 mmol) 2-Ethoxy-1-ethoxycarbonyl-1,2-dihydrochinolin zu. Es wird 12 Stunden bei Raumtemperatur gerührt. Man gießt die Lösung in eine Mischung aus 200 ml Aceton /1300 ml Diethylether und rührt 2 Stunden bei Raumtemperatur. Man filtriert den ausgefallenen Niederschlag ab, löst ihn in einer Mischung aus wenig Ethanol/Wasser und chromatographiert an Kieselgel RP-18 (Laufmittel: Gradient aus Tetrahydrofuran/Acetonitril/Wasser).
Ausbeute: 13,40 g (69 % d. Th.) eines farblosen, amorphen Pulvers
Wassergehalt: 9,1 %

| Elementaranalyse (berechnet auf wasserfreie Substanz): | | | | | |
|---|---|---|---|---|---|
| ber. | C 34,37 | H 3,79 | N 6,87 | F 24,41 | Gd 12,86 |
| gef. | C 34,18 | H 3,95 | N 6,71 | F 24,25 | Gd 12,70 |

### Beispiel 10

### a) Perfluoroctylsulfonsäure-N-[(2-benzyloxycarbonylamino)-ethyl]-amid

40 g (173,4 mmol) 1-Benzyloxycarbonylamino-2-aminoethan, Hydrochlorid, 87,1 g (173,4 mmol) Perfluoroctylsulfofluorid und 35.42 g (350 mmol) Triethylamin werden 10 Stunden auf 80°C erwärmt. Man kühlt auf Raumtemperatur ab und gibt direkt auf eine Kieselgelsäule zur chromatographischen Aufreinigung (Laufmittel: Dichlormethan/Aceton= 20:1).
Ausbeute: 42,22 g (36 % d. Th.) eines farblosen Feststoffes

| Elementaranalyse: | | | | | |
|---|---|---|---|---|---|
| ber. | C 31,97 | H 1,94 | N 4,14 | F 47,75 | S 4,74 |
| gef. | C 31,83 | H 2,11 | N 4,03 | F 47,63 | S 4,63 |

### b) Perfluoroctylsulfonsäure-N-[(2-amino)-ethyl]-amid

30 g (44,36 mmol) der Titelverbindung aus Beispiel 10a werden in 300 ml Methanol gelöst und 5 g Palladiumkatalysator (10 % Pd/C) zugegeben, man hydriert über Nacht bei Raumtemperatur. Man filtriert zum Katalysator ab und dampft das Filtrat im Vakuum zur Trockne ein.
Ausbeute: 24.05 g (quantitativ) eines farblosen Feststoffes

| Elementaranalyse: | | | | |
|---|---|---|---|---|
| ber. | C 22,15 | H 1,30 | N 5,17 | F 59,57 |
| gef. | C 22,04 | H 1,41 | N 5,05 | F 59,62 |

### c) 1,4,7-Tris(carboxylatomethyl)-10-{(3-aza-4-oxo-hexan-5-yl)-säure-N-[(2-perfluoroctylsulfonylamino)-ethyl]-amid}-1,4,7,10-tetraazacyclododecan, Gadoliniumkomplex

10 g (15,88 mmol) vom Gadoliniumkomplex der 10-[1-(carboxymethylcarboamoyl)-ethyl]-1,4,7,10-tetraazacyclododecan-1,4,7-triessigsäure und 1,35 g (31,76 mmol) Lithiumchlorid und 3,66 g (31,76 mmol) N-Hydroxysuccinimid werden bei 60 °C in 100 ml Dimethylsulfoxid gelöst. Man kühlt auf 15 °C ab und gibt 3,51 g (17 mmol) N,N'-Dicyciohexylcarbodiimid zu und rührt 5 Stunden bei 15 °C. Zur Abtrennung des Harnstoffes wird die Lösung filtriert. Zum Filtrat gibt man 8,61 g (15,88 mmol) der Titelverbindung aus Beispiel 10b und 2,02 g (20 mmol) Triethylamin zu und rührt 12 Stunden bei Raumtemperatur. Man gießt die Lösung in 1500 ml Diethylether/100 ml Aceton und rührt 30 Minuten. Der ausgefallene Feststoff wird abfiltriert und an Kieselgel RP-18 chromatographiert (Laufmittel: Gradient aus Tetrahydrofuran/ Acetonitril/Wasser).
Ausbeute: 15,76 g (86 % d. Th.) eines farblosen, amorphen Pulvers
Wassergehalt: 6,5 %

| Elementaranalyse (berechnet auf wasserfreie Substanz): | | | | | | |
|---|---|---|---|---|---|---|
| ber. | C 30,19 | H 3,06 | N 8,50 | F 27,99 | Gd 13,63 | S 2,78 |
| gef. | C 30,03 | H 3,18 | N 8,41 | F 27,81 | Gd 13,50 | S 2,61 |

### Beispiel 11

### a) 2H, 2H, 4H, 4H, 5H, 5H-3-oxa-perfluortridecansäure-N-(2-benzyloxycarboxylamino-ethyl]-amid

Zu 30 g (57,45 mmol) 2H, 2H, 4H, 4H, 5H, 5H-3-oxa-perfluortridecansäure in 300 ml Dichlormethan gibt man 8,90 g (70 mmol) Oxalylchlorid und rührt 12 Stunden bei Raumtemperatur. Es wird im Vakuum zur Trockne eingedampft. Der Rückstand wird in 100 ml Dichlormethan gelöst und bei 0°C zu einer Lösung aus 13,84 g (60 mmol) 1-Benzyloxycarbonylamin-2-amino-ethan, Hydrochlorid und 12,14 g (120 mmol) Triethylamin, gelöst in 200 ml Dichlormethan getropft. Man rührt 3 Stunden bei 0°C, anschließend 5 Stunden bei Raumtemperatur. Man gibt 300 ml 5 %ige aqu. Salzsäure zu und rührt 15 Minuten gut durch. Die organische Phase wird abgetrennt, über Magnesiumsulfat getrocknet und im Vakuum zur Trockne eingedampft. Der Rückstand wird an Kieselgel (Laufmittel: Dichlormethan/Aceton= 20:1) chromatographiert.
Ausbeute: 33,30 g (83 % d. Th.) eines farblosen Feststoffes

| Elementaranalyse: | | | | |
|---|---|---|---|---|
| ber. | C 37,84 | H 2,74 | N 4,01 | F 46.25 |
| gef. | C 37,67 | H 2,89 | N 3,88 | F 46,11 |

### b) 2H, 2H, 4H, 4H, 5H, 5H-3-oxa-perfluortridecansäure-N-[(2-amino)-ethyl]-amid

30 g (42,96 mmol) der Titelverbindung aus Beispiel 11a werden in 500 ml Methanol gelöst und 5 g Palladiumkatalysator (10 % Pd/C) zugegeben, man hydriert über Nacht bei Raumtemperatur. Man filtriert zum Katalysator ab und dampft das Filtrat im Vakuum zur Trockne ein.
Ausbeute: 24,24 g (quantitativ) eines farblosen Feststoffes

| Elementaranalyse: | | | | |
|---|---|---|---|---|
| ber. | C 29,80 | H 2,32 | N 4,96 | F 57,24 |
| gef. | C 29,67 | H 2,41 | N 4,88 | F 57,15 |

### c) 1,4,7-Tris-(carboxylatomethyl)-10-{(3-aza-4-oxo-hexan-5-yl)-säure-N-[3-aza-6-oxa-4-oxo-(C₉-C₁₆-heptadecafluor)-hexadecyl]-amid}-1,4,7,10-tetraazacyclododecan- Gadoliniumkomplex

10 g (15,88 mmol) vom Gadoliniumkomplex der 10-[1-(carboxymethylcarboamoyl)-ethyl]-1,4,7,10-tetraazacyclododecan-1,4,7-triessigsäure und 1,35 g (31,76 mmol) Lithiumchlorid und 3,66 g (31,76 mmol) N-Hydroxysuccinimid werden bei 60 °C in 100 ml Dimethylsulfoxid gelöst. Man kühlt auf 15 °C ab und gibt 3,51 g (17 mmol) N,N'-Dicyclohexylcarbodiimid zu und rührt 5 Stunden bei 15 °C. Zur Abtrennung des Harnstoffes wird die Lösung filtriert. Zum Filtrat gibt man 8,96 g (15,88 mmol) der Titelverbindung aus Beispiel 11b und 2,02 g (20 mmol) Triethylamin zu und rührt 12 Stunden bei Raumtemperatur. Man gießt die Lösung in 1500 ml Diethylether/100 ml Aceton und rührt 30 Minuten. Der ausgefallene Feststoff wird abfiltriert und an Kieselgel RP-18 chromatographiert (Laufmittel: Gradient aus Tetrahydrofuran/ Acetonitrit/Wasser).
Ausbeute: 15,31 g (82 % d. Th.) eines farblosen, amorphen Pulvers Wassergehalt: 6.3 %

| Elementaranalyse (berechnet auf wasserfreie Substanz): | | | | | |
|---|---|---|---|---|---|
| ber. | C 33,71 | H 3,51 | N 8,34 | F 27,46 | Gd 13.37 |
| gef. | C 33.61 | H 3.63 | N 8.17 | F 27.31 | Gd 13.20 |

### Beispiel 12

### a) 2H, 2H, 4H, 4H, 5H, 5H-3-oxa-perfluorundecansäure-N-[(2-hydroxy)-ethyl]-amid

Zu 24.25 g (57.45 mmol) 2H. 2H, 4H. 4H, 5H, 5H-3-oxa-perfluortridecansäure in 300 ml Dichlormethan gibt man 8.90 g (70 mmol) Oxalylchlorid und rührt 12 Stunden bei Raumtemperatur. Es wird im Vakuum zur Trockne eingedampft. Der Rückstand wird in 100 ml Dichlormethan gelöst und bei 0°C zu einer Lösung aus 3,66 g (60 mmol) Ethanolamin und 6,07 g (60 mmol) Triethylamin, gelöst in 200 ml Dichlormethan getropft. Man rührt 3 Stunden bei 0°C, anschließend 6 Stunden bei Raumtemperatur. Man gibt 300 ml 5 %ige aqu. Salzsäure zu und rührt 15 Minuten gut durch. Die organische Phase wird abgetrennt, über Magnesiumsulfat getrocknet und im Vakuum zur Trockne eingedampft. Der Rückstand wird an Kieselgel (Laufmittel: Dichlormethan/Aceton= 20:1) chromatographiert.
Ausbeute: 24,86 g (93 % d. Th.) eines farblosen Feststoffes

| Elementaranalyse: | | | | |
|---|---|---|---|---|
| ber. | C 30,98 | H 2,60 | N 3,01 | F 53,09 |
| gef. | C 30,71 | H 2,81 | N 2,87 | F 52,82 |

### b) N-(2-Hydroxyethyl)-N-1H, 1H, 2H, 2H, 4H, 4H, 5H. 5H-3-oxa-perfluorundecyl)-amin

24 g (51,59 mmol) der Titelverbindung aus Beispiel 12a werden in 300 ml Tetrahydrofuran gelöst und 31 ml 10 M Borandimethylsulfid (in Tetrahydrofuran) zugegeben. Man kocht 12 Stunden unter Rückfluß. Es wird auf 0°C abgekühlt und 200 ml Methanol zugetropft. anschließend wird im Vakuum zur Trockne eingedampft. Der Rückstand wird in einer Mischung aus 300 ml Ethanol/50 ml 10 %iger aqu. Salzsäure aufgenommen und 8 Stunden bei 40°C gerührt. Man dampft im Vakuum zur Trockne ein, nimmt den Rückstand in 300 ml 5 %iger aqu. Natronlauge auf und extrahiert 3 mal mit je 300 ml Dichlormethan. Die organischen Phasen werden über Magnesiumsulfat getrocknet. im Vakuum zur Trockne eingedampft und der Rückstand an Kieselgel chromatographiert (Laufmittel: Dichlormethan/2-Propanol= 20:1).
Ausbeute: 20,95 g (90 % d. Th.) eines farblosen Feststoffes

| Elementaranalyse: | | | | |
|---|---|---|---|---|
| ber. | C 31,94 | H 3,13 | N 3,10 | F 54,73 |
| gef. | C 31,71 | H 3,31 | N 3.01 | F 54,58 |

### c) 1,4,7-Tris-(carboxylatomethyl)-10-{(3-aza-4-oxo-hexan-5-yl)-säure-N-[(2-hydroxy)-ethyl-N-(1H, 1H, 2H, 2H, 4H, 4H, 5H, 5H-3oxa)-perfluorundecyl]-amid}-1,4,7,10-tetraazacyclododecan- Gadoliniumkomplex

10 g (15.88 mmol) vom Gadoliniumkomplex der 10-[1-(carboxymethylcarboamoyl)-ethyl]-1,4,7,10-tetraazacyclododecan-1,4,7-triessigsäure und 1,35 g (31,76 mmol) Lithiumchlorid werden bei 60 °C in 100 ml Dimethylsulfoxid gelöst. Man kühlt auf 15 °C ab und gibt 8.98 g (15,88 mmol) der Titelverbindung aus Beispiel 12b zu. Man rührt 10 Minuten und gibt dan 7,42 g (30 mmol) 2-Ethoxy-1-ethoxycarbonyl-1,2-dihydrochinolin zu. Es wird 12 Stunden bei Raumtemperatur gerührt. Man gießt die Lösung in eine Mischung aus 200 ml Aceton /1300 ml Diethylether und rührt 2 Stunden bei Raumtemperatur. Man filtriert den ausgefallenen Niederschlag ab, löst ihn in einer Mischung aus wenig Ethanol/Wasser und chromatographiert an Kieselgel RP-18 (Laufmittel: Gradient aus Tetrahydrofuran/Acetonitril/Wasser).
Ausbeute: 14,01 g (83 % d. Th.) eines farblosen, amorphen Pulvers

| Elementaranalyse: | | | | | |
|---|---|---|---|---|---|
| ber. | C 35,03 | H 3,98 | N 7,91 | F 23.24 | Gd 14,79 |
| gef. | C 34.85 | H 4,19 | N 7,75 | F 23,05 | Gd 14,58 |

### Beispiel 13

### a) 2H, 2H, 4H, 4H, 5H. 5H-3-oxa-perfluorundecansäure-N-(3,6,9,12-tetraoxatridecyl)-amid

Zu 24,25 g (57,45 mmol) 2H, 2H, 4H, 4H, 5H, 5H-3-oxa-perfluorundecansäure in 300 ml Dichlormethan gibt man 8,90 g (70 mmol) Oxalylchlorid und rührt 12 Stunden bei Raumtemperatur. Es wird im Vakuum zur Trockne eingedampft. Der Rückstand wird in 100 ml Dichlormethan gelöst und bei 0°C zu einer Lösung aus 12,44 g (60 mmol) 3,6,9,12-Tetraoxa-tridecylamin und 6,07 g (60 mmol) Triethylamin, gelöst in 200 ml Dichlormethan, getropft. Man rührt 3 Stunden bei 0°C, anschließend 6 Stunden bei Raumtemperatur. Man gibt 300 ml 5 %ige aqu. Salzsäure zu und rührt 15 Minuten gut durch. Die organische Phase wird abgetrennt, über Magnesiumsulfat getrocknet und im Vakuum zur Trockne eingedampft. Der Rückstand wird an Kieselgel (Laufmittel: Dichlormethan/Aceton= 15:1) chromatographiert.
Ausbeute: 31,61 g (90 % d. Th.) eines farblosen Feststoffes

| Elementaranalyse: | | | | |
|---|---|---|---|---|
| ber. | C 37,33 | H 4,29 | N 2,29 | F 40,40 |
| gef. | C 37,15 | H 4,41 | N 2,12 | F 40,18 |

### b) N-(3,6,9,12-Tetraoxatridecyl)-N-(1H, 1H, 2H, 2H, 4H, 4H, 5H, 5H-3-oxa-perfluorundecyl)-amin

31 g (50,7 mmol) der Titelverbindung aus Beispiel 13a werden in 300 ml Tetrahydrofuran gelöst und 32 ml 10 M Borandimethylsulfid (in Tetrahydrofuran) zugegeben. Man kocht 16 Stunden unter Rückfluß. Es wird auf 0°C abgekühlt und 200 ml Methanol zugetropft, anschließend wird im Vakuum zur Trockne eingedampft. Der Rückstand wird in einer Mischung aus 300 ml Ethanot/50 ml 10 %iger aqu. Salzsäure aufgenommen und 8 Stunden bei 40°C gerührt. Man dampft im Vakuum zur Trockne ein, nimmt den Rückstand in 300 ml 5 %iger aqu. Natronlauge auf und extrahiert 3 mal mit je 300 ml Dichlormethan. Die organischen Phasen werden über Magnesiumsulfat getrocknet, im Vakuum zur Trockne eingedampft und der Rückstand an Kieselgel chromatographiert (Laufmittel: Dichlormethan/2-Propanol= 20:1).
Ausbeute: 28.17 g (93 % d. Th.) eines farblosen Feststoffes

| Elementaranalyse (berechnet auf wasserfreie Substanz): | | | | |
|---|---|---|---|---|
| ber. | C 38.20 | H 4,72 | N 2,34 | F 41,34 |
| gef. | C 38,05 | H 4,83 | N 2,40 | F 41,50 |

### c) 1,4,7-Tris-(carboxylatomethyl)-10-{(3-aza-4-oxo-hexan-5-yl)-säure-N-[(3,6,9,12-tetraoxa)-tridecyl-N-(1H, 1H, 2H, 2H, 4H, 4H, 5H, 5H-3oxa)-perfluorundecyl]-amid}-1,4,7,10-tetraazacyclododecan- Gadoliniumkomplex

10 g (15.88 mmol) vom Gadoliniumkomplex der 10-[1-(carboxymethylcarboamoyl)-ethyl]-1,4,7,10-tetraazacyclododecan-1,4,7-triessigsäure und 1,35 g (31,76 mmol) Lithiumchlorid werden bei 60 °C in 100 ml Dimethylsulfoxid gelöst. Man kühlt auf 15 °C ab und gibt 9,49 g (15,88 mmol) der Titelverbindung aus Beispiel 13b zu. Man rührt 10 Minuten und gibt dann 7,42 g (30 mmol) 2-Ethoxy-1-ethoxycarbonyl-1,2-dihydrochinolin zu. Es wird 12 Stunden bei Raumtemperatur gerührt. Man gießt die Lösung in eine Mischung aus 200 ml Aceton /1300 ml Diethylether und rührt 2 Stunden bei Raumtemperatur. Man filtriert den ausgefallenen Niederschlag ab. löst ihn in einer Mischung aus wenig Ethanol/Wasser und chromatographiert an Kieselgel RP-18 (Laufmittel: Gradient aus Tetrahydrofuran/Acetonitril/Wasser).
Ausbeute: 16,13 g (84 % d. Th.)

| Elementaranalyse: | | | | | |
|---|---|---|---|---|---|
| ber. | C 37,75 | H 4,67 | N 6,95 | F 20,43 | Gd 13,01 |
| gef. | C 37,91 | H 4,81 | N 6,83 | F 20,60 | Gd 13,15 |

### Beispiel 14

### a) 2-N-(1H, 1H, 2H, 2H, 4H, 4H, 5H, 5H-3-oxa-perfluortridecyl)-amino-essigsäure-t.butylester

Zu 32,0 g (58,65 mmol) der Titelverbindung aus Beispiel 5b und 24,89 g (180 mmol) Kaliumcarbonat in 300 ml Acetonitril tropft man bei 50 °C 6,523 g (40 mmol) Bromessigsäure-t.butylester zu und rührt 3 Stunden bei dieser Temperatur. Man gibt 300 ml Dichlormethan zu, filtriert von den ausgefallenen Salzen ab und dampft das Filtrat im Vakuum zur Trockne ein. Der Rückstand wird an Kieselgel chromatographiert (Laufmittel: Dichlormethan/2-Propanol= 20:1).
Ausbeute: 28,11 g (57 % d. Th.) eines farblosen Feststoffes

| Elementaranalyse: | | | | |
|---|---|---|---|---|
| ber. | C 34,80 | H 3,24 | N 2,25 | F 51,98 |
| gef. | C 34,98 | H 3,31 | N 2,20 | F 52,16 |

### b) 1,4,7-Tris-(carboxylatomethyl)-10-{(3-aza-4-oxo-hexan-5-yl)-säure-N-[(t.butyloxycarbonylmethyl)-N-(1H, 1H, 2H, 2H, 4H, 4H, 5H, 5H-3oxa)-per fluortridecyl]-amid}-1,4,7,10-tetraazacyciododecan- Gadoliniumkomplex

10 g (15.88 mmol) vom Gadoliniumkomplex der 10-[1-(carboxymethylcarboamoyl)-ethyl]-1,4,7,10-tetraazacyclododecan-1,4,7-triessigsäure und 1,35 g (31,76 mmol) Lithiumchlorid werden bei 60 °C in 100 ml Dimethylsulfoxid gelöst. Man kühlt auf 15 °C ab und gibt 9.87 g (15,88 mmol) der Titelverbindung aus Beispiel 14a zu. Man rührt 10 Minuten und gibt dann 7,42 g (30 mmol) 2-Ethoxy-1-ethoxycarbonyl-1,2-dihydrochinolin zu. Es wird 12 Stunden bei Raumtemperatur gerührt. Man gießt die Lösung in eine Mischung aus 200 ml Aceton /1300 ml Diethylether und rührt 2 Stunden bei Raumtemperatur. Man filtriert den ausgefallenen Niederschlag ab, löst ihn in einer Mischung aus wenig Ethanol/Wasser und chromatographiert an Kieselgel RP-18 (Laufmittel: Gradient aus Tetrahydrafuran/Acetonitril/Wasser). Ausbeute: 16,64 g (85 % d. Th.)

| Elementaranalyse: | | | | | |
|---|---|---|---|---|---|
| ber. | C 36,04 | H 3,92 | N 6,82 | F 26,19 | Gd 12.72 |
| gef. | C 35,92 | H 3,83 | N 6,91 | F 26.29 | Gd 12,84 |

### c) 1,4,7-Tris-(carboxylatomethyl)-10-{(3-aza-4-oxo-hexan-5-yl)-säure-N-[(carboxymethyl)-N-(1H, 1H, 2H, 2H, 4H, 4H, 5H, 5H-3oxa)-perfluortridecyl]-amid}-1,4,7,10-tetraazacyclododecan- Gadoliniumkomplex

10 g (8,11 mmol) der Titelverbindung aus Beispiel 14b werden in 50 ml Trifluoressigsäure gelöst und 5 Stunden bei Raumtemperatur gerührt. Man dampft im Vakuum zur Trockne ein und chromatographiert den Rückstand an Kieselgel RP-18 (Laufmittel: Gradient aus Tetrahydrofuran/Acetonitril/Wasser). Nach dem Eindampfen der produkthaltigen Fraktionen wird der Rückstand in Wasser gelöst und mit 5 %iger aqu. Natronlauge auf pH 7,2 gestellt. Die Lösung wird filtriert und das Filtrat gefriergetrocknet.
Ausbeute: 10,48 g (91 % d. Th.)

| Elementaranalyse (berechnet auf wasserfreie Substanz): | | | | | | |
|---|---|---|---|---|---|---|
| ber. | C 33,06 | H 3,28 | N 7,01 | F 26,94 | Gd 13,12 | Na 1,92 |
| gef. | C 33,19 | H 3,40 | N 7,20 | F 27,14 | Gd 13,25 | Na 2,00 |

### Beispiel 15

### a) 2H, 2H, 4H, 4H, 5H, 5H-3-oxa-perfluortridecansäure-N-(2-hydroxyethyl)-amid

Zu 32 g (56,61 mmol) der Titelverbindung aus Beispiel 4a gibt man 2,96 g (74 mmol) Natriumhydrid (aus 60 % Natriumhydrid in Parafinöl) in 300 ml Tetrahydrofuran und rührt 3 Stunden bei Raumtemperatur unter Stickstoff. Man tropft 7,67 g (74 mmol) Bromessigsäure-t.butylester, gelöst in 20 ml Tetrahydrofuran, zu und rührt 5 Stunden bei 50 °C. Man gibt 50 ml Methanol zu und dampft im Vakuum zur Trockne ein. Der Rückstand wird an Kieselgel chromatographiert (Laufmittel: Dichlormethan(/2-Propanol= 20:1).
Ausbeute: 23.46 g (61 % d. Th.)

| Elementaranalyse: | | | | |
|---|---|---|---|---|
| ber. | C 35,36 | H 3,26 | N 2,06 | F 47,54 |
| gef. | C 35.52 | H 3,40 | N 2.17 | F 47,40 |

### b) N-(1H, 1H, 2H, 2H, 4H, 4H, 5H, 5H-3-oxa-perfluartridecyl)-N-[4-t.butyloxycarbonyl-3-oxa)-butyl]-amin

35,0 g (51,52 mmol) der Titelverbindung aus Beispiel 15a werden in 300 ml Tetrahydrofuran gelöst und 31 ml 10 M Borandimethylsulfid (in Tetrahydrofuran) zugegeben. Man kocht 16 Stunden unter Rückfluß. Es wird auf 0°C abgekühlt und 200 ml Methanol zugetropft, anschließend wird im Vakuum zur Trockne eingedampft. Der Rückstand wird in einer Mischung aus 300 ml Ethanol/50 ml 10 %iger aqu. Salzsäure aufgenommen und 8 Stunden bei 40°C gerührt. Man dampft im Vakuum zur Trockne ein, nimmt den Rückstand in 300 ml 5 %iger aqu. Natronlauge auf und extrahiert 3 mal mit je 300 ml Dichlormethan. Die organischen Phasen werden über Magnesiumsulfat getrocknet, im Vakuum zur Trockne eingedampft und der Rückstand an Kieselgel chromatographiert (Laufmittel: Dichlormethan/2-Propanol= 20:1).
Ausbeute: 31.88 g (93 % d. Th.)

| Elementaranalyse: | | | | |
|---|---|---|---|---|
| ber. | C 36,10 | H 3,64 | N 2,11 | F 48,54 |
| gef. | C 35,90 | H 3,75 | N 2,20 | F 48,71 |

### c) 1,4,7-Tris-(carboxylatomethyl)-10-{(3-aza-4-oxo-hexan-5-yl)-säure-N-[(4-t.butyloxycarbonyl-3-oxa)-butyl)-N-(1H, 1H, 2H, 2H, 4H, 4H, 5H, 5H-3oxa)-perfluortridecyl]-amid}-1,4,7,10-tetraazacyclododecan. Gadoliniumkomplex

10 g (15,88 mmol) vom Gadoliniumkomplex der 10-[1-(carboxymethylcarboamoyl)-ethyl]-1,4,7,10-tetraazacyclododecan-1,4,7-triessigsäure und 1,35 g (31,76 mmol) Lithiumchlorid werden bei 60 °C in 100 ml Dimethylsulfoxid gelöst. Man kühlt auf 15 °C ab und gibt 10,57 g (15.88 mmol) der Titelverbindung aus Beispiel 15b zu. Man rührt 10 Minuten und gibt dann 7,42 g (30 mmol) 2-Ethoxy-1-ethoxycarbonyl-1,2-dihydrochinolin zu. Es wird 12 Stunden bei Raumtemperatur gerührt. Man gießt die Lösung in eine Mischung aus 200 ml Aceton /1300 ml Diethylether und rührt 2 Stunden bei Raumtemperatur. Man filtriert den ausgefallenen Niederschlag ab, löst ihn in einer Mischung aus wenig Ethanol/Wasser und chromatographiert an Kieselgel RP-18 (Laufmittel: Gradient aus Tetrahydrofuran/Acetonitril/Wasser).
Ausbeute: 16,63 g (82 % d. Th.)

| Elementaranalyse: | | | | | |
|---|---|---|---|---|---|
| ber. | C 36,68 | H 4,10 | N 6,58 | F 25,29 | Gd 12.31 |
| gef. | C 36,81 | H 4,20 | N 6,41 | F 25,40 | Gd 12,19 |

### d) 1,4,7-Tris-(carboxylatomethyl)-10-{(3-aza-4-oxo-hexan-5-yl)-säure-[N-(4-carboxy-3-oxa)-butyl)-N-(1H, 1H, 2H, 2H, 4H, 4H, 5H, 5H-3oxa)-peffluortridecyl]-amid}-1,4,7,10-tetraazacyclododecan, Gadoliniumkomplex

12 g (9,40 mmol) der Titelverbindung aus Beispiel 15c werden in 50 ml Trifluoressigsäure gelöst und 5 Stunden bei Raumtemperatur gerührt. Man dampft im Vakuum zur Trockne ein und chromatographiert den Rückstand an Kieselgel RP-18 (Laufmittel: Gradient aus Tetrahydrofuran/Acetonitril/Wasser). Nach dem Eindampfen der produkthaltigen Fraktionen wird der Rückstand in Wasser gelöst und mit 5 %iger aqu. Natronlauge auf pH 7,2 gestellt. Die Lösung wird filtriert und das Filtrat gefriergetrocknet.
Ausbeute: 11,41 g (92 % d. Th.)
Wassergehalt: 5,8 %

| Elementaranalyse (berechnet auf wasserfreie Substanz): | | | | | | |
|---|---|---|---|---|---|---|
| ber. | C 33,82 | H 3,49 | N 6,76 | F 25,98 | Gd 12,65 | Na 1,85 |
| gef. | C 33,95 | H 3,60 | N 6,88 | F 26,15 | Gd 12,49 | Na 1,93 |

### Beispiel 16

### a) 2H, 2H, 4H, 4H. 5H, 5H-3-oxa-perfluortridecansäure-N-(2H, 2H, 4H, 4H, 5H, 5H-3-oxa-perfluortridecyl)-amid

Zu 30 g (57,45 mmol) 2H, 2H, 4H, 4H, 5H, 5H-3-oxa-perfluortridecansäure in 300 ml Dichlormethan gibt man 8,90 g (70 mmol) Oxalylchlorid und rührt 12 Stunden bei Raumtemperatur. Es wird im Vakuum zur Trockne eingedampft. Der Rückstand wird in 100 ml Dichlormethan gelöst und bei 0°C zu einer Lösung aus 32,62 g (60 mmol) der Titelverbindung aus Beispiel 5b und 6,07 g (60 mmol) Triethylamin, gelöst in 200 ml Dichlormethan getropft. Man rührt 3 Stunden bei 0°C, anschließend 6 Stunden bei Raumtemperatur. Man gibt 300 ml 5 %ige aqu. Salzsäure zu und rührt 15 Minuten gut durch. Die organische Phase wird abgetrennt, über Magnesiumsulfat getrocknet und im Vakuum zur Trockne eingedampft- Der Rückstand wird an Kieselgel (Laufmittel: Dichlormethan/Aceton= 15:1) chromatographiert.
Ausbeute: 52,87 g (91 % d. Th.)

| Elementaranalyse: | | | | |
|---|---|---|---|---|
| ber. | C 28,50 | H 1,49 | N 1,38 | F 63,87 |
| gef. | C 28,65 | H 1,61 | N 1,50 | F 64,01 |

### b) N-Bis-(1H, 1H, 2H, 2H, 4H, 4H, 5H, 5H-3-oxa)-perfluortridecyl)-amin

52 g (51,42 mmol) der Titelverbindung aus Beispiel 16a werden in 500 ml Tetrahydrofuran gelöst und 31 ml 10 M Borandimethylsulfid (in Tetrahydrofuran)-zugegeben. Man kocht 16 Stunden unter Rückfluß. Es wird auf 0°C abgekühlt und 200 ml Methanol zugetropft, anschließend wird im Vakuum zur Trockne eingedampft. Der Rückstand wird in einer Mischung aus 400 ml Ethanol/70 ml 10 %iger aqu. Salzsäure aufgenommen und 8 Stunden bei 40°C gerührt. Man dampft im Vakuum zur Trockne ein, nimmt den Rückstand in 400 ml 5 %iger aqu. Natronlauge auf und extrahiert 3 mal mit je 400 ml Dichlormethan. Die organischen Phasen werden über Magnesiumsulfat getrocknet, im Vakuum zur Trockne eingedampft und der Rückstand an Kieselgel chromatographiert (Laufmittel: Didhiormethan/2-Propanol= 20:1).
Ausbeute: 47,18 g (92 % d. Th.) eines farblosen Feststoffes

| Elementaranalyse: | | | | |
|---|---|---|---|---|
| ber. | C 28,90 | H 1,72 | N 1,40 | F 64.77 |
| gef. | C 30.03 | H 1,81 | N 1,55 | F 65.00 |

### c) 1,4,7-Tris(carboxylatomethyl)-10-[(3-aza-4-oxo-hexan-5-yl)-säure-N-bis-(1H, 1H, 2H, 2H, 4H, 4H, 5H, 5H-3-oxa-perfiuortridecyl)-amid]-1,4,7,10-tetraazacyclododecan. Gadoliniumkomplex

10 g (15.88 mmol) vom Gadoliniumkomplex der 10-[1-(carboxymethylcarboamoyl)-ethyl]-1,4,7,10-tetraazacyclododecan-1,4,7-triessigsäure und 1,35 g (31,76 mmol) Lithiumchlorid werden bei 60 °C in 100 ml Dimethylsulfoxid gelöst. Man kühlt auf 15 °C ab und gibt 15,84 g (15,88 mmol) der Titelverbindung aus Beispiel 16b zu. Man rührt 10 Minuten und gibt dann 7,42 g (30 mmol) 2-Ethoxy-1-ethoxycarbonyl-1,2-dihydrochinolin zu. Es wird 12 Stunden bei Raumtemperatur gerührt. Man gießt die Lösung in eine Mischung aus 200 ml Aceton /1300 ml Diethylether und rührt 2 Stunden bei Raumtemperatur. Man filtriert den ausgefallenen Niederschlag ab, löst ihn in einer Mischung aus wenig Ethanol/Wasser und chromatographiert an Kieselgel RP-18 (Laufmittel: Gradient aus Tetrahydrofuran/Acetonitril/Wasser).
Ausbeute: 20,95 g (82 % d. Th.)

| Elementaranalyse: | | | | | |
|---|---|---|---|---|---|
| ber. | C 32,10 | H 2,82 | N 5,22 | F 40,14 | Gd 9,77 |
| gef. | C 29,87 | H 2,91 | N 5,09 | F 40,28 | Gd 9,98 |

### Beispiel 17

### a) 2H, 2H, 4H. 4H. 5H. 5H-3-oxa-perfluortridecansäure-N-(5-hydroxy-3-oxapentyl)-amid

Zu 32 g (52,52 mmol) der Titelverbindung aus Beispiel 3a gibt man 2,80 g (70 mmol) Natriumhydrid (aus 60 % Natriumhydrid in Parafinöl) in 300 ml Tetrahydrofuran und rührt 3 Stunden bei Raumtemperatur unter Stickstoff. Man tropft 9,68 g (70 mmol) Bromessigsäure-t.butylester gelöst in 20 ml Tetrahydrofuran zu und rührt 5 Stunden bei 50 °C. Man gibt 50 ml Methanol zu und dampft im Vakuum zur Trockne ein. Der Rückstand wird an Kieselgel chromatographiert (Laufmittel: Dichlorrnethan(/2-Propanol= 20:1).
Ausbeute: 19.31 g (59 % d. Th.)

| Elementaranalyse: | | | | |
|---|---|---|---|---|
| ber. | C 32,76 | H 2,91 | N 2,25 | F 51,82 |
| gef. | C 32,98 | H 2,99 | N 2,36 | F 51,98 |

### b) N-(3,6-Dioxa-heptyl)-N-(1H, 1H, 2H, 2H, 4H, 4H, 5H, 5H-3-oxa-perfluortridecyl)-amin

32 g (51,34 mmol) der Titelverbindung aus Beispiel 17a werden in 300 ml Tetrahydrofuran gelöst und 31 ml 10 M Borandimethylsulfid (in Tetrahydrofuran) zugegeben. Man kocht 16 Stunden unter Rückfluß. Es wird auf 0°C abgekühlt und 200 ml Methanol zugetropft, anschließend wird im Vakuum zur Trockne eingedampft. Der Rückstand wird in einer Mischung aus 300 ml Ethanol/50 ml 10 %iger aqu. Salzsäure aufgenommen und 8 Stunden bei 40°C gerührt. Man dampft im Vakuum zur Trockne ein, nimmt den Rückstand in 300 ml 5 %iger aqu. Natronlauge auf und extrahiert 3 mal mit je 300 ml Dichlormethan. Die organischen Phasen werden über Magnesiumsulfat getrocknet, im Vakuum zur Trockne eingedampft und der Rückstand an Kieselgel chromatographiert (Laufmittel: Dichlormethan/2-Propanol= 20:1).
Ausbeute: 28,47 g (91 % d. Th.)

| Elementaranalyse: | | | | |
|---|---|---|---|---|
| ber. | C 33.51 | H 3,31 | N 2.30 | F 53.01 |
| gef. | C 33.63 | H 3,41 | N 2,21 | F 52,87 |

### c) 1,4,7-Tris(carboxylatomethyl)-10-[(3-aza-4-oxo-hexan-5-yl)-säure-N-(3,6-dioxa)-heptyl-N-(1H, 1H, 2H, 2H, 4H, 4H, 5H, 5H-3-oxa-pertluortridecyl)-amid]-1,4,7,10-tetraazacyclododecan, Gadoliniumkomplex

10 g (15,88 mmol) vom Gadoliniumkomplex der 10-[1-(carboxymethylcarboamoyl)-ethyl]-1,4,7,10-tetraazacyclododecan-1,4,7-triessigsäure und 1,35 g (31,76 mmol) Lithiumchlorid werden bei 60 °C in 100 ml Dimethylsulfoxid gelöst. Man kühlt auf 15 °C ab und gibt 9,68 g (15,88 mmol) der Titelverbindung aus Beispiel 17b zu. Man rührt 10 Minuten und gibt dann 7,42 g (30 mmol) 2-Ethoxy-1-ethoxycarbonyl-1,2-dihydrochinolin zu. Es wird 12 Stunden bei Raumtemperatur gerührt. Man gießt die Lösung in eine Mischung aus 200 ml Aceton /1300 ml Diethylether und rührt 2 Stunden bei Raumtemperatur. Man filtriert den ausgefallenen Niederschlag ab, löst ihn in einer Mischung aus wenig Ethanol/Wasser und chromatographiert an Kieselgel RP-18 (Laufmittel: Gradient aus Tetrahydrofuran/Acetonitril/Wasser).
Ausbeute: 16,09 g (83 % d. Th.)

| Elementaranalyse: | | | | | |
|---|---|---|---|---|---|
| ber. | C 35,41 | H 3,96 | N 6,88 | F 26,45 | Gd 12,88 |
| gef. | C 35,57 | H 4,11 | N 6,72 | F 26,58 | Gd 12,97 |

### Beispiel 18

### a) 2H, 2H, 4H, 4H, 5H, 5H-3-oxa-perfluortridecansäure-N-(hexyl)-amid

Zu 30 g (57,45 mmol) 2H, 2H, 4H, 4H, 5H, 5H-3-oxa-perfluortridecansäure in 300 ml Dichlormethan gibt man 8,90 g (70 mmol) Oxalylchlorid und rührt 12 Stunden bei Raumtemperatur. Es wird im Vakuum zur Trockne eingedampft. Der Rückstand wird in 100 ml Dichlormethan gelöst und bei 0°C zu einer Lösung aus 6,07 g (60 mmol) nHexylamin und 6,07 g (60 mmol) Triethylamin, gelöst in 200 ml Dichlormethan getropft. Man rührt 3 Stunden bei 0°C, anschließend 6 Stunden bei Raumtemperatur. Man gibt 300 ml 5 %ige aqu. Salzsäure zu und rührt 15 Minuten gut durch. Die organische Phase wird abgetrennt. über Magnesiumsulfat getrocknet und im Vakuum zur Trockne eingedampft. Der Rückstand wird an Kieselgel (Laufmittel: Dichlormethan/Aceton= 20:1) chromatographiert.
Ausbeute: 30,95 g (89 % d. Th.)

| Elementaranalyse: | | | | |
|---|---|---|---|---|
| ber. | C 35,72 | H 3,33 | N 2,31 | F 53,35 |
| gef. | C 35,60 | H 3,45 | N 2,43 | F 53,63 |

### b) N-(Hexyl)-N-(1H, 1H, 2H, 2H, 4H, 4H, 5H, 5H-3-oxa-perfluortridecyl)-amin

31 g (51,21 mmol) der Titelverbindung aus Beispiel 18a werden in 300 ml Tetrahydrofuran gelöst und 31 ml 10 M Borandimethylsulfid (in Tetrahydrofuran) zugegeben. Man kocht 16 Stunden unter Rückfluß. Es wird auf 0°C abgekühlt und 200 ml Methanol zugetropft, anschließend wird im Vakuum zur Trockne eingedampft. Der Rückstand wird in einer Mischung aus 300 ml Ethanol/50 ml 10 %iger aqu. Salzsäure aufgenommen und 8 Stunden bei 40°C gerührt. Man dampft im Vakuum zur Trockne ein, nimmt den Rückstand in 300 ml 5 %iger aqu. Natronlauge auf und extrahiert 3 mal mit je 300 ml Dichlormethan. Die organischen Phasen werden über Magnesiumsulfat getrocknet, im Vakuum zur Trockne eingedampft und der Rückstand an Kieselgel chromatographiert (Laufmittel: Dichlormethan/2-Propanol= 20:1).
Ausbeute: 28,16 g (93 % d. Th.)

| Elementaranalyse: | | | | |
|---|---|---|---|---|
| ber. | C 36,56 | H 3,75 | N 2,37 | F 54,62 |
| gef. | C 36,40 | H 3,82 | N 2,27 | F 54,81 |

### c) 1,4,7-Tris(carboxylatomethyl)-10-{(3-aza-4-oxo-hexan-5-yl)-säure-[N-(hexyl)-N-(1H, 1H, 2H, 2H, 4H, 4H, 5H, 5H-3-oxa-perfluortridecyl]-amid}-1,4,7,10-tetraazacyclododecan, Gadoliniumkomplex

10 g (15.88 mmol) vom Gadoliniumkomplex der 10-[1-(carboxymethylcarboamoyl)-ethyl]-1,4,7,10-tetraazacyclododecan-1,4,7-triessigsäure und 1,35 g (31,76 mmol) Lithiumchlorid werden bei 60 °C in 100 ml Dimethylsulfoxid gelöst. Man kühlt auf 15 °C ab und gibt 10,98 g (15,88 mmol) der Titelverbindung aus Beispiel 18b zu. Man rührt 10 Minuten und gibt dann 7,42 g (30 mmol) 2-Ethoxy-1-ethoxycarbonyl-1,2-dihydrochinoün zu. Es wird 12 Stunden bei Raumtemperatur gerührt. Man gießt die Lösung in eine Mischung aus 200 ml Aceton /1300 ml Diethylether und rührt 2 Stunden bei Raumtemperatur. Man filtriert den ausgefallenen Niederschlag ab, löst ihn in einer Mischung aus wenig Ethanol/Wasser und chromatographiert an Kieselgel RP-18 (Laufmittel: Gradient aus Tetrahydrofuran/Acetonitril/Wasser).
Ausbeute: 16.29 g (84 % d. Th.)

| Elementaranalyse: | | | | | |
|---|---|---|---|---|---|
| ber. | C 36.94 | H 4,19 | N 6,99 | F 26,85 | Gd 13,07 |
| gef. | C 37,18 | H 4,31 | N 7,18 | F 26,67 | Gd 13,19 |

### Beispiel 19

### a) 2H, 2H, 4H, 4H, 5H, 5H-3-oxa-perfluortridecansäure-N-[(10-t.butyloxycarbonyl)-decyl]-amid

Zu 30 g (57,45 mmol) 2H, 2H, 4H, 4H, 5H, 5H-3-oxa-perfluortridecansäure in 300 ml Dichlormethan gibt man 8,90 g (70 mmol) Oxalylchlorid und rührt 12 Stunden bei Raumtemperatur. Es wird im Vakuum zur Trockne eingedampft. Der Rückstand wird in 100 ml Dichlormethan gelöst und bei 0°C zu einer Lösung aus 15,45 g (60 mmol) 11-Amino-undecansäure-t.butylester und 6,07 g (60 mmol) Triethylamin, gelöst in 200 ml Dichlormethan, getropft. Man rührt 3 Stunden bei 0°C, anschließend 6 Stunden bei Raumtemperatur. Man gibt 300 ml 5 %ige aqu. Salzsäure zu und rührt 15 Minuten gut durch. Die organische Phase wird abgetrennt, über Magnesiumsulfat getrocknet und im Vakuum zur Trockne eingedampft. Der Rückstand wird an Kieselgel (Laufmittel: Dichlormethan/Aceton= 20:1) chromatographiert.
Ausbeute: 42,04 g (92 % d. Th.)

| Elementaranalyse: | | | | |
|---|---|---|---|---|
| ber. | C 42,58 | H 4,76 | N 1,84 | F 42,41 |
| gef. | C 42,74 | H 4,90 | N 1,73 | F 42,61 |

### b) N-(10-t.Butyloxycarbonyl-decyl)-N-(1H, 1H, 2H, 2H, 4H, 4H, 5H, 5H-3-oxa-perfluortridecyl)-amin

39 g (51,21 mmol) der Titelverbindung aus Beispiel 19a werden in 300 ml Tetrahydrofuran gelöst und 31 ml 10 M Borandimethylsulfid (in Tetrahydrofuran) zugegeben. Man kocht 16 Stunden unter Rückfluß. Es wird auf 0°C abgekühlt und 200 ml Methanol zugetropft, anschließend wird im Vakuum zur Trockne eingedampft. Der Rückstand wird in einer Mischung aus 400 ml Ethanol/70 ml 10 %iger aqu. Salzsäure aufgenommen und 8 Stunden bei 40°C gerührt. Man dampft im Vakuum zur Trockne ein, nimmt den Rückstand in 350 ml 5 %iger aqu. Natronlauge auf und extrahiert 3 mal mit je 400 ml Dichlormethan. Die organischen Phasen werden über Magnesiumsulfat getrocknet, im Vakuum zur Trockne eingedampft und der Rückstand an Kieselgel chromatographiert (Laufmittel: Dichlormethan/2-Propanol= 20:1).
Ausbeute: 34,84 g (91 % d. Th.)

| Elementaranalyse: | | | | |
|---|---|---|---|---|
| ber. | C 43,38 | H 5,12 | N 1,87 | F 43,20 |
| gef. | C 43,22 | H 5,23 | N 1,96 | F 43,33 |

### c) 1,4,7-Tris(carboxylatomethyl)-10-{(3-aza-4-oxo-hexan-5-yl)-säure-[N-(10-t.butyloxycarbonyl)-decyl-N-(1H, 1H, 2H, 2H, 4H, 4H, 5H, 5H-3-oxa-perfluortridecyl]-amid}-1,4,7,10-tetraazacyclododecan, Gadoliniumkomplex

10 g (15.88 mmol) vom Gadoliniumkomplex der 10-[1-(carboxymethylcarboamoyl)-ethyl]-1,4,7,10-tetraazacyclododecan-1,4,7-triessigsäure und 1,35 g (31,76 mmol) Lithiumchlorid werden bei 60 °C in 100 ml Dimethylsulfoxid gelöst. Man kühlt auf 15 °C ab und gibt 11,87 g (15,88 mmol) der Titelverbindung aus Beispiel 19b zu. Man rührt 10 Minuten und gibt dann 7,42 g (30 mmol) 2-Ethoxy-1-ethoxycarbonyl-1,2-dihydrochinolin zu. Es wird 12 Stunden bei Raumtemperatur gerührt. Man gießt die Lösung in eine Mischung aus 200 ml Aceton /1300 ml Diethylether und rührt 2 Stunden bei Raumtemperatur. Man filtriert den ausgefallenen Niederschlag ab, löst ihn in einer Mischung aus wenig Ethanol/Wasser und chromatographiert an Kieselgel RP-18 (Laufmittel: Gradient aus Tetrahydrofuran/Acetonitril/Wasser).
Ausbeute: 17,92 g (83 % d. Th.)

| Elementaranalyse: | | | | | |
|---|---|---|---|---|---|
| ber. | C 40,65 | H 4,89 | N 6,18 | F 23,76 | Gd 11,57 |
| gef. | C 40,81 | H 4,99 | N 6,32 | F 23,94 | Gd 11,73 |

### d) 1,4,7-Tris-(carboxylatomethyl)-10-{(3-aza-4-oxo-hexan-5-yl)-säure-[N-(10-carboxy)-decyl-N-(1H, 1H, 2H, 2H, 4H, 4H, 5H, 5H-3oxa)-perfluortridecyl]-amid}-1,4,7,10-tetraazacyclododecan, Gadoliniumkomplex, Natriumsalz

12 g (8,83 mmol) der Titelverbindung aus Beispiel 19c werden in 50 ml Trifluoressigsäure gelöst und 5 Stunden bei Raumtemperatur gerührt. Man dampft im Vakuum zur Trockne ein und chromatographiert den Rückstand an Kieselgel RP-18 (Laufmittel: Gradient aus Tetrahydrofuran/Acetonitril/Wasser). Nach dem Eindampfen der produkthaltigen Fraktionen wird der Rückstand in Wasser gelöst und mit 5 %iger aqu. Natronlauge auf pH 7,2 gestellt. Die Lösung wird filtriert und das Filtrat gefriergetrocknet.
Ausbeute: 12,48 g (92 % d. Th.)
Wassergehalt: 6,2 %

| Elementaranalyse (berechnet auf wasserfreie Substanz): | | | | | | |
|---|---|---|---|---|---|---|
| ber. | C 38,07 | H 4,34 | N 6,34 | F 24,37 | Gd 11,87 | Na 1,73 |
| gef. | C 37,89 | H 4,44 | N 6,22 | F 24,51 | Gd 12,01 | Na 1,80 |

### Beispiel 20

### a) 15-Benzyl-3,6,9,12,15-Pentaoxa-hexadecylsäure-N-(1H, 1H, 2H, 2H, 4H, 4H, 5H, 5H-3oxa)-perfluortridecyl)-amid

Zu 19,67 g (57,45 mmol) 15-Benzyl-3,6,9,12,15-Pentaoxahexadecylsäure in 250 ml Dichlormethan gibt man 8,90 g (70 mmol) Oxalylchlorid und rührt 12 Stunden bei Raumtemperatur. Es wird im Vakuum zur Trockne eingedampft. Der Rückstand wird in 100 ml Dichlormethan gelöst und bei 0°C zu einer Lösung aus 32,62 g (60 mmol) 1H, 1H, 2H, 2H, 4H, 4H, 5H, 5H-3-oxa-perfluor-tridecylamin, Hydrochlorid und 6,07 g (60 mmol) Triethylamin, gelöst in 200 ml Dichlormethan getropft. Man rührt 3 Stunden bei 0°C, anschließend 6 Stunden bei Raumtemperatur. Man gibt 300 ml 5 %ige aqu. Salzsäure zu und rührt 15 Minuten gut durch. Die organische Phase wird abgetrennt, über Magnesiumsulfat getrocknet und im Vakuum zur Trockne eingedampft. Der Rückstand wird an Kieselgel (Laufmittel: Dichlormethan/Aceton= 20:1) chromatographiert.
Ausbeute: 44,91 g (94 % d. Th.) eines farblosen Feststoffes

| Elementaranalyse: | | | | |
|---|---|---|---|---|
| ber. | C 41,89 | H 4,12 | N 1,68 | F 38,84 |
| gef. | C 42,02 | H 4,25 | N 1,83 | F 39,07 |

### b) N-15-Benzyl-3,6,9,12,15-pentaoxa-hexadecyl)-N-(1H, 1H, 2H, 2H, 4H, 4H, 5H, 5H-3oxa)-perfluortridecyl)-amin

43 g (51,72 mmol) der Titelverbindung aus Beispiel 20a werden in 400 ml Tetrahydrofuran gelöst und 31 ml 10 M Borandimethylsulfid (in Tetrahydrofuran) zugegeben. Man kocht 16 Stunden unter Rückfluß. Es wird auf 0°C abgekühlt und 200 ml Methanol zugetropft, anschließend wird im Vakuum zur Trockne eingedampft. Der Rückstand wird in einer Mischung aus 400 ml Ethanol/50 ml 10 %iger aqu. Salzsäure aufgenommen und 8 Stunden bei 40°C gerührt. Man dampft im Vakuum zur Trockne ein, nimmt den Rückstand in 350 ml 5 %iger aqu. Natronlauge auf und extrahiert 3 mal mit je 400 ml Dichlormethan. Die organischen Phasen werden über Magnesiumsulfat getrocknet, im Vakuum zur Trockne eingedampft und der Rückstand an Kieselgel chromatographiert (Laufmittel: Dichlormethan/2-Propanol= 20:1).
Ausbeute: 39,32 g (93 % d. Th.)

| Elementaranalyse: | | | | |
|---|---|---|---|---|
| ber. | C 42,60 | H 4,12 | N 1,68 | F 38,84 |
| gef. | C 42,45 | H 4,23 | N 1,57 | F 38,99 |

### c) 1,4,7-Tris-(carboxylatomethyl)-10-{(3-aza-4-oxo-hexan-5-yl)-säure-[N-(15-benzyl-3,6,9,12,15-pentaoxa)-hexadecyl-N-(1H, 1H, 2H, 2H, 4H, 4H, 5H, 5H-3-oxa)-tridecyl]-amid}-1,4,7,10-tetraazacyclododecan, Gadoliniumkomplex

10 g (15,88 mmol) vom Gadoliniumkomplex der 10-[1-(carboxymethylcarboamoyl)-ethyl]-1,4,7,10-tetraazacyclododecan-1,4,7-triessigsäure und 1,35 g (31,76 mmol) Lithiumchlorid werden bei 60 °C in 100 ml Dimethylsulfoxid gelöst. Man kühlt auf 15 °C ab und gibt 12,98 g (15,88 mmol) der Titelverbindung aus Beispiel 20b zu. Man rührt 10 Minuten und gibt dann 7,42 g (30 mmol) 2-Ethoxy-1-ethoxycarbonyl-1,2-dihydrochinolin zu. Es wird 12 Stunden bei Raumtemperatur gerührt. Man gießt die Lösung in eine Mischung aus 200 ml Aceton /1300 ml Diethylether und rührt 2 Stunden bei Raumtemperatur. Man filtriert den ausgefallenen Niederschlag ab, löst ihn in einer Mischung aus wenig Ethanol/Wasser und chromatographiert an Kieselgel RP-18 (Laufmittel: Gradient aus Tetrahydrofuran/Acetonitril/Wasser).
Ausbeute: 18,84 g (83 % d. Th.)

| Elementaranalyse: | | | | | |
|---|---|---|---|---|---|
| ber. | C 40,34 | H 4,51 | N 5,88 | F 22,60 | Gd 11,00 |
| gef. | C 40,50 | H 4,62 | N 5,76 | F 22,73 | Gd 11,16 |

### d) 1,4,7-Tris-(carboxylatomethyl)-10-{(3-aza-4-oxo-hexan-5-yl)-säure-[N-(14-hydroxy-3,6,9,12-tetraoxa)-tetradecyl-N-(1H, 1H, 2H, 2H, 4H, 4H, 5H, 5H-3-oxa)-perfluortridecyl]-amid}-1,4,7,10-tetraazacyclododecan, Gadoliniumkomplex

12 g (8,40 mmol) der Titelverbindung aus Beispiel 20c werden in 150 ml Methanol gelöst und 1,0 g Palladiumkatalysator (10 % Pd/C) zugegeben, man hydriert über Nacht bei Raumtemperatur. Man filtriert zum Katalysator ab und dampft das Filtrat im Vakuum zur Trockne ein.
Ausbeute: 10,13 g (95 % d. Th.)

| Elementaranalyse: | | | | | |
|---|---|---|---|---|---|
| ber. | C 38,80 | H 4,61 | N 1,10 | F 25,45 | Gd 12,39 |
| gef. | C 38,87 | H 4,73 | N 1,20 | F 25,58 | Gd 12,50 |

### Beispiel 21

In vivo Vergleich der Verbindungen aus den Beispielen 4c und 5e mit Dy - DTPA

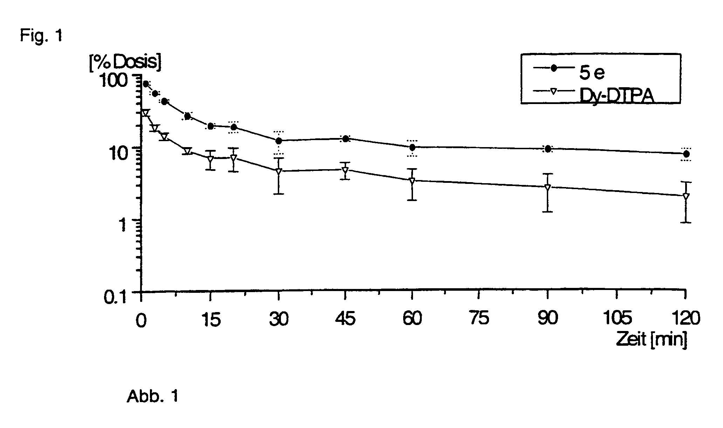

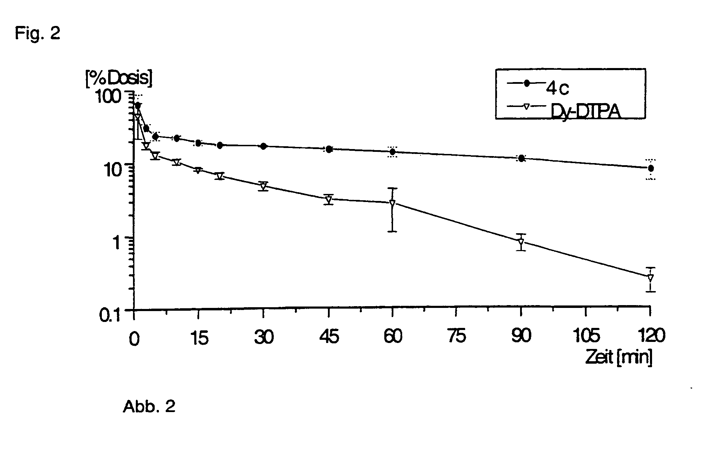

Als Versuchstiere dienen drei 350 g schwere, männliche (Schering-SPF-) Ratten. Je Tier werden 0.33-0.37 ml (jeweils 100 mmol/L) der folgenden Kontrastmittel-Lösung intravenös appliziert: Gemisch aus je 1 Teil einer perfluoralkylhaltigen Verbindung und dem Dysprosium-Komplex der (Dy-DTPA). Die applizierte Dosis beträgt jeweils 100 µmol Gd bzw. Dy/kg KG. Über einen Katheter in der Arteria carotis communis werden Blutproben zu folgenden Zeitpunkten entnommen: 1, 3, 5, 10, 15, 20, 30, 45, 60, 90, 120 min p.i.. In den gewonnenen Blutproben werden jeweils parallel die Konzentrationen an Gadolinium (Gd) und Dysprosium (Dy) mittels Atomemissionsspektrometrie (ICP-AES) gemessen. Der im Blutraum verbliebene Anteil der injizierten Verbindungen (Gd-haltige perfluoralkylhaltige Verbindung und Dy-haltige Vergleichssubstanz) kann durch die unterschiedliche Markierung im gleichen Tier verglichen werden. Aus den Blutkonzentrationen kann mittels spezieller Software (Topfit-Programm) die α- und β-Halb-wertzeit, das Verteilungsvolumen sowie die total Clearance errechnet werden. Damit liefern diese Daten Angaben über den Verbleib der Verbindungen im Intravasalraum, die Verteilungsverhältnisse im Organismus und die Eliminierung.
Ergebnisse: Zu allen Untersuchungszeitpunkten werden deutlich höhere Blutkonzentrationen der perfluoralkylhaltigen Verbindungen (Substanzen aus Beispiel 4c bzw. 5e) verglichen mit dem extrazellulären Kontrastmittel (Dy-DTPA) erhalten. Siehe dazu Figur 1 und 2:
Figur 1, Abb. 1 zeigt den Blutspiegel (in % der Dosis) von Gd (perfluoralkylhaltige Verbindung aus Beispiel 5e) und Dy (Dy-DTPA) nach intravenöser Applikation von jeweils 100 µmol/kg KG in Ratten (n=3)
In Tabelle 1 sind pharmakokinetischen Parameter (Plasma) von der Verbindung aus Beispiel 5e) und Dy-DTPA nach intravenöser Applikation von jeweils 100 µmol/kg KG in Ratten (n=3) angegeben:

**Tab. 1:**

| | | 5e | Dy-DTPA |
|---|---|---|---|
| α-t½ | min | 3.77 ± 0.75 | 2.19 ± 0.59 |
| β-t½ | min | 102.29 ± 24.48 | 62.59 ± 37.47 |
| Vd ss | Ukg | 0.16 ± 0.03 | 0.29 ± 0.09 |
| Total Clearance | ml/min*kg | 1.22 ± 0.05 | 4.24 ± 1.52 |

Figur 2, Abb. 2 zeigt den Blutspiegel (in % der Dosis) von Gd (perfluoralkylhaltige Verbindung aus Beispiel 4c) und Dy (Dy-DTPA) nach intravenöser Applikation von jeweils 100 µmol/kg KG in Ratten (n=3)

**Tab. 2**

| | | 4c | Dy-DTPA |
|---|---|---|---|
| α-^{t½} | min | 1.01 ± 0.32 | 0.89 ± 0.32 |
| β-^{t½} | min | 79.68 ± 12.26 | 21.37 ± 2.18 |
| Vd ss | L/kg | 0.12 ± 0.00 | 0.15 ± 0.03 |
| Total Clearance | ml/min*kg | 1.14 ± 0.19 | 5.77 ± 1.08 |

In Tabelle 2 sind die pharmakokinetischen Parameter (Plasma) von Verbindung 4c) und Dy-DTPA nach intravenöser Applikation von jeweils 100 µmol/kg KG in Ratten (n=3) angegeben.

Die deutlich höheren Blutkonzentrationen der perfluoralkylhaltigen Verbindungen (Substanzen aus den Beispielen 4c bzw. 5e) weisen auf ein deutlich kleineres Verteilungsvolumen verglichen zum Dy-DTPA hin (siehe auch Vd ss in Tab. 1 und 2), d.h. diese perfluoralkylhaltigen Verbindungen verteilen sich nicht wie Dy-DTPA im Intravasalraum (Gefäße) *und* im Extrazellularraum, sondern größtenteils *nur* im Intravasalraum (besonders zu frühen Zeitpunkten). Im weiteren Verlauf fällt der Blutspiegel der perfluoralkylhaltigen Verbindungen jedoch ab und die Eliminations- oder β-Halbwertzeiten sind deutlich kürzer als bei anderen blood-pool-agents. Die totale Blut-Clearance der perfluoralkylhaltigen Verbindungen ist nur geringfügig kleiner verglichen zu Dy-DTPA, was auf eine vergleichbar gute Nierenelimination schließen läßt.
Die in Beispiel 21 beschriebenen perfluoralkylhaltigen Verbindungen zeigen effiziente Elimination aus dem Blut (über die Nieren), aber ein deutlich kleineres Verteilungsvolumen als es ein extrazelluläres Kontrastmittel wie Dy - DTPA zeigt.

### Beispiel 22

### Lymphknotenanreicherung am Meerschweinchen

Verschiedene perfluoralkylhaltige Gadolinium-Komplexe wurden 30 und 90 min nach subkutaner Gabe (10 µmol Gesamtgadolinium/kg KG, Hinterpfote s.c.) an stimulierten Meerschweinchen (komplettes Freund-Adjuvant; jeweils 0,1 ml i.m. in den rechten und linken Ober- und Unterschenkel; 2 Wochen vor Gabe der Prüfsubstanzen) hinsichtlich ihrer Lymphknotenanreicherung in drei aufeinanderfolgenden Lymphknotenstationen (popliteal, inguinal, iliakal) untersucht. Hierbei wurden die nachfolgend aufgelisteten Ergebnisse (Ermittlung der Gadolinium-Konzentration mittels ICP-AES) erhalten:

| **Substanz** | **Zeitpunkt der Lymphknotenentnahme** | **Gadolinium- Gehalt in drei aufeinander-folgenden** **Lymphknotenstationen** **[µmol/l]** **[% Dosis/g Gewebe]** | | |
|---|---|---|---|---|
| **Beispiel** | | **Popliteal** | **Inguinal** | **lliakal** |
| 7 | 30 min | 452 µmol/l 13,1 % | 181 µmol/l 5,2 % | 228 µmol/l 6,6 % |
| 6b | 30 min | 442 µmol/l 12,6 % | 339 µmol/l 9,6 % | 322 µmol/l 9,1 % |
| 5e | 30 min | 581 µmol/l 16,9% | 166 µmol/l 4,8% | 111 µmol/l 3,2% |
| 3c | 90 min | 346 µmol/l 10,1 % | 184 µmol/l 5,4 % | 171 µmol/l 5,0 % |

### Beispiel 23

### Lymphknotendarstellung (MRT) nach interstitieller Gabe des Kontrastmittels

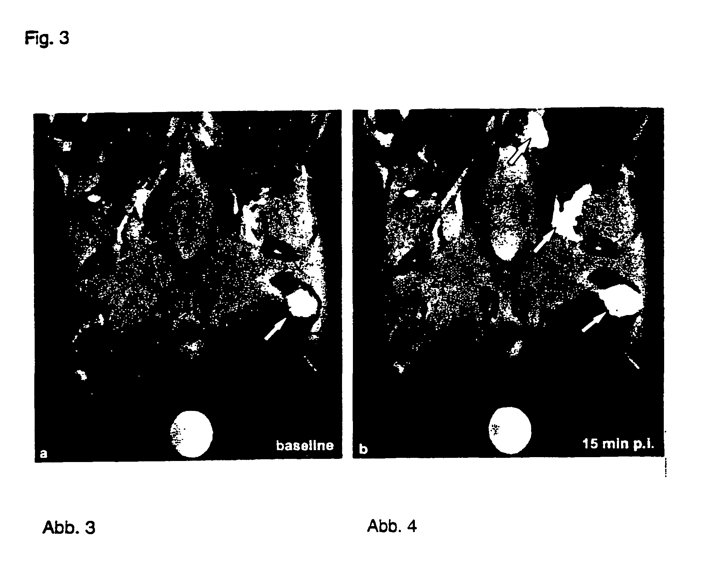

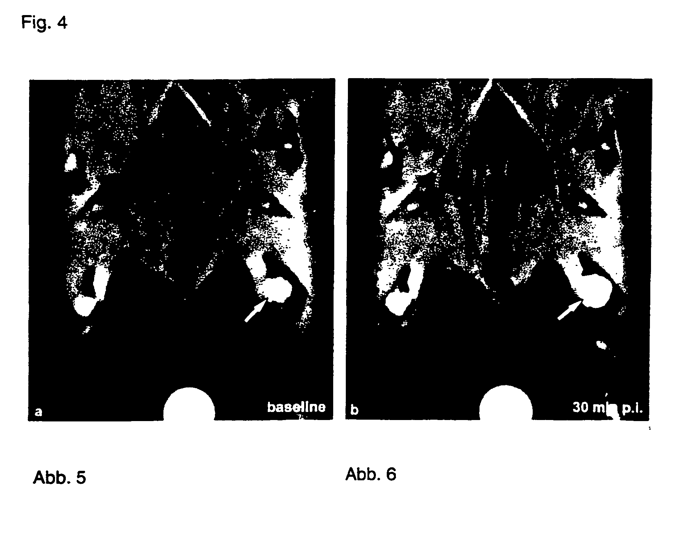

Die Figuren 3 und 4 zeigen MR-Aufnahmen von poplitealen, inguinalen und iliakalen Lymphknoten sowohl vor (Abb. 3: Präkontrast), als auch 15 bzw. 30 min nach (Abb. 4) subkutaner Applikation (Meerschweinchen, Hinterpfote, Zwischenzehenraum) von Substanz aus Beispiel 5e (Fig. 3: Abb. 3 und Abb. 4) bzw. aus Beispiel 3c (Fig. 4: Abb. 5 und Abb. 6) (jeweils 10 µmol Gd/kg KG). Die T₁-gewichteten, Gradientenecho-Aufnahmen (TR 10 ms, flash outphase, TE 5 ms, α 40°) verdeutlichen den starken Signalanstieg in den verschiedenen Lymphknoten der injizierten (Pfeil) im Vergleich zur nicht-injizierten Körperseite, bzw. zum Präkontrast-Bild.

### Beispiel 24

### Retention des kontrastgebenden Metalls am Injektionsort

Nach s.c. Gabe von 10 µmol Gesamtgadolinium/kg KG in die Meerschweinchenpfote wurde die Retention des Metalls am Injektionsort zu verschiedenen Zeitpunkten untersucht.

| **Substanz** **Beispiel** | **Gadolinium-Gehalt an der Injektionsstelle** **(Pfote)** **[% Dosis]** | | |
|---|---|---|---|
| | **30 min p.i.** | **90 min p.i.** | **7 d p.i.** |
| 7 | 54.2% | 36.8% | 1.3 % |
| 6b | 66.4% | 26.1 % | 0.6% |
| 5e | 8.5% | 9.4% | - |
| 3c | 6.5% | 4.9% | 1.7% |

### Beispiel 25

### Organverteilung des Kontrastmittels nach s.c. Gabe

Nach subkutaner Gabe von 10 µmol Gesamtgadolinium/kg KG in die Hinterpfote von stimulierten Meerschweinchen (komplettes Freund-Adjuvant; jeweils 0,1 ml i.m. in den rechten und linken Ober- und Unterschenkel; 2 Wochen vor Gabe der Prüfsubstanzen) wurde 7 Tage nach Applikation die Retention des Metalls in der Leber sowie in Nieren und Milz untersucht.

| **Substanz** **Beispiel** | **Gadolinium- Gehalt in verschiedenen** **Organen** **[% Dosis]** | | |
|---|---|---|---|
| | **Leber** | **Nieren** | **Milz** |
| 7 | 6.2 % | 0.2 % | 0.0 % |
| 6b | 1.5 % | 0.1 % | 0.0 % |
| 3c | 1.3 % | 0.1 % | 0.0 % |

### Beispiel 26

### Relaxivity erfindungsgemäßer Verbindungen

| **Substanz** **Beispiel** | **R1** **[L/mmol∗sec]** **bei 0.47 T und 37° C** | |
|---|---|---|
| | **Wasser** | **Plasma** |
| 7 | 18,1 | 21,0 |
| 6b | 11,6 | 13,3 |
| 5e | 12,4 | 30,3 |
| 3c | 14,0 | 21,0 |
| 1c | 13,8 | 25,7 |
| 2c | 11,8 | 19,6 |
| 4c | 14,4 | 21,9 |
| 10c | 21,6 | 27,8 |

### Beispiel 27

### Verträglichkeit erfindungsgemäßer Verbindungen

| Substanz Beispiel | LD 50 [mmol Gd / kg KW] |
|---|---|
| 2c | 3 |
| 3c | 8 |
| 4c | 0,3 |
| 5e | 15 |

## Patentansprüche

1. Metallkomplexe der allgemeinen Formel I, 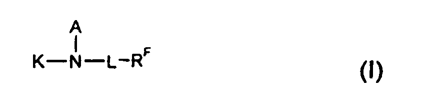 worin
K einen Komplexbildner oder einen Metallkomplex der allgemeinen Formel II 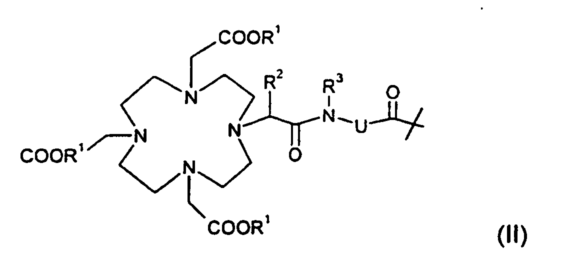 wobei
R¹ für ein Wasserstoffatom oder ein Metallionenäquivalent der Ordnungszahlen 21-29, 31, 32, 37-39, 42-44, 49 oder 57-83,
R² und R³ für ein Wasserstoffatom, eine C₁-C₇-Alkylgruppe, eine Benzylgruppe, eine Phenylgruppe, -CH₂OH oder -CH₂-OCH₃,
U für den Rest L, wobei aber L und U unabhängig voneinander gleich oder verschieden sein können,
steht,
bedeutet,
A ein Wasserstoffatom, eine gereadkettige oder verzweigte C₁-C₃₀-Alkylgruppe, die gegebenenfalls unterbrochen ist durch 1-15 Sauerstoffatome, und/oder gegebenenfalls substituiert ist mit 1-10 Hydroxygruppen, 1-2 COOH-Gruppen, einer Phenylgruppe, einer Benzylgruppe und/oder 1-5 - OR⁴-Gruppen, mit R⁴ in der Bedeutung eines Wasserstoffatoms oder eines C₁-C₇-Alkylrestes, oder -L-R^{F} bedeutet,
L eine geradkettige oder verzweigte C₁-C₃₀-Alkylengruppe, die gegebenenfalls unterbrochen ist durch 1-10 Sauerstoffatome, 1-5 -NH-CO-Gruppen, 1-5 -CO-NH- Gruppen, durch eine gegebenenfalls durch eine COOH-Gruppe substituierte Phenylengruppe, 1-3 Schwefelatome, 1-2 -N(B¹)-SO₂ Gruppen, und/oder 1-2 -SO₂-N(B¹)- Gruppen mit B¹ in der Bedeutung von A, und/oder gegebenenfalls substituiert ist mit dem Rest R^{F},
bedeutet und
R^{F} einen geradkettigen oder verzweigten perfluorierten Alkylrest der Formel CₙF₂ₙX,
wobei 4 ≤n ≤20 ist und
X für ein endständiges Fluoratom, Chloratom, lodatom oder ein Wasserstoffatom steht,
bedeutet,
und gegebenenfalls vorhandene Säuregruppen gegebenenfalls als Salze organischer und/oder anorganischer Basen oder Aminosäuren oder Aminosäureamide vorliegen können.

2. Verbindungen gemäß Anspruch 1, **dadurch** gekennzeichent, daß das Metallionenäquivalent R¹ ein Element der Ordnungszahlen 21 - 29, 39, 42, 44 oder 57 - 83 ist.

3. Verbindungen gemäß Anspruch 1, **dadurch** gekennzeichent, daß das Metallionenäquivalent R¹ ein Element der Ordnungszahlen 27, 29, 31, 32, 37 - 39, 43, 49, 62, 64, 70, 75 und 77 ist.

4. Verbindungen gemäß Anspruch 1, daduch **gekennzeichnet**, daß R², R³ und R⁴ unabhängig voneinander Wasserstofff oder eine C₁-C₄-Alkylgruppe bedeuten.

5. Verbindungen gemäß Anspruch 1, **dadurch gekennzeichnet, daß** A Wasserstoff, einen C₁-C₁₅-Alkylrest,
die Reste C₂H₄-O-CH₃, C₃H₆-O-CH₃,
C₂H₄-O-(C₂H₄-O)ₜ-C₂H₄-OH,
C₂H₄-O-(C₂H₄-O)ₜ-C₂H₄-OCH₃,
C₂H₄OH, C₃H₆OH, C₄H₈OH, C₅H₁₀OH, C₆H₁₂OH, C₇H₁₄OH,
CH(OH)CH₂OH,
CH(OH)CH(OH)CH₂OH, CH₂[CH(OH)]ᵤCH₂OH,
CH[CH₂(OH)]CH(OH)CH₂OH,
C₂H₄CH(OH)CH₂OH,
(CH₂)ₛCOOH,
C₂H₄-O-(C₂H₄-O)ₜ-CH₂COOH oder
C₂H₄-O-(C₂H₄-O)ₜ-C₂H₄-CₙF₂ₙX bedeutet,
wobei
s für die ganzen Zahlen 1 bis 15,
t für die ganzen Zahlen 0 bis 13,
u für die ganzen Zahlen 1 bis 10,
n für die ganzen Zahlen 4 bis 20 steht, und
X für ein Fluor-, Chlor-, Brom- oder lodatom
sowie, falls möglich, ihre verzweigten Isomeren.

6. Verbindungen gemäß Anspruch 1, **dadurch gekennzeichnet, daß** A Wasserstoff, C₁-C₁₀-Alkyl,
C₂H₄-O-CH₃, C₃H₆-O-CH₃,
C₂H₄-O-(C₂H₄-O)ₓ-C₂H₄-OH, C₂H₄-O-(C₂H₄-O)ₓ-C₂H₄-OCH₃,
C₂H₄OH, C₃H₆OH,
CH₂[CH(OH)]_{y}CH₂OH,
CH[CH₂(OH)]CH(OH)CH₂OH,
(CH₂)_{w}COOH,
C₂H₄-O-(C₂H₄-O)ₓ-CH₂COOH,
C₂H₄-O-(C₂H₄-O)ₓ-C₂H₄-CₚF₂ₚX
wobei
x für die ganzen Zahlen 0 bis 5,
y für die ganzen Zahlen 1 bis 6,
w für die ganzen Zahlen 1 bis 10,
p für die ganzen Zahlen 4 bis 15 und
X für ein Fluoratom steht, sowie, falls möglich, ihre verzweigten Isomeren.

7. Verbindungen gemäß Anspruch 1, **dadurch gekennzeichnet, daß** L
α-(CH₂)ₖ-β
α-CH₂-CH₂-(O-CH₂-CH₂-)ᵣ-β
α-CH₂-(O-CH₂-CH₂-)ᵣ-β,
α-CH₂-NH-CO-β
α-CH₂-CH₂-NH-SO₂-β
α-CH₂-NH-CO-CH₂-N(CH₂COOH)-SO₂-β
α-CH₂-NH-CO-CH₂-N(C₂H₅)-SO₂-β
α-CH₂-NH-CO-CH₂-N(C₁₀H₂₁)-SO₂-β
α-CH₂-NH-CO-CH₂-N(C₆H₁₃)-SO₂-β
α-CH₂-NH-CO-(CH₂)₁₀-N(C₂H₅)-SO₂-β
α-CH₂-NH-CO-CH₂-N(-CH₂-C₆H₅)-SO₂-β
α-CH₂-NH-CO-CH₂-N(-CH₂-CH₂-OH)SO₂-β
α-CH₂-NHCO-(CH₂)₁₀-S-CH₂CH₂-β
α-CH₂NHCOCH₂-O-CH₂CH₂-β
α-CH₂-CH₂NHCOCH₂-O-CH₂CH₂-β
α-CH₂-(CH₂-CH₂-O)ₜ-(CH₂)₃NHCO-CH₂-O-CH₂CH₂-β
α-CH₂NHCO(CH₂)₁₀-O-CH₂CH₂-β
α-CH₂CH₂NHCO(CH₂)₁₀-O-CH₂CH₂-β
α-CH₂-C₆H₄-O-CH₂CH₂-β wobei die Phenylengruppe 1,4 oder 1,3 verknüpft ist
α-CH₂-O-CH₂-C(CH₂-OCH₂CH₂-C₆F₁₃)₂-CH₂-OCH₂-CH₂-β
α-CH₂-NHCOCH₂CH₂CON-CH₂CH₂NHCOCH₂N(C₂H₅)SO₂C₈F₁₇β
α-CH₂-CH₂NHCOCH₂N(C₂H₅)-SO₂-β
α-CH₂-O-CH₂-CH(OC₁₀H₂₁)-CH₂-O-CH₂CH₂-β
α-(CH₂NHCO)₄-CH₂O-CH₂CH₂-β
α-(CH₂NHCO)₃-CH₂O-CH₂CH₂-β
α-CH₂-OCH₂C(CH₂OH)₂-CH₂-O-CH₂CH₂-β 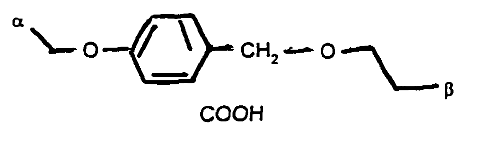 α-CH₂NHCOCH₂N(C₆H₅)-SO₂-β
α-NHCO-CH₂-CH₂-β
α-NHCO-CH₂-O-CH₂CH₂-β
α-NH-CO-β
α-NH-CO-CH₂-N(CH₂COOH)-SO₂-β
α-NH-CO-CH₂-N(C₂H₅)-SO₂-β
α-NH-CO-CH₂-N(C₁₀H₂₁)-SO₂-β
α-NH-CO-CH₂-N(C₆H₁₃)-SO₂-β
α-NH-CO-(CH₂)₁₀-N(C₂H₅)-SO₂-β
α-NH-CO-CH₂-N(-CH₂-C₆H₅)-SO₂-β
α-NH-CO-CH₂-N(-CH₂-CH₂-OH)SO₂-β
α-NH-CO-CH₂-β
α-CH₂-O-C₆H₄-O-CH₂-CH₂-β
α-CH₂-C₆H₄-O-CH₂-CH₂-β
α-N(C₂H₅)-SO₂-β
α-N(C₆H₅)-SO₂-β
α-N(C₁₀H₂₁)-SO₂-β
α-N(C₆H₁₃)-SO₂-β
α-N(C₂H₄OH)-SO₂-β
α-N(CH₂COOH)-SO₂-β
α-N(CH₂C₆H₅)-SO₂-β
α-N-[CH(CH₂OH)₂]-SO₂-β
α-N-[CH(CH₂OH)CH(OH)(CH₂OH)]-SO₂-β bedeutet,
wobei
k für die ganzen Zahlen 1 bis 15 und
r für die ganzen Zahlen 1 bis 6 steht.

8. Verbindungen gemäß Anspruch 1, **dadurch gekennzeichnet, daß** L
α-CH₂-O-CH₂CH₂-β,
α-CH₂-CH₂-(O-CH₂-CH₂-)_{y}-β,
α-CH₂-(O-CH₂-CH₂-)_{y}-β,
α-CH₂-CH₂-NH-SO₂-β,
α-CH₂NHCOCH₂-O-CH₂CH₂-β,
α-CH₂-CH₂NHCOCH₂-O-CH₂CH₂-β,
α-CH₂(CH₂CH₂-O)_{y}-(CH₂)₃NHCO-CH₂O-CH₂CH₂-β,
α-CH₂NHCO(CH₂)₁₀-O-CH₂CH₂-β,
α-CH₂CH₂NHCO(CH₂)₁₀-O-CH₂CH₂-β,
α-CH₂-O-CH₂-CH(OC₁₀H₂₁)-CH₂-O-CH₂CH₂-β,
α-CH₂-O-C₆H₄-O-CH₂-CH₂-β oder
α-CH₂-C₆H₄-O-CH₂-CH₂-β bedeutet,
wobei
y für die ganzen Zahlen 1 bis 6 steht.

9. Verbindungen gemäß Anspruch 1, **dadurch gekennzeichnet, daß** R^{F} einen geradkettigen oder verzweigten perfluorierten Alkylrest der Formel CₚF₂ₚX bedeutet, wobei 4 kleiner gleich p kleiner gleich 15 ist, und X für ein endständiges Fluoratom steht.

10. Verwendung von Verbindungen gemäß Anspruch 2 zur Herstellung eines Kontrastmittels zur Anwendung in der NMR- und Röntgendiagnostik.

11. Verwendung von Verbindungen gemäß Anspruch 3 zur Herstellung eines Kontrastmittels zur Anwendung in der Radiodiagnostik und Radiotherapie.

12. Verwendung von Verbindungen gemäß Anspruch 1 zur Herstellung eines Kontrastmittels zur Anwendung in der indirekten Lymphographie.

13. Verwendung von Verbindungen gemäß Anspruch 1 zur Herstellung von lymphspezifischen Kontrastmittelzubereitungen zur Diagnose von Veränderungen des Lymphsystems.

14. Verwendung von Verbindungen gemäß Anspruch 1 zur Herstellung eines Kontrastmittels zur Anwendung in der i.v. Lymphographie.

15. Pharmazeutische Mittel, enthaltend mindestens eine physiologisch verträgliche Verbindung gemäß Anspruch 1, gegebenenfalls mit den in der Galenik üblichen Zusätzen.

16. Verfahren zur Herstellung der Verbindungen der allgemeinen Formel I 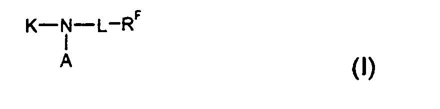 mit
K in der Bedeutung eines Metallkomplexes der allgemeinen Formel II 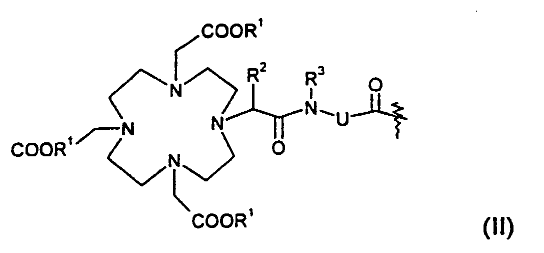 worin die Reste R², R³, und U die in Anspruch 1 angegebenen Bedeutungen haben und R¹ für Wasserstoff oder ein Metallionenäquivalent der Ordnungszahlen 21-29, 31, 32, 37-39, 42-44, 49 oder 57-83 steht,
und L, R^{F} und A die in Anspruch 1 angegebenen Bedeutungen haben,
**dadurch gekennzeichnet, daß** in an sich bekannter Weise eine Verbindung der allgemeinen Formel IIIb 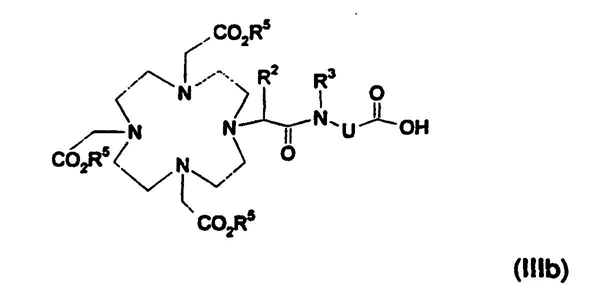 worin R⁵ ein Metallionenäquivalent der Ordnungszahlen 21-29, 31, 32, 37-39, 42-44, 49 oder 57-83 oder eine Carboxylschutzgruppe bedeutet,
in gegebenenfalls aktivierter Form mit einem Amin der allgemeinen Formel IV, 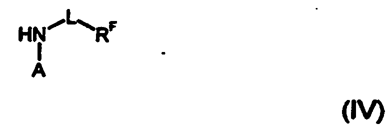 worin A, L und R^{F} die oben angegebenen Bedeutungen haben, in einer Kupplungsreaktion und gegebenenfalls nachfolgender Abspaltung gegebenenfalls vorhandener Schutzgruppen zu einer Verbindung der aligmeinen Formel Ia 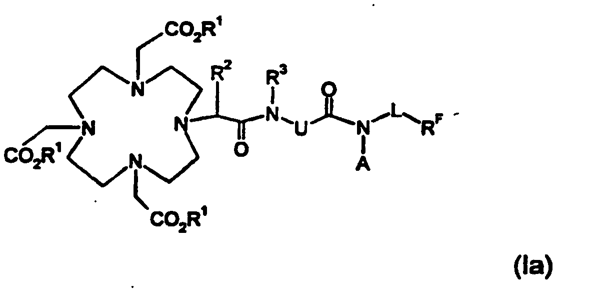 worin die Reste R², R³, U, L, R^{F} und A die oben angegebenen Bedeutungen haben und R¹ für ein Metallionenäquivalent der Ordnungszahlen 21-29, 31, 32, 37-39, 42-44, 49 oder 57-83 steht,
umgesetzt wird oder, wenn R⁵ die Bedeutung einer Schutzgruppe hat, nach Abspaltung dieser Schutzgruppen in einem Folgeschritt in an sich bekannter Weise mit mindestens einem Metalloxid oder Metallsalz eines Elements der Ordnungszahlen 21-29, 31, 32, 37-39, 42-44, 49 oder 57-83 umgesetzt wird, und anschließend falls gewünscht gegebenenfalls vorhandene acide Wasserstoffatome durch Kationen anorganischer und/oder organischer Basen, Aminosäuren oder Aminosäureamide substituiert werden.

## Claims

1. Metal complexes of general formula I 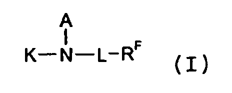 in which
K is a complexing agent or a metal complex of general formula II 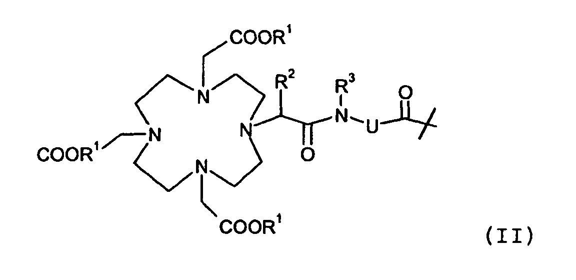 where
R¹ is a hydrogen atom or a metal ion equivalent of atomic numbers 21-29, 31, 32, 37-39, 42-44, 49 or 57-83,
R² and R³ are a hydrogen atom, a C₁-C₇ alkyl group, a benzyl group, a phenyl group, -CH₂OH or -CH₂-OCH₃,
U has the same meaning as the radical L, whereby L and U, independently of one another, can be the same or different,
A is a hydrogen atom, a straight-chain or branched C₁-C₃₀ alkyl group, which optionally is interrupted by 1-15 oxygen atoms and/or optionally is substituted with 1-10 hydroxy groups, 1-2 COOH groups, a phenyl group, a benzyl group and/or 1-5-OR⁴ groups, where R⁴ is a hydrogen atom or a C₁-C₇ alkyl radical, or -L-R^{F},
L is a straight-chain or branched C₁-C₃₀ alkylene group, which optionally is interrupted by 1-10 oxygen atoms, 1-5-NH-CO-groups, 1-5-CO-NH-groups, by a phenylene group that is optionally substituted by a COOH group, 1-3 sulphur atoms; 1-2-N(B¹)-SO₂ - groups, and/or 1-2-SO₂-N(B¹)-groups where B¹ is as defined for A, and/or optionally is substituted with the radical R^{F}, and
R^{F} is a straight-chain or branched perfluorinated alkyl radical of the formula Cₙ F₂ₙ X,
whereby 4 ≤ n ≤ 20 and
X is a terminal fluorine atom, chlorine atom, iodine atom or a hydrogen atom,
and optionally present acid groups optionally can be present as salts of organic and/or inorganic bases or amino acids or amino acid amides.

2. Metal complexes according to Claim 1, **characterized in that** the metal ion equivalent R¹ is an element of atomic number 21-29, 39, 42, 44 or 57-83.

3. Compounds according to Claim 1, **characterized in that** the metal ion equivalent R¹ is an element of atomic number 27, 29, 31, 32, 37-39, 43, 49, 62, 64, 70, 75 or 77.

4. Compounds according to Claim 1, **characterized in that** R², R³ and R⁴, independently of one another, are hydrogen or a C₁-C₄ alkyl group.

5. Compounds according to Claim 1, **characterized in that** A is hydrogen, a C₁-C₁₅ alkyl radical, the radicals
C₂H₄-O-CH₃, C₃H₆-O-CH₃, C₂H₄-O-(C₂H₄-O)ₜ-C₂H₄-OH,
C₂H₄-O-(C₂H₄-O)ₜ-C₂H₄-OCH₃, C₂H₄OH, C₃H₆OH, C₄H₈OH, C₅H₁₀OH, C₆H₁₂OH, C₇H₁₄OH,
CH(OH)CH₂OH, CH(OH)CH(OH)CH₂OH, CH₂[CH(OH)]ᵤCH₂OH,
CH[CH₂(OH)]CH(OH)CH₂OH, C₂H₄CH(OH)CH₂OH, (CH₂)ₛCOOH,
C₂H₄-O-(C₂H₄-O)ₜ-CH₂COOH, or C₂H₄-O-(C₂H₄-O)ₜ-C₂H₄-CₙF₂ₙX,
where
s is an integer of 1 to 15,
t is an integer of 0 to 13,
u is an integer of 1 to 10,
n is an integer of 4 to 20, and
X is a fluorine, chlorine, bromine or iodine atom, or a branched isomer of one of these groups.

6. Compounds according to Claim 1, **characterized in that** A is hydrogen, C₁-C₁₀ alkyl,
C₂H₄-O-CH₃, C₃H₆-O-CH₃, C₂H₄-O-(C₂H₄-O)ₓ-C₂H₄-OH, C₂H₄-O-(C₂H₄-O)ₓ O₂H₄-OCH₃,
C₂H₄OH, C₃H₆OH,CH₂[CH(OH)]_{y}CH₂OH,CH[CH₂
(OH)]CH(OH)CH₂OH, (CH₂)_{w}COOH,
C₂H₄-O-(C₂H₄-O)ₓ-CH2COOH, C₂H₄-O-(C₂H₄-O)ₓ-C₂H₄-CₚF₂ₚX)
where
x is an integer of 0 to 5,
y is an integer of 1 to 6,
w is an integer of 1 to 10,
p is an integer of 4 to 15, and
X stands for a fluorine atom, or a branched isomer of one of these groups.

7. Compounds according to Claim 1, **characterized in that** L is
α-(CH₂)ₖ-β
α-CH₂-CH₂-(O-CH₂-CH₂-)ᵣ-β
α-CH₂-(O-CH₂-CH₂-)ᵣ-β)
α-CH₂-NH-CO-β
α-CH₂-CH₂-NH-SO₂-β
α-CH₂-NH-CO-CH₂-N(CH₂COOH)-SO₂-β
α-CH₂-NH-CO-CH₂-N(C₂H₅)-SO₂-β
α-CH₂-NH-CO-CH₂-N(C₁₀H₂₁)-SO₂-β
α-CH₂-NH-CO-CH₂-N(C₆H₁₃)-SO₂-β
α-CH₂-NH-CO-(CH₂)₁₀-N(C₂H₅)-SO₂-β
α-CH₂-NH-CO-CH₂-N(-CH₂-C₆H₅)-SO₂-β
α-CH₂-NH-CO-CH₂-N(-CH₂-CH₂-OH)SO₂-β
α-CH₂-NHCO-(CH₂)₁₀-S-CH₂CH₂-β
α-CH₂NHCOCH₂-O-CH₂CH₂-β
α-CH₂-CH₂NHCOCH₂-O-CH₂CH₂-β
α-CH₂-(CH₂-CH₂-O)ₜ-(CH₂)₃NHCO-CH₂-O-CH₂CH₂-β
α-CH₂NHCO(CH₂)₁₀-O-CH₂CH₂-β
α-CH₂CH₂NHCO(CH₂)₁₀-O-CH₂CH₂-β
α-CH₂-C₆H₄-O-CH₂CH₂-β wherein the phenylene group is 1,4- or 1,3-linked
α-CH₂-O-CH₂-C(CH₂-OCH₂CH₂-C₆F₁₃)₂-CH₂-OCH₂-CH₂-β
α-CH₂-NHCOCH₂CH₂CON-CH₂CH₂NHCOCH₂N(C₂H₅)SO₂C₈F₁₇β
α-CH₂-CH₂NHCOCH₂N(C₂H₅)-SO₂-β
α-CH₂-O-CH₂-CH(OC₁₀H₂₁)-CH₂-O-CH₂CH₂-β
α-(CH₂NHCO)₄-CH₂O-CH₂CH₂-β
α-(CH₂NHCO)₃-CH₂O-CH₂CH₂-β
α-CH₂-OCH₂C(CH₂OH)₂-CH₂-O-CH₂CH₂-β 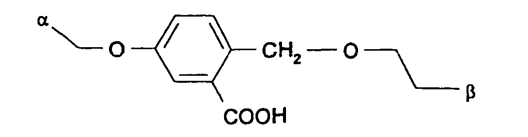 α-CH₂NHCOCH₂N(C₆H₅)-SO₂-β
α-NHCO-CH₂-CH₂-β
α-NHCO-CH₂-O-CH₂CH₂-β
α-NH-CO-β
α-NH-CO-CH₂-N(CH₂COOH)-SO₂-β
α-NH-CO-CH₂-N(C₂H₅)-SO₂-β
α-NH-CO-CH₂-N(C₁₀H₂₁)-SO₂-β
α-NH-CO-CH₂-N(C₆H₁₃)-SO₂-β
α-NH-CO-(CH₂)₁₀-N(C₂H₅)-SO₂-β
α-NH-CO-CH₂-N(-CH₂-C₆H₅)-SO₂-β
α-NH-CO-CH₂-N(-CH₂-CH₂-OH)SO₂-β
α-NH-CO-CH₂-β
α-CH₂-O-C₆H₄-O-CH₂-CH₂-β
α-CH₂-C₆H₄-O-CH₂-CH₂-β
α-N(C₂H₅)-SO₂-β
α-N(C₆H₅)-SO₂-β
α-N(C₁₀H₂₁)-SO₂-β
α-N(C₆H₁₃)-SO₂-β
α-N(C₂H₄OH)-SO₂-β
α-N(CH₂COOH)-SO₂-β
α-N(CH₂C₆H₅)-SO₂-β
α-N-[CH(CH₂OH)₂]-SO₂-β, or
α-N-[CH(CH₂OH)CH(OH)(CH₂OH)]-SO₂-β
wherein
k is an integer of 1 to 15, and
r is an integer of 1 to 6.

8. Compounds according to Claim 1, **characterized in that** L is
α-CH₂-O-CH₂CH₂-β,
α-CH₂-CH₂-(O-CH₂-CH₂-)_{y}-β,
α-CH₂-(O-CH₂-CH₂-)_{y}-β,
α-CH₂-CH₂-NH-SO₂-β,
α-CH₂NHCOCH₂-O-CH₂CH₂-β,
α-CH₂-CH₂NHCOCH₂-O-CH₂CH₂-β,
α-CH₂-(CH₂-CH₂-O)_{y}-(CH₂)₃NHCO-CH₂-O-CH₂CH₂-β,
α-CH₂NHCO(CH₂)₁₀-O-CH₂CH₂-β,
α-CH₂CH₂NHCO(CH₂)₁₀-O-CH₂CH₂-β,
α-CH₂-O-CH₂-CH(OC₁₀H₂₁)-CH₂-O-CH₂CH₂-β,
α-CH₂-O-C₆H₄-O-CH₂-CH₂-β or
α-CH₂-C₆H₄-O-CH₂-CH₂-β
wherein y is an integer of 1 to 6.

9. Compounds according to Claim 1, **characterized in that** R^{F} is a straight-chain or branched perfluorinated alkyl radical of the formula Cₚ F₂ₚ X, where 4 is equal to or less than p, which is equal to or less than 15, and X stands for a terminal fluorine atom.

10. Use of compounds according to Claim 2 for producing a contrast medium for use in NMR or x-ray diagnosis.

11. Use of compounds according to Claim 3, for producing a contrast medium for use in radiodiagnosis or radiotherapy.

12. Use of compounds according to Claim 1, for producing a contrast medium preparation for use in indirect lymphography.

13. Use of compounds according to Claim 1, for producing a lymph-specific contrast medium preparation for use in diagnosis of changes of the lymphatic system.

14. Use of compounds according to Claim 1, for producing a contrast medium for use in intravenous lymphography.

15. Pharmaceutical composition which comprises at least one physiologically compatible compound according to Claim 1, optionally with the auxiliaries customarily used in galenics.

16. Process for preparing the compounds of the general formula I 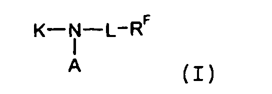 with
K in the meaning of a metal complex of the general formula II 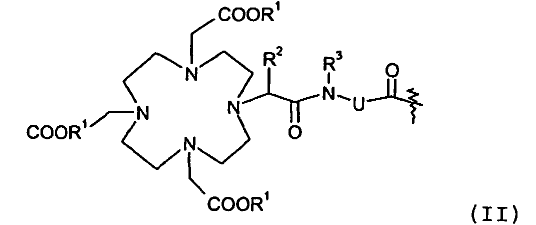 wherein the radicals R², R³ and U have the meanings given in Claim 1 and R¹ is hydrogen or a metal ion equivalent of atomic numbers 21-29, 31, 32, 37-39, 42-44, 49 or 57-83,
and L, R^{F} and A have the meanings given in Claim 1, **characterized in that**
a compound of the general formula IIIb 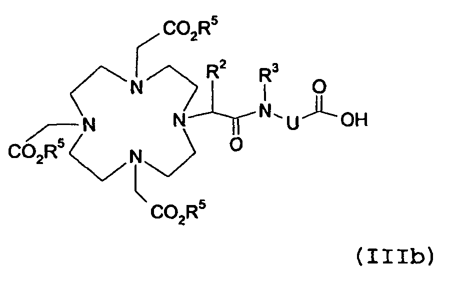 in which R⁵ is a metal ion equivalent of atomic numbers 21-29, 31, 32, 37-39, 42-44, 49 or 57-83 or a carboxyl protective group, in optionally activated form,
is reacted in a manner known per se with an amine of the general formula IV 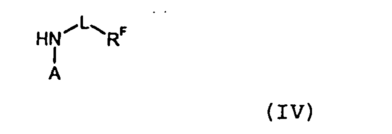 in which A, L and R^{F} have the above-indicated meanings, by a coupling reaction, and optionally subsequently cleaving optionally present protective groups to provide a compound of the general formula Ia 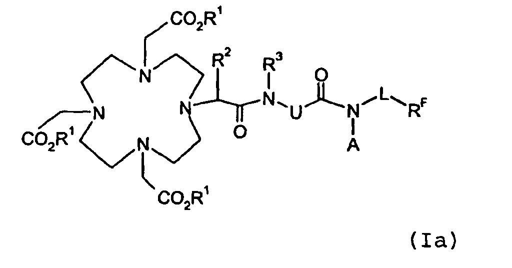 in which radicals R², R³, U, L, R^{F} and A have the above-indicated meanings, and R¹ stands for a metal ion equivalent of atomic numbers 21-29, 31, 32, 37-39, 42-44, 49 or 57-83,
if R⁵ has the meaning of a protective group, reacting the compound of formula Ia after cleaving of the protective groups in a subsequent step in a manner known per se with at least one metal oxide or metal salt of an element of atomic numbers 21-29, 31, 32, 37-39, 42-44, 49 or 57-83, and then, if desired,
substituting optionally present acid hydrogen atoms by cations of inorganic and/or organic bases, amino acids or amino acid amides.

## Revendications

1. Complexes métalliques de formule générale I, 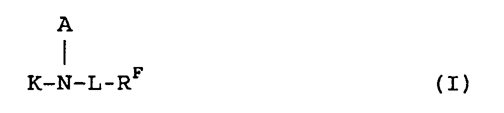 dans laquelle
K représente un agent complexant ou un complexe métallique de formule générale II 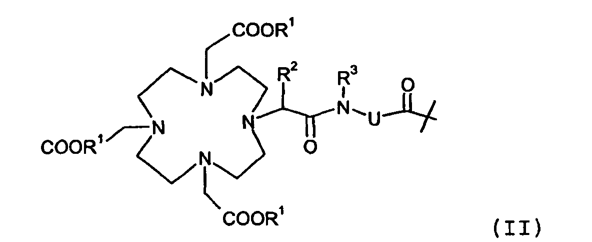 où
R¹ représente un atome d'hydrogène ou un équivalent d'ion métallique des nombres atomiques 21-29, 31, 32, 37-39, 42-44, 49 ou 57-83,
R² et R³ représentent un atome d'hydrogène, un groupe alkyle en C₁-C₇, un groupe benzyle, un groupe phényle, -CH₂OH ou -CH₂OCH₃,
U représente le reste L, L et U, indépendamment l'un de l'autre, pouvant toutefois être identiques ou différents,
A représente un atome d'hydrogène, un groupe alkyle en C₁-C₃₀ à chaîne droite ou ramifiée, qui est éventuellement interrompu par 1-15 atomes d'oxygène et/ou est éventuellement substitué par 1-10 groupes hydroxy, 1-2 groupes COOH, par un groupe phényle, par un groupe benzyle et/ou 1-5 groupes OR⁴, R⁴ représentant un atome d'hydrogène ou un radical alkyle en C₁-C₇, ou représente L-R^{F},
L représente un groupe alkylène en C₁-C₃₀ à chaîne droite ou ramifiée, qui est éventuellement interrompu par 1-10 atomes d'oxygène, 1-5 groupes -NH-CO-, 1-5 groupes -CO-NH-, par un groupe phénylène éventuellement substitué par un groupe COOH, 1-3 atomes de soufre, 1-2 groupes -N(B¹)-SO₂- et/ou 1-2 groupes - SO₂-N(B¹)-, B¹ ayant la signification de A, et/ou est éventuellement substitué par le reste R^{F},
et
R^{F} représente un radical alkyle perfluoré à chaîne droite ou ramifiée, de formule CₙF₂ₙX,
où 4 ≤ n ≤ 20 et
X représente un atome de fluor, un atome de chlore, un atome d'iode en bout de chaîne ou un atome d'hydrogène,
et des groupes acides éventuellement présents pouvant éventuellement se trouver sous forme de sels de bases organiques et/ou minérales ou d'acides aminés ou d'amides d'acides aminés.

2. Composés selon la revendication 1, **caractérisés en ce que** l'équivalent d'ion métallique R¹ est un élément des nombres atomiques 21-29, 39, 42, 44 ou 57-83.

3. Composés selon la revendication 1, **caractérisés en ce que** l'équivalent d'ion métallique R¹ est un élément des nombres atomiques 27, 29, 31, 32, 37-39, 43, 49, 62, 64, 70, 75 et 77.

4. Composés selon la revendication 1, **caractérisés en ce que** R², R³ et R⁴ représentent, indépendamment les uns des autres, un atome d'hydrogène ou un groupe alkyle en C₁-C₄.

5. Composés selon la revendication 1, **caractérisés en ce que** A représente un atome d'hydrogène, un radical alkyle en C₁-C₁₅,
les radicaux C₂H₄-O-CH₃, C₃H₆-O-CH₃,
C₂H₄-O-(C₂H₄-O)ₜ-C₂H₄-OH,
C₂H₄-O-(C₂H₄-O)ₜ-C₂H₄-OCH₃,
C₂H₄OH, C₃H₆OH, C₄H₈OH, C₅H₁₀OH, C₆H₁₂OH, C₇H₁₄OH,
CH(OH)CH₂OH,
CH(OH)CH(OH)CH₂OH, CH₂[CH(OH)]ᵤCH₂OH,
CH[CH₂(OH)]CH(OH)CH₂OH,
C₂H₄CH(OH)CH₂OH,
(CH₂)ₛCOOH,
C₂H₄-O-(C₂H₄-O)ₜ-CH₂COOH ou
C₂H₄-O-(C₂H₄-O)ₜ-C₂H₄-CₙF₂ₙX,
s représentant les nombres entiers de 1 à 15,
t représentant les nombres entiers de 0 à 13,
u représentant les nombres entiers de 1 à 10,
n représentant les nombres entiers de 4 à 20, et
X représentant un atome de fluor, chlore, brome ou iode,
ainsi que, si possible, leurs isomères ramifiés.

6. Composés selon la revendication 1, **caractérisés en ce que** A représente un atome d'hydrogène, un groupe alkyle en C₁-C₁₀,
C₂H₄-O-CH₃, C₃H₆-O-CH₃,
C₂H₄-O-(C₂H₄-O)ₓ-C₂H₄-OH, C₂H₄-O-(C₂H₄-O)ₓ-C₂H₄-O-CH₃,
C₂H₄OH, C₃H₆OH,
CH₂[CH(OH)]_{y}CH₂OH,
CH [CH₂(OH)]CH(OH) CH₂OH,
(CH₂)_{w}COOH,
C₂H₄-O-(C₂H₄-O)ₓ-CH₂COOH,
C₂H₄-O-(C₂H₄-O)ₓ-C₂H₄-CₚF₂ₚX
x représentant les nombres entiers de 0 à 5,
y représentant les nombres entiers de 1 à 6,
w représentant les nombres entiers de 1 à 10,
p représentant les nombres entiers de 4 à 15, et
X représentant un atome de fluor,
ainsi que, si possible, leurs isomères ramifiés.

7. Composés selon la revendication 1, **caractérisés en ce que** L représente
α-(CH₂)ₖ-β
α-CH₂-CH₂-(O-CH₂-CH₂-)ᵣ-β,
α-CH₂-(O-CH₂-CH₂-)ᵣ-β,
α-CH₂-NH-CO-β,
α-CH₂-CH₂-NH-SO₂-β,
α-CH₂-NH-CO-CH₂-N(CH₂COOH)-SO₂-β
α-CH₂-NH-CO-CH₂-N(C₂H₅)-SO₂-β
α-CH₂-NH-CO-CH₂-N(C₁₀H₂₁)-SO₂-β
α-CH₂-NH-CO-CH₂-N(C₆H₁₃)-SO₂-β
α-CH₂-NH-CO-(CH₂)₁₀-N(C₂H₅)-SO₂-β
α-CH₂-NH-CO-CH₂-N(-CH₂-C₆H₅)-SO₂-β
α-CH₂-NH-CO-CH₂-N(-CH₂-CH₂-OH)SO₂-β
α-CH₂-NHCO-(CH₂)₁₀-S-CH₂CH₂-β
α-CH₂NHCOCH₂-O-CH₂CH₂-β
α-CH₂-CH₂NHCOCH₂-O-CH₂CH₂-β
α-CH₂-(CH₂-CH₂-O)ₜ-(CH₂)₃NHCO-CH₂-O-CH₂CH₂-β
α-CH₂NHCO(CH₂)₁₀-O-CH₂CH₂-β
α-CH₂CH₂NHCO(CH₂)₁₀-O-CH₂CH₂-β
α-CH₂-C₆H₄-O-CH₂CH₂-β, le groupe phénylène étant lié en positions 1,4 ou 1,3
α-CH₂-O-CH₂-C(CH₂-OCH₂CH₂-C₆F₁₃)₂-CH₂-OCH₂-CH₂-β
α-CH₂-NHCOCH₂CH₂CON-CH₂CH₂NHCOCH₂N(C₂H₅)SO₂C₈F₁₇β
α-CH₂-CH₂NHCOCH₂N(C₂H₅)-SO₂-β
α-CH₂-O-CH₂-CH(OC₁₀H₂₁)-CH₂-O-CH₂CH₂-β
α-(CH₂NHCO)₄-CH₂O-CH₂CH₂-β
α-(CH₂NHCO)₃-CH₂O-CH₂CH₂-β
α-CH₂-OCH₂C(CH₂OH)₂-CH₂-O-CH₂CH₂-β 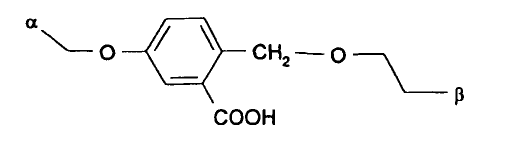 α-CH₂NHCOCH₂N(C₆H₅)-SO₂-β
α-NHCO-CH₂-CH₂-β
α-NHCO-CH₂-O-CH₂CH₂-β
α-NH-CO-β
α-NH-CO-CH₂-N(CH₂COOH)-SO₂-β
α-NH-CO-CH₂-N(C₂H₅)-SO₂-β
α-NH-CO-CH₂-N(C₁₀H₂₁)-SO₂-β
α-NH-CO-CH₂-N(C₆H₁₃)-SO₂-β
α-NH-CO-(CH₂)₁₀-N(C₂H₅)-SO₂-β
α-NH-CO-CH₂-N(-CH₂-C₆H₅)-SO₂-β
α-NH-CO-CH₂-N(-CH₂-CH₂-OH)SO₂-β
α-NH-CO-CH₂-β
α-CH₂-O-C₆H₄-O-CH₂-CH₂-β
α-CH₂-C₆H₄-O-CH₂-CH₂-β
α-N(C₂H₅)-SO₂-β
α-N(C₆H₅)-SO₂-β
α-N(C₁₀H₂₁)-SO₂-β
α-N(C₆H₁₃)-SO₂-β
α-N(C₂H₄OH)-SO₂-β
α-N(CH₂COOH)-SO₂-β
α-N(CH₂C₆H₅)-SO₂-β
α-N[CH(CH₂OH)₂]-SO₂-β
α-N[CH(CH₂OH)CH(OH)(CH₂OH)]-SO₂-β,
k représentant les nombres entiers de 1 à 15,
r représentant les nombres entiers de 1 à 6.

8. Composés selon la revendication 1, **caractérisés en ce que** L représente
α-CH₂-O-CH₂CH₂-β
α-CH₂-CH₂-(O-CH₂-CH₂-)_{y}-β,
α-CH₂-(O-CH₂-CH₂-)_{y}-β,
α-CH₂-CH₂-NH-SO₂-β,
α-CH₂NHCOCH₂-O-CH₂CH₂-β
α-CH₂-CH₂NHCOCH₂-O-CH₂CH₂-β
α-CH₂-(CH₂-CH₂-O)_{y}-(CH₂)₃NHCO-CH₂-O-CH₂CH₂-β
α-CH₂NHCO(CH₂)₁₀-O-CH₂CH₂-β
α-CH₂CH₂NHCO(CH₂)₁₀-O-CH₂CH₂-β
α-CH₂-O-CH₂-CH(OC₁₀H₂₁)-CH₂-O-CH₂CH₂-β
α-CH₂-O-C₆H₄-O-CH₂-CH₂-β ou
α-CH₂-C₆H₄-O-CH₂-CH₂-β
y représentant les nombres entiers de 1 à 6.

9. Composés selon la revendication 1, **caractérisés en ce que** R^{F} représente un radical alkyle perfluoré à chaîne droite ou ramifiée, de formule CₚF₂ₚX, où 4 ≤ p ≤ 15 et X représente un atome de fluor en bout de chaîne.

10. Utilisation de composés selon la revendication 2, pour la préparation d'un produit de contraste destiné à l'utilisation dans le diagnostic radiologique et par RMN.

11. Utilisation de composés selon la revendication 3, pour la préparation d'un produit de contraste destiné à l'utilisation dans le diagnostic radiologique et la radiothérapie.

12. Utilisation de composés selon la revendication 1, pour la préparation d'un produit de contraste destiné à l'utilisation dans la lymphographie indirecte.

13. Utilisation de composés selon la revendication 1, pour la production de préparations de produits de contraste spécifiques de la lymphe, destinées au diagnostic d'altérations du système lymphatique.

14. Utilisation de composés selon la revendication 1, pour la préparation d'un produit de contraste destiné à l'utilisation dans la lymphographie i.v.

15. Composition pharmaceutique contenant au moins un composé physiologiquement acceptable selon la revendication 1, éventuellement avec les additifs usuels en galénique.

16. Procédé pour la préparation des composés de formule générale I, 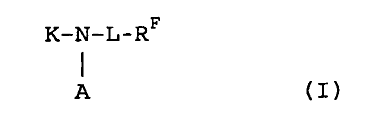 dans laquelle
K représente un complexe métallique de formule générale II 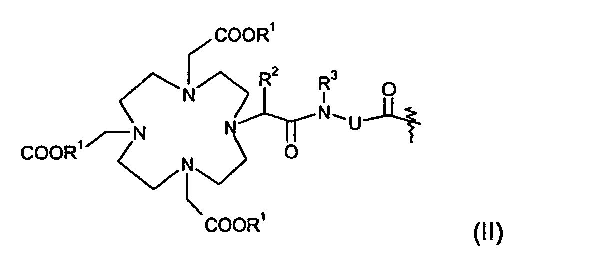 dans laquelle les radicaux R², R³ et U ont les significations données dans la revendication 1 et R¹ représente un atome d'hydrogène ou un équivalent d'ion métallique des nombres atomiques 21-29, 31, 32, 37-39, 42-44, 49 ou 57-83,
et L, R^{F} et A ont les significations données dans la revendication 1,
**caractérisé en ce qu'**on fait réagir d'une façon connue en soi un composé de formule générale IIIb 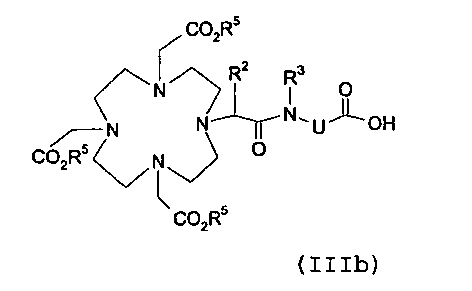 dans laquelle R⁵ représente un équivalent d'ion métallique des nombres atomiques 21-29, 31, 32, 37-39, 42-44, 49 ou 57-83, ou un groupe protecteur de fonction carboxy,
éventuellement sous forme activée, avec une amine de formule générale IV, 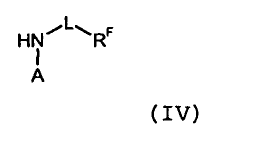 dans laquelle A, L et R^{F} ont les significations données plus haut, dans une réaction de couplage, éventuellement suivie d'élimination de groupes protecteurs éventuellement présents, pour obtenir un composé de formule générale Ia 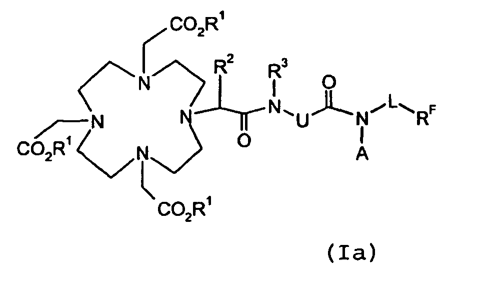 dans laquelle les radicaux R², R³, U, L, R^{F} et A ont les significations indiquées plus haut et R¹ représente un équivalent d'ion métallique des nombres atomiques 21-29, 31, 32, 37-39, 42-44, 49 ou 57-83,
ou, lorsque R⁵ a la signification d'un groupe protecteur, après élimination de ces groupes protecteurs on le fait réagir dans une étape subséquente, d'une façon connue en soi, avec au moins un oxyde métallique ou sel métallique d'un élément des nombres atomiques 21-29, 31, 32, 37-39, 42-44, 49 ou 57-83, et ensuite, si on le désire, on remplace des atomes d'hydrogènes acides éventuellement présents par des cations de bases organiques et/ou minérales, d'acides aminés ou d'amides d'acides aminés.
